# EUROPEAN PATENT SPECIFICATION

(11) **EP 4 266 390 B1**
(45) Date of publication and mention of the grant of the patent: **30.07.2025**
(21) Application number: 22760040.0
(22) Date of filing: 23.02.2022
(51) Int. Cl.: H10K 85/60, H10K 50/11, H10K 101/10, H10K 101/00

(54) **ORGANIC LIGHT EMITTING DEVICE**
ORGANISCHE LICHTEMITTIERENDE VORRICHTUNG
DISPOSITIF ÉLECTROLUMINESCENT ORGANIQUE

(30) Priority: 26.02.2021 KR 20210026796; 22.02.2022 KR 20220023070
(43) Date of publication of application: 25.10.2023
(73) Proprietor: LG Chem, Ltd., Seoul 07336 (KR)
(72) Inventor: HAN, Su Jin, Daejeon 34122 (KR); LEE, Dong Hoon, Daejeon 34122 (KR); SUH, Sang Duk, Daejeon 34122 (KR); JUNG, Min Woo, Daejeon 34122 (KR); LEE, Jungha, Daejeon 34122 (KR); PARK, Seulchan, Daejeon 34122 (KR); HWANG, Sunghyun, Daejeon 34122 (KR)
(74) Representative: Hoffmann Eitle
(86) International application number: PCT/KR2022/002645
(87) International publication number: WO 2022/182124

(56) References cited:
- WO-A1-2007/063754
- WO-A1-2008/056746
- WO-A1-2018/087346
- WO-A1-2020/185038
- KR-A- 20150 117 173
- KR-A- 20170 037 276
- KR-A- 20170 134 035
- KR-A- 20190 110 775
- KR-A- 20200 110 226

## Description

### [TECHNICAL FIELD]

### Cross-reference to Related Application(s)

This application claims the benefit of Korean Patent Applications No. 10-2021-0026796 filed on February 26, 2021 and No. 10-2022-0023070 filed on February 22, 2022 in the Korean Intellectual Property Office.

The present disclosure relates to an organic light emitting device.

### [BACKGROUND OF ART]

In general, an organic light emitting phenomenon refers to a phenomenon where electric energy is converted into light energy by using an organic material. The organic light emitting device using the organic light emitting phenomenon has characteristics such as a wide viewing angle, an excellent contrast, a fast response time, an excellent luminance, driving voltage and response speed, and thus many studies have proceeded.

The organic light emitting device generally has a structure which comprises an anode, a cathode, and an organic material layer interposed between the anode and the cathode. The organic material layer frequently has a multilayered structure that comprises different materials in order to enhance efficiency and stability of the organic light emitting device, and for example, the organic material layer may be formed of a hole injection layer, a hole transport layer, a light emitting layer, an electron transport layer, an electron injection layer, and the like. In the structure of the organic light emitting device, if a voltage is applied between two electrodes, the holes are injected from an anode into the organic material layer and the electrons are injected from the cathode into the organic material layer, and when the injected holes and electrons meet each other, an exciton is formed, and light is emitted when the exciton falls to a ground state again.

There is a continuing need for the development of new materials for the organic materials used in the organic light emitting devices as described above.

### [PRIOR ART LITERATURE]

### [Patent Literature]

(Patent Literature 0001) Korean Unexamined Patent Publication No. 10-2000-0051826
(Patent Literature 0002) WO2018/087346 A1
(Patent Literature 0003) WO2020/185038 A1
(Patent Literature 0004) WO2008/056746 A1
(Patent Literature 0005) WO2007/063754 A1

### [DETAILED DESCRIPTION OF THE INVENTION]

### [Technical Problem]

The present disclosure relates to an organic light emitting device.

### [Technical Solution]

There is provided an organic light emitting device including:
an anode;
a cathode that is provided opposite to the anode; and
a light emitting layer that is provided between the anode and the cathode,
wherein the light emitting layer includes a first compound represented by the following Chemical Formula 1 and a second compound represented by the following Chemical Formula 2: 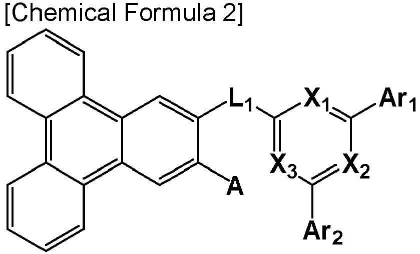 in the Chemical Formula 1,
   A is substituted or unsubstituted carbazolyl,
   L₁ is a single bond, or substituted or unsubstituted C₆₋₆₀ arylene,
   X₁, X₂ and X₃ are each independently N, or CH, provided that at least one of X₁, X₂ and X₃ is N, and
   Ar₁ and Ar₂ are each independently substituted or unsubstituted C₆₋₆₀ aryl, or substituted or unsubstituted C₂₋₆₀ heteroaryl containing at least one heteroatom selected from the group consisting of N, O and S, 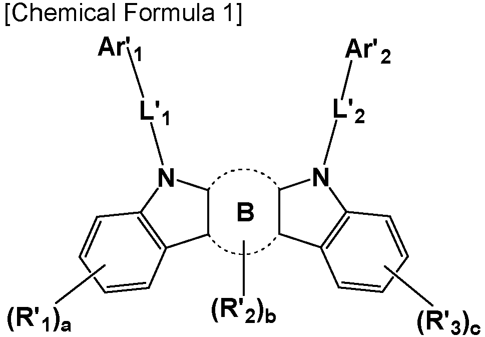 in the Chemical Formula 2,
      B is a benzene ring fused with two adjacent pentagonal rings,
      L'₁ and L'₂ are each independently a single bond, or substituted or unsubstituted C₆₋₆₀ arylene,
      Ar'₁ and Ar'₂ are each independently substituted or unsubstituted C₆₋₆₀ aryl, or substituted or unsubstituted C₂₋₆₀ heteroaryl containing at least one heteroatom selected from the group consisting of **N,** O and S,
      R'₁, R'₂ and R'₃ are each independently hydrogen, deuterium, substituted or unsubstituted C₆₋₆₀ aryl, or substituted or unsubstituted C₂₋₆₀ heteroaryl containing at least one heteroatom selected from the group consisting of **N,** O and S,
      a is an integer of 0 to 4,
      b is an integer of 0 to 2, and
      c is an integer of 0 to 4.

### [ADVANTAGEOUS EFFECTS]

The above-described organic light emitting device can exhibit improved efficiency, driving voltage, and/or lifespan by including two kinds of host compounds in the light emitting layer.

### [BRIEF DESCRIPTION OF THE DRAWINGS]

FIG. 1 shows an example of an organic light emitting device including a substrate 1, an anode 2, a light emitting layer 3, and a cathode 4.
FIG. 2 shows an example of an organic light emitting device including a substrate 1, an anode 2, a hole injection layer 5, a hole transport layer 6, an electron blocking layer 7, a light emitting layer 3, a hole blacking layer 8, an electron transport and injection layer 9, and a cathode 4.

### [DETAILED DESCRIPTION OF THE EMBODIMENTS]

Hereinafter, embodiments of the present disclosure will be described in more detail to facilitate understanding of the invention.

As used herein, the notation 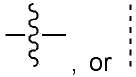 means a bond linked to another substituent group, D means deuterium, and Ph means a phenyl group.

As used herein, the term "substituted or unsubstituted" means being unsubstituted or substituted with one or more substituents selected from the group consisting of deuterium; a halogen group; a nitrile group; a nitro group; a hydroxyl group; a carbonyl group; an ester group; an imide group; an amino group; a phosphine oxide group; an alkoxy group; an aryloxy group; an alkylthioxy group; an arylthioxy group; an alkylsulfoxy group; an arylsulfoxy group; a silyl group; a boron group; an alkyl group; a cycloalkyl group; an alkenyl group; an aryl group; an aralkyl group; an aralkenyl group; an alkylaryl group; an alkylamine group; an aralkylamine group; a heteroarylamine group; an arylamine group; an arylphosphine group; and a heterocyclic group containing at least one of N, O and S atoms, or being unsubstituted or substituted with a substituent in which two or more substituents of the above-exemplified substituents are connected. For example, "a substituent in which two or more substituents are connected" may be a biphenyl group. Namely, a biphenyl group may be an aryl group, or it may also be interpreted as a substituent in which two phenyl groups are connected.

In the present disclosure, the carbon number of a carbonyl group is not particularly limited, but is preferably 1 to 40. Specifically, the carbonyl group may be a group having the following structural formulae, but is not limited thereto.

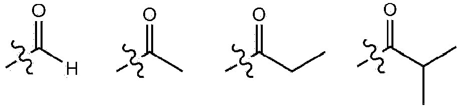

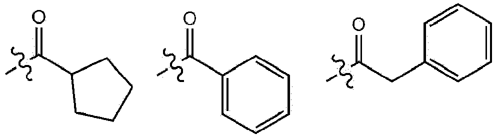

In the present disclosure, an ester group may have a structure in which oxygen of the ester group is substituted by a straight-chain, branched-chain, or cyclic alkyl group having 1 to 25 carbon atoms, or an aryl group having 6 to 25 carbon atoms. Specifically, the ester group may be a group having the following structural formulae, but is not limited thereto.

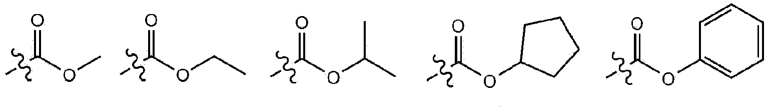

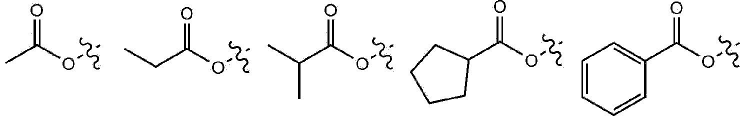

In the present disclosure, the carbon number of an imide group is not particularly limited, but is preferably 1 to 25. Specifically, the imide group may be a group having the following structural formulae, but is not limited thereto.

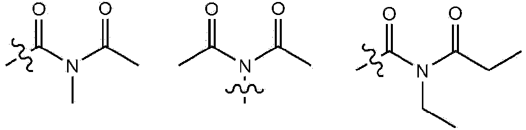

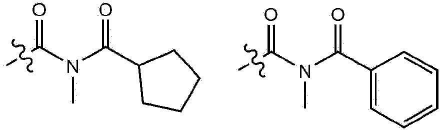

**In** the present disclosure, a silyl group specifically includes a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but is not limited thereto.

In the present disclosure, a boron group specifically includes a trimethylboron group, a triethylboron group, a t-butyldimethylboron group, a triphenylboron group, a phenylboron group and the like, but is not limited thereto.

In the present disclosure, examples of a halogen group include fluorine, chlorine, bromine, or iodine.

In the present disclosure, the alkyl group may be straight-chain, or branched-chain, and the carbon number thereof is not particularly limited, but is preferably 1 to 40. According to one embodiment, the carbon number of the alkyl group is 1 to 20. According to another embodiment, the carbon number of the alkyl group is 1 to 10. According to another embodiment, the carbon number of the alkyl group is 1 to 6. Specific examples of the alkyl group include methyl, ethyl, propyl, n-propyl, isopropyl, butyl, n-butyl, isobutyl, tert-butyl, sec-butyl, 1-methyl-butyl, 1-ethyl-butyl, pentyl, n-pentyl, isopentyl, neopentyl, tert-pentyl, hexyl, n-hexyl, 1-methylpentyl, 2-methylpentyl, 4-methyl-2-pentyl, 3,3-dimethylbutyl, 2-ethylbutyl, heptyl, n-heptyl, 1-methylhexyl, cyclopentylmethyl, cyclohexylmethyl, octyl, n-octyl, tert-octyl, 1-methylheptyl, 2-ethylhexyl, 2-propylpentyl, n-nonyl, 2,2-dimethylheptyl, 1-ethyl-propyl, 1,1-dimethyl-propyl, isohexyl, 4-methylhexyl, 5-methylhexyl, and the like, but are not limited thereto.

In the present disclosure, the alkenyl group may be straight-chain or branched-chain, and the carbon number thereof is not particularly limited, but is preferably 2 to 40. According to one embodiment, the carbon number of the alkenyl group is 2 to 20. According to another embodiment, the carbon number of the alkenyl group is 2 to 10. According to another embodiment, the carbon number of the alkenyl group is 2 to 6. Specific examples thereof include vinyl, 1-propenyl, isopropenyl, 1-butenyl, 2-butenyl, 3-butenyl, 1-pentenyl, 2-pentenyl, 3-pentenyl, 3-methyl-1-butenyl, 1,3-butadienyl, allyl, 1-phenylvinyl-1-yl, 2-phenylvinyl-1-yl, 2,2-diphenylvinyl-1-yl, 2-phenyl-2-(naphthyl-1-yl)vinyl-1-yl, 2,2-bis(diphenyl-1-yl)vinyl-1-yl, a stilbenyl group, a styrenyl group, and the like, but are not limited thereto.

In the present disclosure, a cycloalkyl group is not particularly limited, but the carbon number thereof is preferably 3 to 60. According to one embodiment, the carbon number of the cycloalkyl group is 3 to 30. According to another embodiment, the carbon number of the cycloalkyl group is 3 to 20. According to another embodiment, the carbon number of the cycloalkyl group is 3 to 6. Specific examples thereof include cyclopropyl, cyclobutyl, cyclopentyl, 3-methylcyclopentyl, 2,3-dimethylcyclopentyl, cyclohexyl, 3-methylcyclohexyl, 4-methylcyclohexyl, 2,3-dimethylcyclohexyl, 3,4,5-trimethylcyclohexyl, 4-tert-butylcyclohexyl, cycloheptyl, cyclooctyl, adamantyl, and the like, but are not limited thereto.

In the present disclosure, an aryl group is not particularly limited, but the carbon number thereof is preferably 6 to 60, and it may be a monocyclic aryl group or a polycyclic aryl group. According to one embodiment, the carbon number of the aryl group is 6 to 30. According to one embodiment, the carbon number of the aryl group is 6 to 20. The monocyclic aryl group includes a phenyl group, a biphenyl group, a terphenyl group and the like, but is not limited thereto. The polycyclic aryl group includes a naphthyl group, an anthracenyl group, a phenanthryl group, a pyrenyl group, a perylenyl group, a chrysenyl group, a fluorenyl group or the like, but is not limited thereto.

In the present disclosure, a fluorenyl group may be substituted, and two substituents may be bonded to each other to form a spiro structure. In the case where the fluorenyl group is substituted, 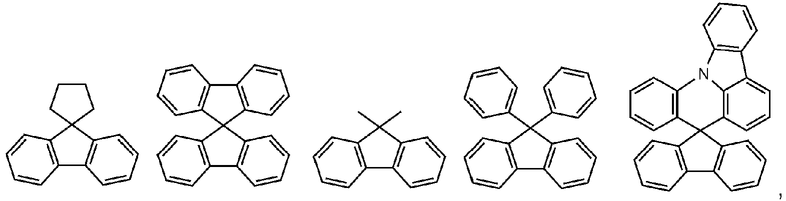 and the like can be formed. However, the structure is not limited thereto.

In the present disclosure, a heteroaryl group is a heterocyclic group containing at least one heteroatom of O, **N,** Si and S as a heterogeneous element, and the carbon number thereof is not particularly limited, but is preferably 2 to 60. Examples of the heteroaryl group include a thiophene group, a furan group, a pyrrole group, an imidazole group, a thiazole group, an oxazol group, an oxadiazol group, a triazol group, a pyridyl group, a bipyridyl group, a pyrimidyl group, a triazine group, an acridyl group, a pyridazine group, a pyrazinyl group, a quinolinyl group, a quinazoline group, a quinoxalinyl group, a phthalazinyl group, a pyridopyrimidinyl group, a pyridopyrazinyl group, a pyrazinopyrazinyl group, an isoquinoline group, an indole group, a carbazole group, a benzoxazole group, a benzoimidazole group, a benzothiazol group, a benzocarbazole group, a benzothiophene group, a dibenzothiophene group, a benzofuranyl group, a phenanthroline group, an isoxazolyl group, a thiadiazolyl group, a phenothiazinyl group, a dibenzofuranyl group, and the like, but are not limited thereto.

In the present disclosure, the aryl group in the aralkyl group, the aralkenyl group, the alkylaryl group, the arylamine group, and the arylsilyl group is the same as the aforementioned examples of the aryl group. In the present disclosure, the alkyl group in the aralkyl group, the alkylaryl group and the alkylamine group is the same as the aforementioned examples of the alkyl group. In the present disclosure, the heteroaryl in the heteroarylamine can apply the aforementioned description of the heteroaryl. In the present disclosure, the alkenyl group in the aralkenyl group is the same as the aforementioned examples of the alkenyl group. In the present disclosure, the aforementioned description of the aryl group may be applied except that the arylene is a divalent group. In the present disclosure, the aforementioned description of the heteroaryl can be applied except that the heteroarylene is a divalent group. In the present disclosure, the aforementioned description of the aryl group or cycloalkyl group can be applied except that the hydrocarbon ring is not a monovalent group but formed by combining two substituent groups. In the present disclosure, the aforementioned description of the heteroaryl can be applied, except that the heterocycle is not a monovalent group but formed by combining two substituent groups.

As used herein, the term "deuterated or substituted with deuterium" means that at least one available hydrogen in each Chemical Formula is substituted with deuterium (D). Specifically, "substituted with deuterium" in the definition of each Chemical Formula or substituent means that at least one position at which hydrogen can be bonded in the molecule is substituted with deuterium. More specifically, it means that at least 10% of the available hydrogen is substituted with deuterium. For example, at least 20%, at least 30%, at least 40%, at least 50%, at least 60%, at least 70%, at least 80%, at least 90%, or 100% are deuterated in each Chemical Formula.

Meanwhile, the organic light emitting device according to an embodiment includes an anode; a cathode that is provided opposite to the anode; and a light emitting layer that is provided between the anode and the cathode, wherein the light emitting layer includes a first compound represented by the Chemical Formula 1 and a second compound represented by the Chemical Formula 2 as host materials of the light emitting layer.

The organic light emitting device according to the present disclosure includes two types of compounds having a specific structure as host materials in the light emitting layer at the same time, thereby improving efficiency, driving voltage, and/or lifespan of the organic light emitting device.

Hereinafter, the present invention will be described in detail for each configuration.

### Anode and cathode

As the anode material, generally, a material having a large work function is preferably used so that holes can be smoothly injected into the organic material layer. Specific examples of the anode material include metals such as vanadium, chrome, copper, zinc, and gold, or an alloy thereof; metal oxides such as zinc oxides, indium oxides, indium tin oxides (ITO), and indium zinc oxides (IZO); a combination of metals and oxides such as ZnO:Al or SnO₂:Sb; conductive polymers such as poly(3-methylthiophene), poly[3,4-(ethylene-1,2-dioxy)thiophene](PEDOT), polypyrrole, and polyaniline, and the like, but are not limited thereto.

As the cathode material, generally, a material having a small work function is preferably used so that electrons can be easily injected into the organic material layer. Specific examples of the cathode material include metals such as magnesium, calcium, sodium, potassium, titanium, indium, yttrium, lithium, gadolinium, aluminum, silver, tin, and lead, or an alloy thereof; a multilayered structure material such as LiF/Al or LiO₂/Al, and the like, but are not limited thereto.

### Hole injection layer

The organic light emitting device according to the present disclosure may include a hole injection layer between an anode and a hole transport layer to be described later, if necessary.

The hole injection layer located on the anode is a layer for injecting holes from the anode, and includes a hole injection material. The hole injection material is preferably a compound which can transport the holes, thus has a hole-injecting effect in the anode and an excellent hole-injecting effect to the light emitting layer or the light emitting material, prevents excitons produced in the light emitting layer from moving to an electron injection layer or the electron injection material, and is excellent in the ability to form a thin film. It is preferable that a HOMO (highest occupied molecular orbital) of the hole injection material is between the work function of the anode material and a HOMO of a peripheral organic material layer.

Specific examples of the hole injection material include metal porphyrine, oligothiophene, an arylamine-based organic material, a hexanitrilehexaazatriphenylene-based organic material, a quinacridone-based organic material, a perylene-based organic material, anthraquinone, polyaniline and polythiophene-based conductive polymer, and the like, but are not limited thereto.

### Hole transport layer

The organic light emitting device according to the present disclosure may include a hole transport layer between an anode and a light emitting layer. The hole transport layer is a layer that receives holes from an anode or a hole injection layer formed on the anode and transports the holes to the light emitting layer, and includes a hole transport material. The hole transport material is suitably a material having large mobility to the holes, which may receive holes from the anode or the hole injection layer and transfer the holes to the light emitting layer. Specific examples thereof include an arylamine-based organic material, a conductive polymer, a block copolymer in which a conjugate portion and a non-conjugate portion are present together, and the like, but are not limited thereto.

### Electron blocking layer

The organic light emitting device according to the present disclosure may include an electron blocking layer between a hole transport layer and a light emitting layer, if necessary. The electron blocking layer is a layer which is formed on the hole transport layer, is preferably provided in contact with the light emitting layer, and thus serves to control hole mobility, to prevent excessive movement of electrons, and to increase the probability of hole-electron bonding, thereby improving the efficiency of the organic light emitting device. The electron blocking layer includes an electron blocking material, and an arylamine-based organic material may be used as the electron blocking material, but is not limited thereto.

### Light emitting layer

The organic light emitting device according to the present disclosure may include a light emitting layer between an anode and a cathode, and the light emitting layer includes the first compound and the second compound as host materials. Specifically, the first compound and the second compound are used in combination, thereby maintaining the ratio of holes to electrons in the light emitting layer. In particular, when the first compound and the second compound are used at the same time, holes and electrons are smoothly transferred to the dopant, thereby improving the efficiency and lifespan of the device. Accordingly, the above device may exhibit low voltage and long lifespan compared to devices using only a single compound among the first compound and the second compound or including a combination of other compounds.

Hereinafter, the first compound and the second compound will be described.

### (First compound)

The first compound is represented by the following Chemical Formula 1. Specifically, the first compound is a compound having carbazolyl and triazine-based (pyridine, pyrimidine) substituents on triphenylenyl, can efficiently transfer electrons to a host material, and thus can increase the probability of hole-electron recombination in the light emitting layer together with a second compound to be described later. 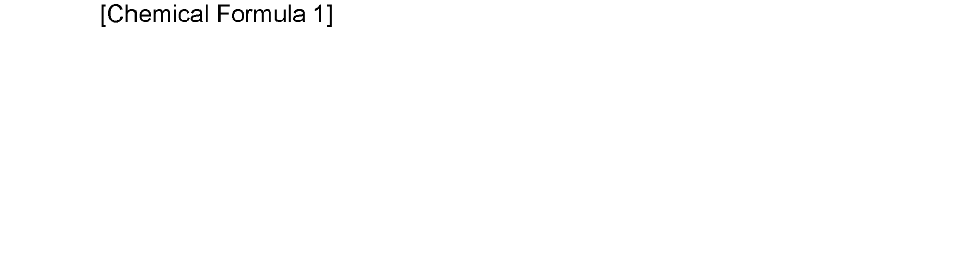 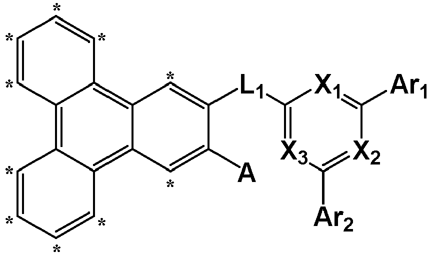 in the Chemical Formula 1,
A is substituted or unsubstituted carbazolyl,
L₁ is a single bond, or substituted or unsubstituted C₆₋₆₀ arylene,
X₁, X₂ and X₃ are each independently N, or CH, provided that at least one of X₁, X₂ and X₃ is N, and
Ar₁ and Ar₂ are each independently substituted or unsubstituted C₆₋₆₀ aryl, or substituted or unsubstituted C₂₋₆₀ heteroaryl containing at least one heteroatom selected from the group consisting of N, O and S.

Meanwhile, in the Chemical Formula 1, the carbon with * means that only hydrogen is substituted, and does not include an additional substituent.

The first compound may be represented by the following Chemical Formula 1-1 or Chemical Formula 1-2, depending on the substitution position of A: 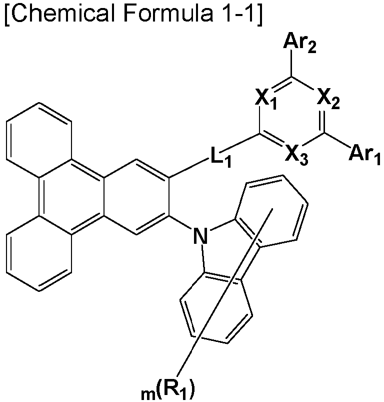 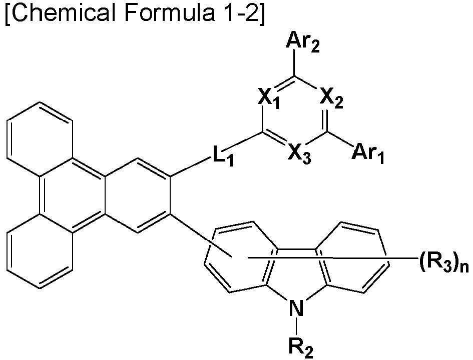 in the Chemical Formula 1-1 or Chemical Formula 1-2,
L₁, X₁, X₂, X₃, Ar₁, and Ar₂ are as defined above,
R₁ are each independently hydrogen, deuterium, substituted or unsubstituted C₁₋₆₀ alkyl, substituted or unsubstituted C₆₋₆₀ aryl, or substituted or unsubstituted C₂₋₆₀ heteroaryl containing at least one heteroatom selected from the group consisting of N, O and S,
R₂ is substituted or unsubstituted C₁₋₆₀ alkyl, or substituted or unsubstituted C₆₋₆₀ aryl,
R₃ are each independently hydrogen, deuterium, substituted or unsubstituted C₁₋₆₀ alkyl, substituted or unsubstituted C₆₋₆₀ aryl, or substituted or unsubstituted C₅₋₆₀ heteroaryl containing at least one heteroatom selected from the group consisting of N, O and S,
m is an integer of 0 to 8, and
n is an integer of 0 to 7.

Herein, m representing the number of R₁ is 0, 1, 2, 3, 4, 5, 6, 7, or 8, and n representing the number of R₃ is 0, 1, 2, 3, 4, 5, 6, or 7.

Preferably, L₁ is a single bond, phenylene or biphenylylene, and the phenylene and biphenylylene may each independently be unsubstituted or substituted with at least one deuterium.

Preferably, Ar₁ and Ar₂ are each independently phenyl, biphenylyl, terphenylyl, dimethylfluorenyl, naphthyl, phenanthrenyl, triphenylenyl, dibenzofuranyl, dibenzothiophenyl, carbazol-9-yl, 9-phenyl-9H-carbazolyl, benzoxazolyl, benzothiazolyl, 2-phenylbenzoxazolyl or 2-phenylbenzothiazolyl, and they may each independently be unsubstituted or substituted with at least one deuterium.

Preferably, R₁ are each independently hydrogen, deuterium, phenyl, dibenzofuranyl, dibenzothiophenyl, or carbazolyl, and the phenyl, dibenzofuranyl, dibenzothiophenyl, and carbazolyl may each independently be unsubstituted or substituted with at least one deuterium.

Preferably, R₂ may each independently be phenyl unsubstituted or substituted with at least one deuterium.

Preferably, R₃ are each independently hydrogen, deuterium, phenyl, dibenzofuranyl, dibenzothiophenyl, or carbazolyl, and the phenyl, dibenzofuranyl, dibenzothiophenyl, and carbazolyl may each independently be unsubstituted or substituted with at least one deuterium.

Representative examples of the first compound represented by the Chemical Formula 1 are as follows:

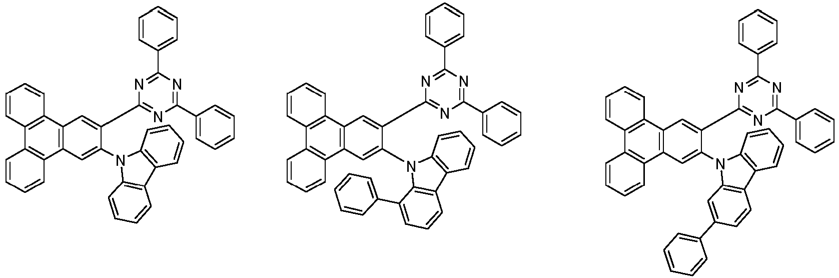

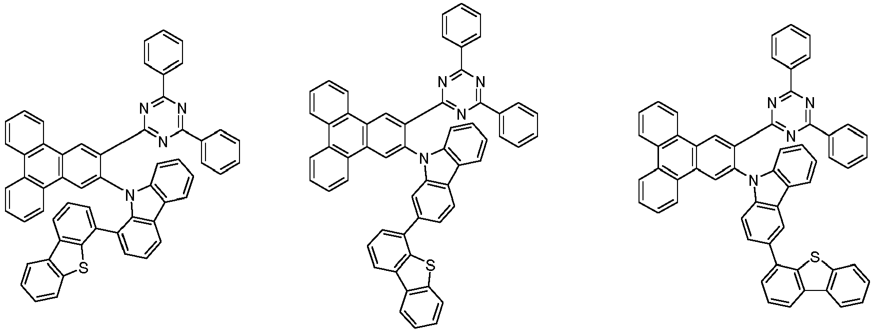

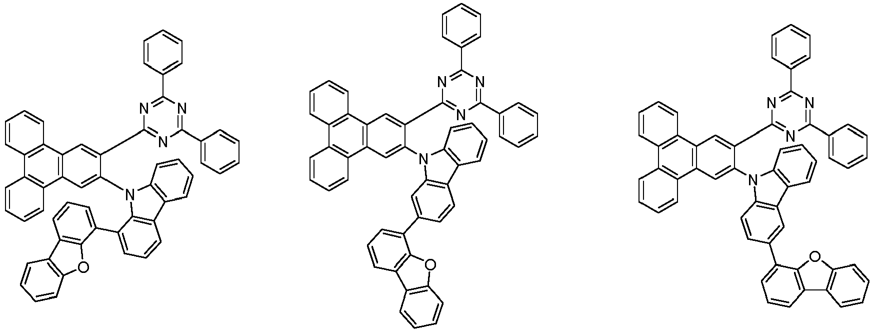

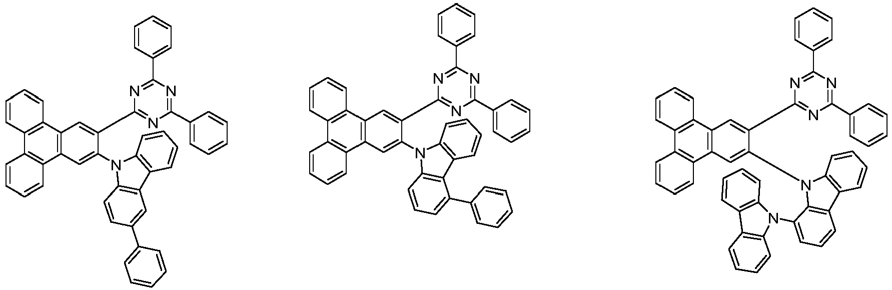

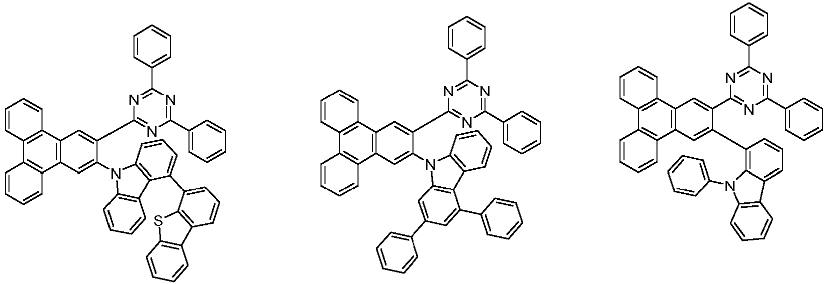

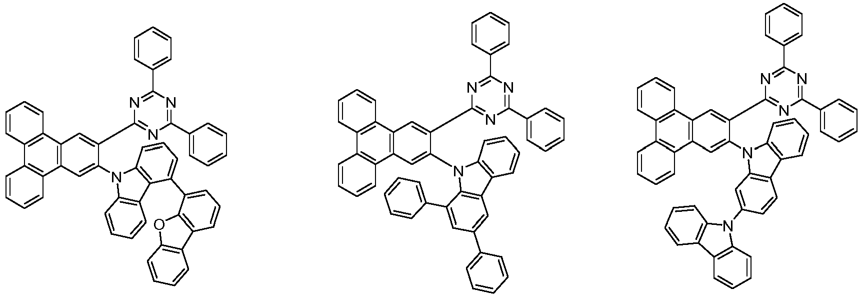

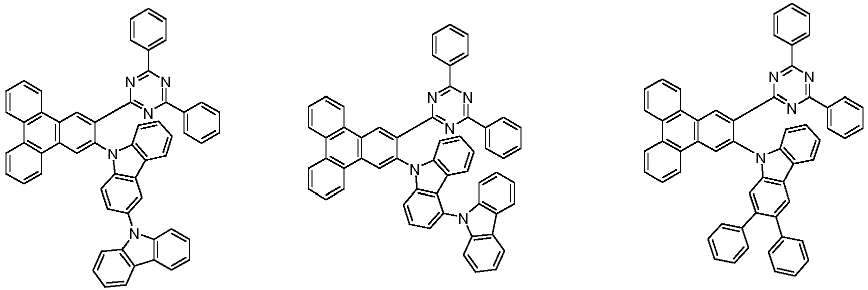

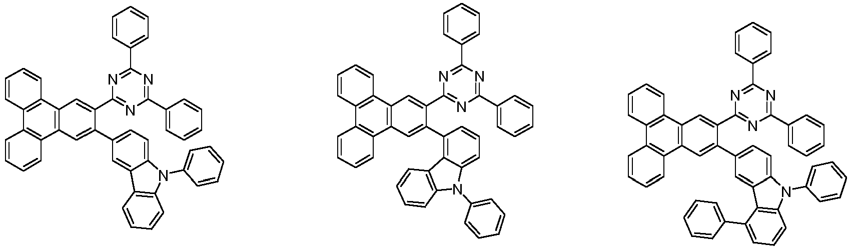

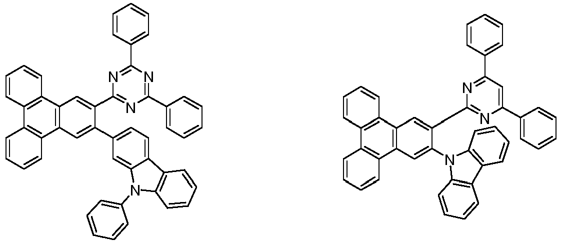

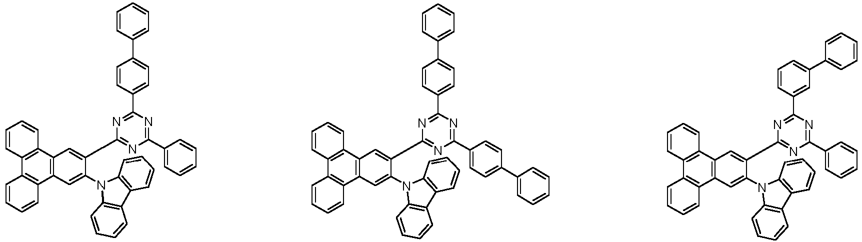

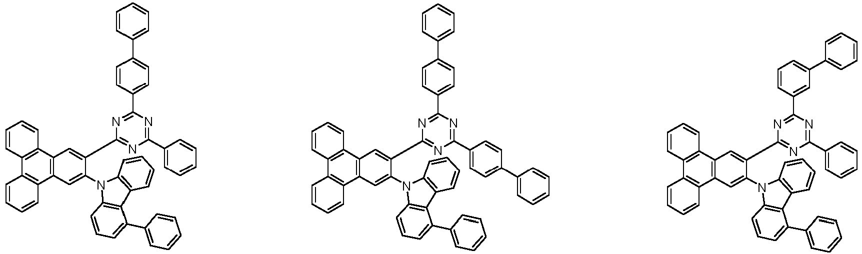

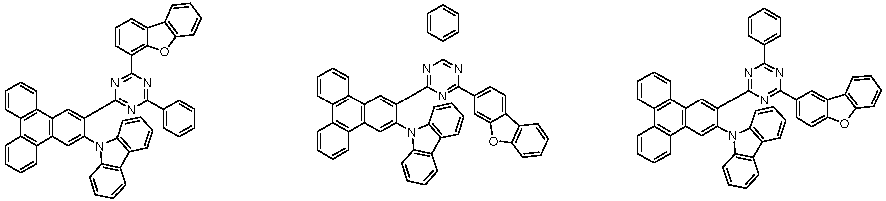

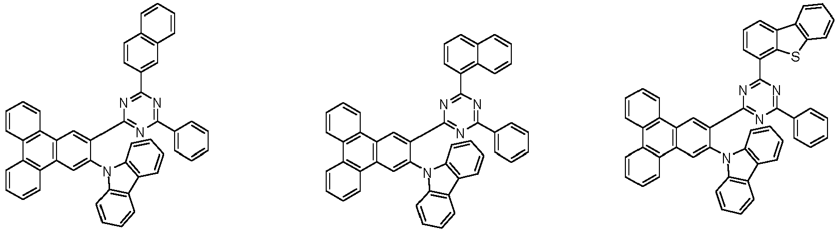

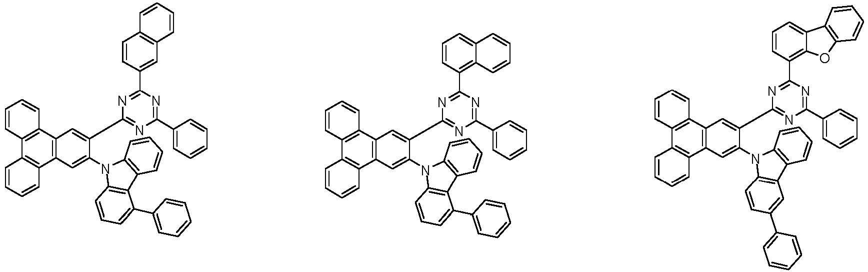

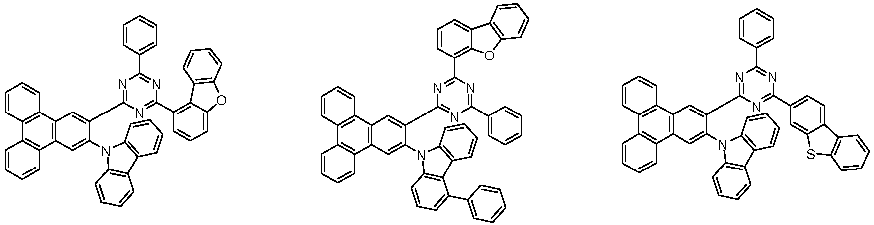

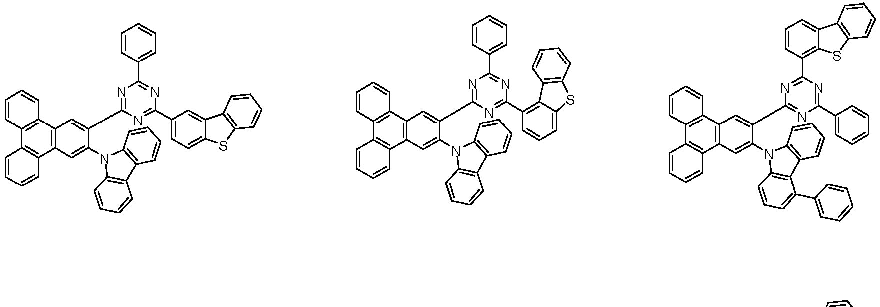

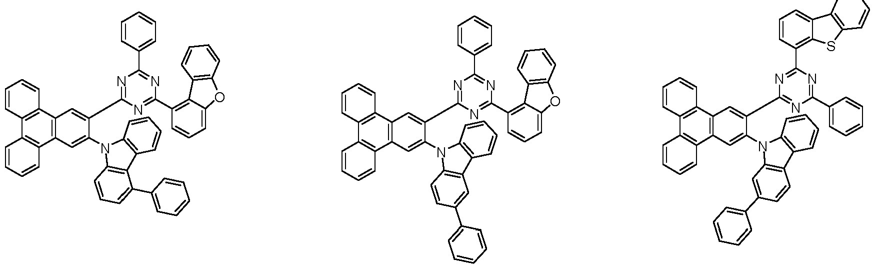

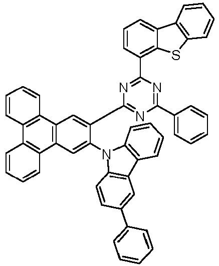

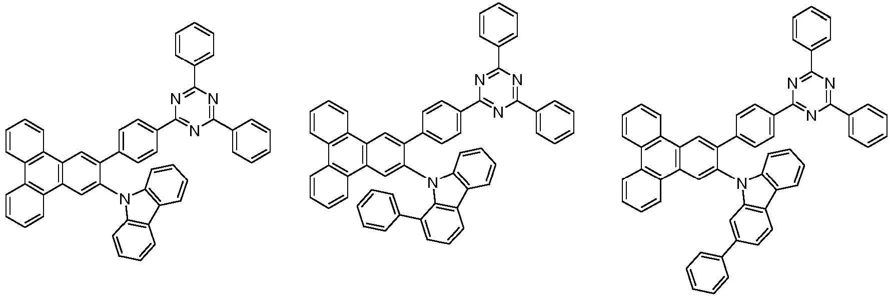

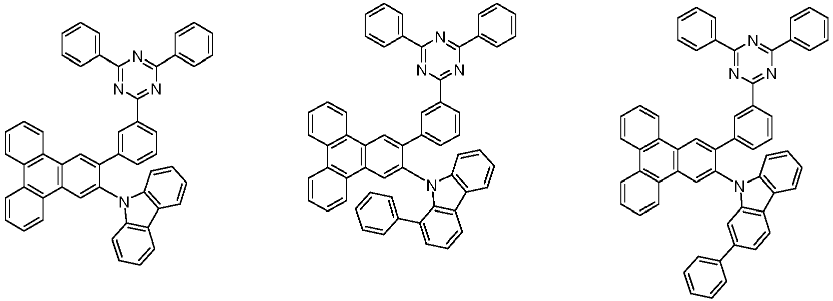

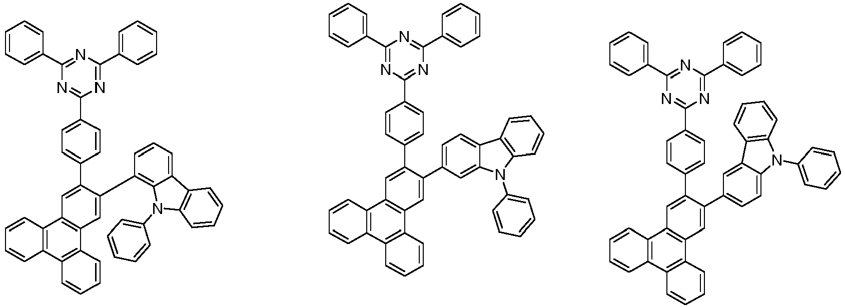

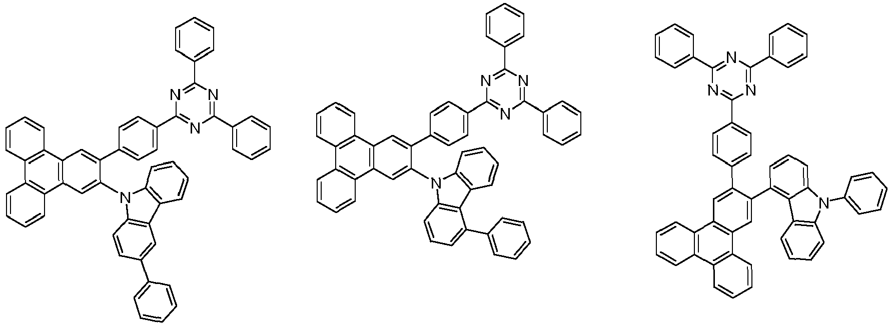

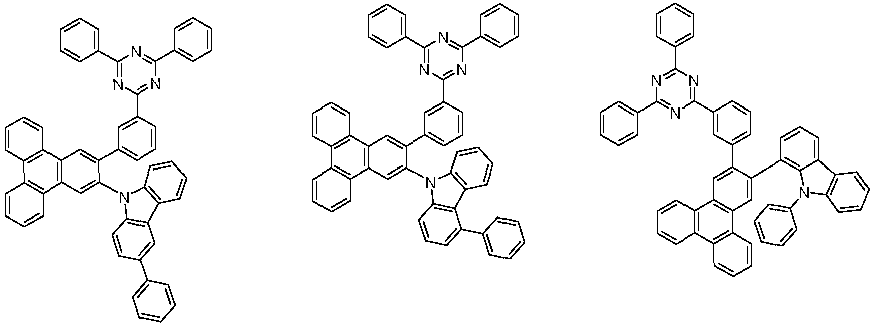

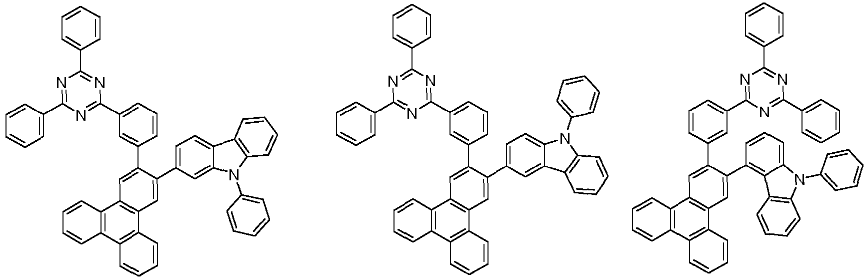

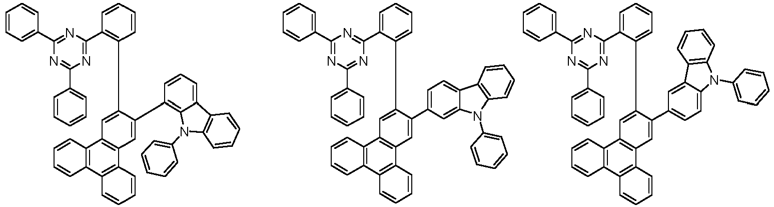

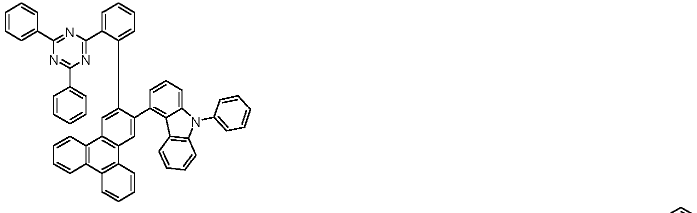

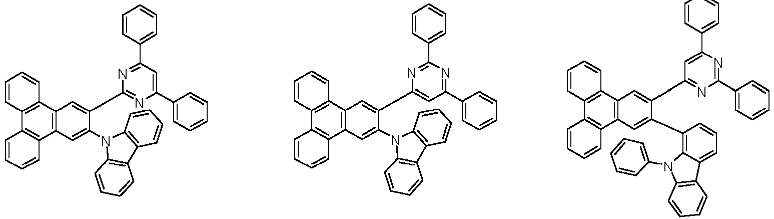

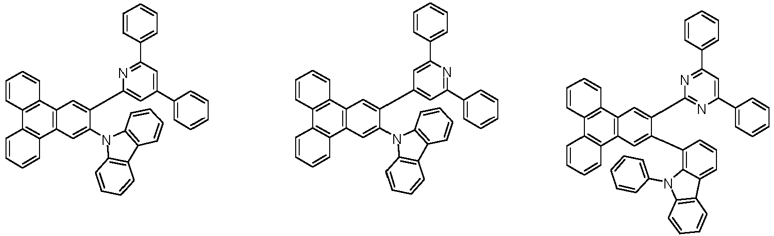

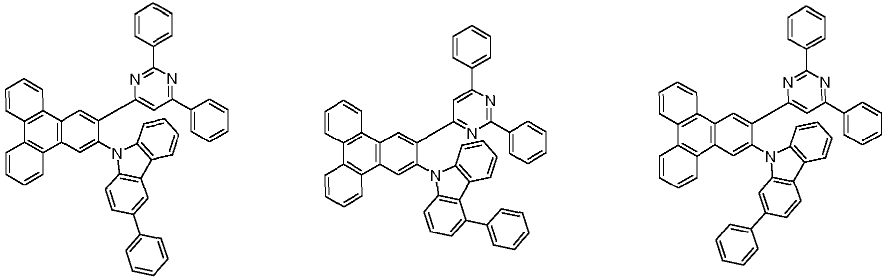

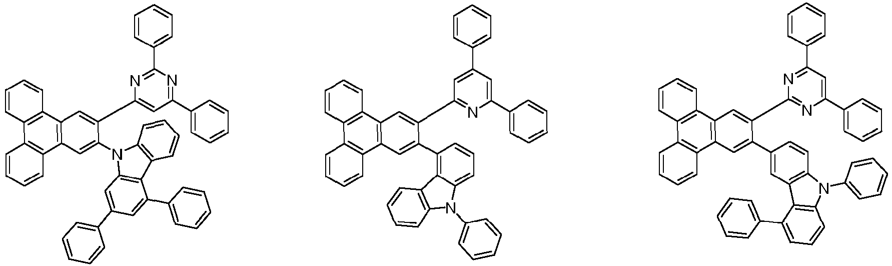

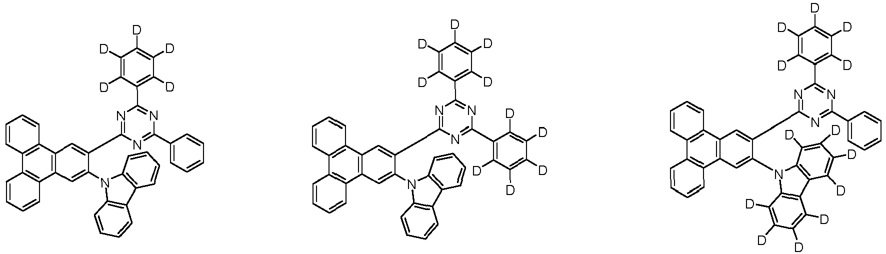

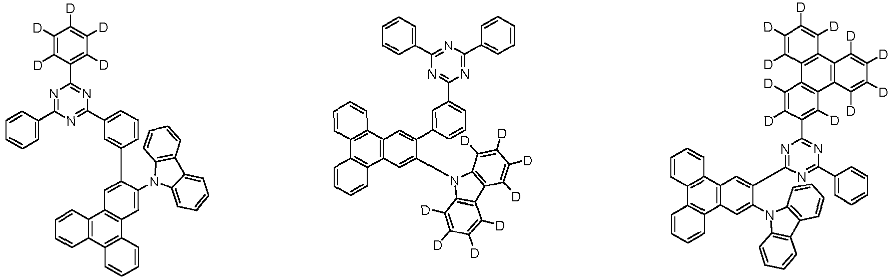

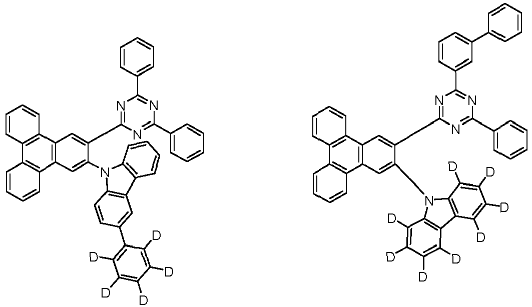

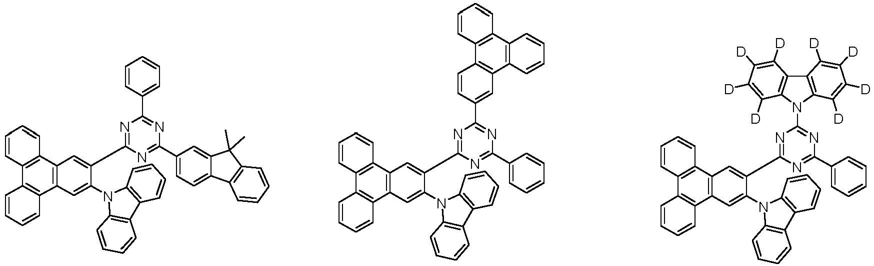

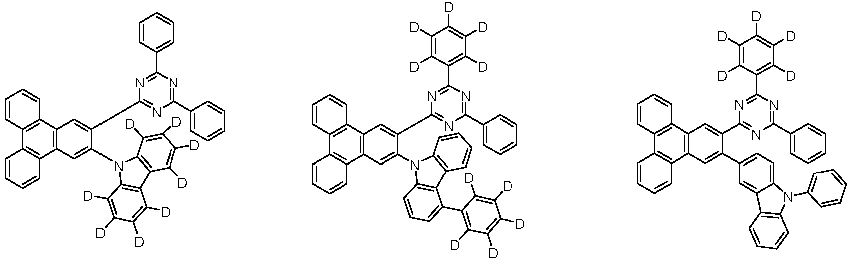

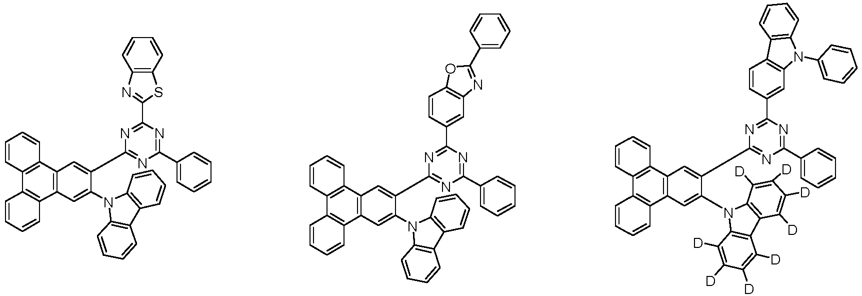

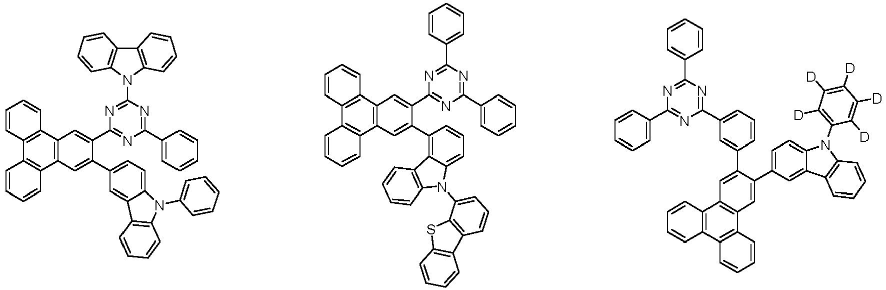

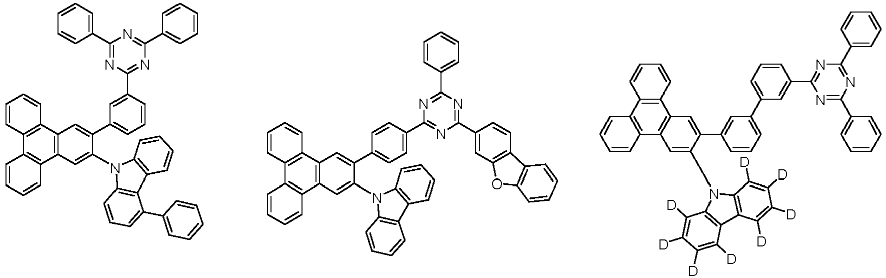

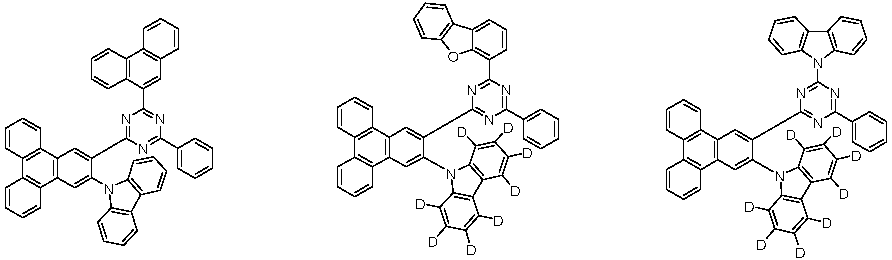

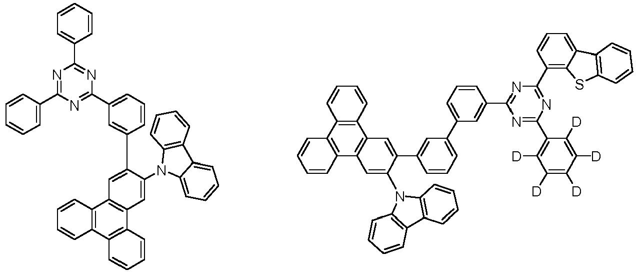

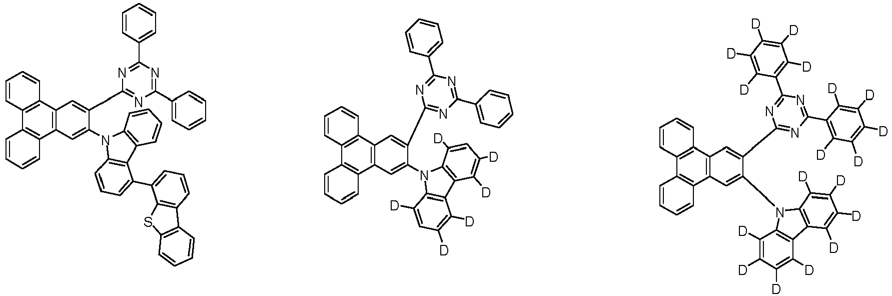

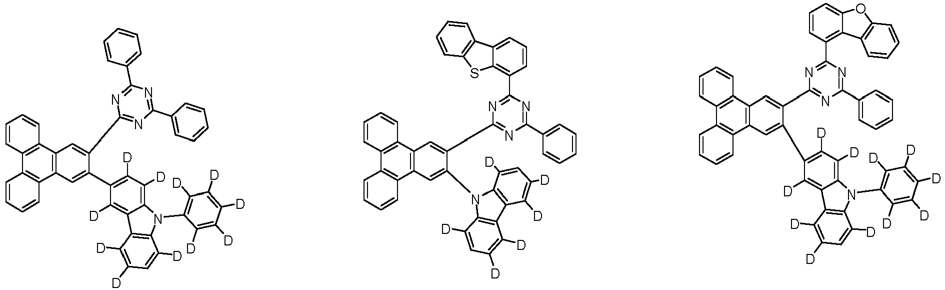

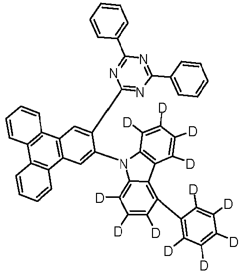

Meanwhile, the first compound may be prepared by, for example, a preparation method as shown in Reaction Schemes 1-A and 1-B below.

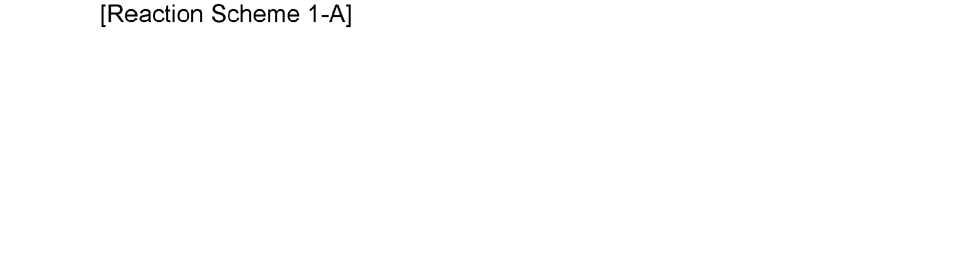

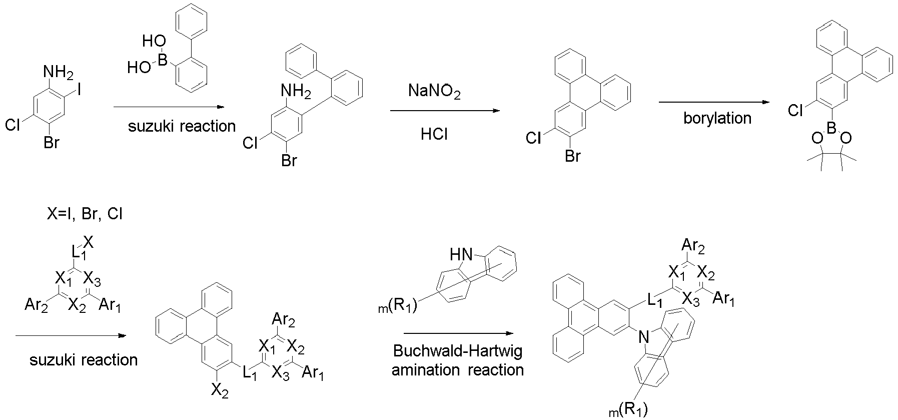

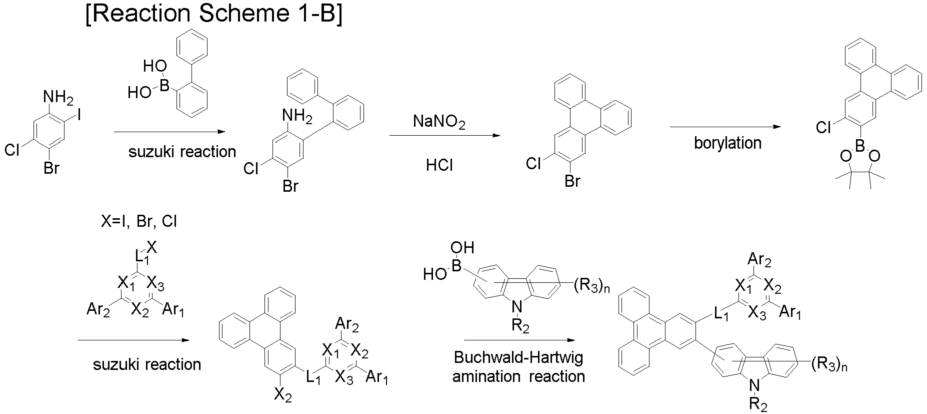

In the Reaction Schemes 1-A and 1-B, each X is independently halogen, preferably bromo, or chloro, and the definitions of other substituents are the same as described above.

Specifically, the compound represented by the Chemical Formula 1 is prepared by a Suzuki coupling reaction and an amine substitution reaction, and these reactions are preferably performed in the presence of a palladium catalyst and a base. In addition, the reactive group for these reactions may be appropriately changed, and the method for preparing the compound represented by the Chemical Formula 1 may be more specifically described in Synthesis Examples described below.

### (Second compound)

The second compound is represented by the following Chemical Formula 2. Specifically, the second compound is an indolocarbazole compound, can efficiently transfer holes to a dopant material, and thus can increase the probability of hole-electron recombination in the light emitting layer together with the first compound described above. 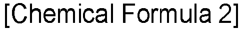 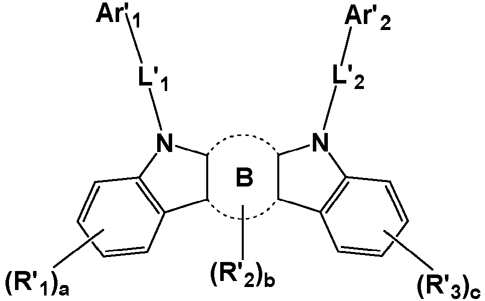 in the Chemical Formula 2,
B is a benzene ring fused with two adjacent pentagonal rings,
L'₁ and L'₂ are each independently a single bond, or substituted or unsubstituted C₆₋₆₀ arylene,
Ar'₁ and Ar'₂ are each independently substituted or unsubstituted C₆₋₆₀ aryl, or substituted or unsubstituted C₂₋₆₀ heteroaryl containing at least one heteroatom selected from the group consisting of N, O and S,
R'₁, R'₂ and R'₃ are each independently hydrogen, deuterium, substituted or unsubstituted C₆₋₆₀ aryl, or substituted or unsubstituted C₂₋₆₀ heteroaryl containing at least one heteroatom selected from the group consisting of N, O and S,
a is an integer of 0 to 4,
b is an integer of 0 to 2, and
c is an integer of 0 to 4.

The second compound may be represented by any one of the following Chemical Formulae 2-1 to 2-5, depending on the fused position of B: 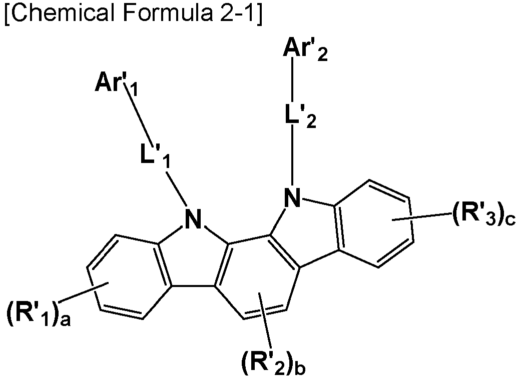 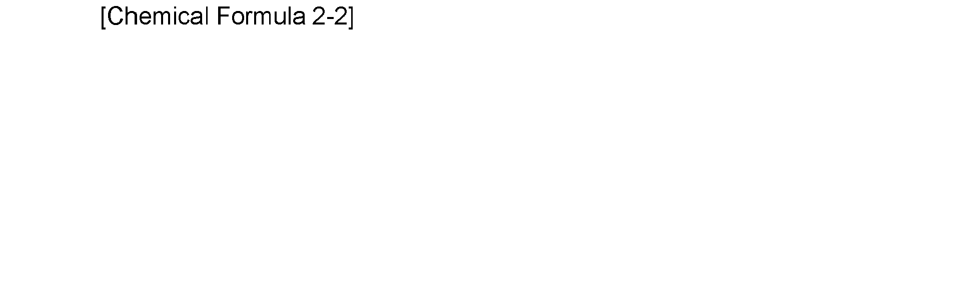 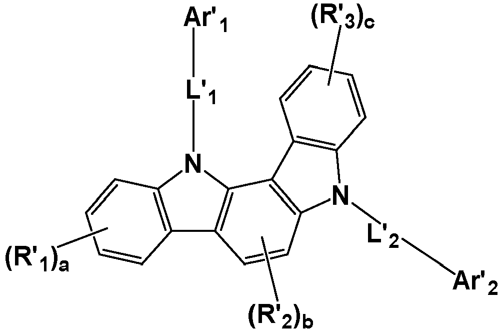 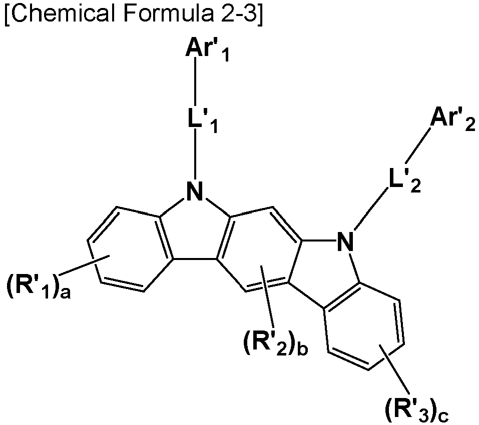 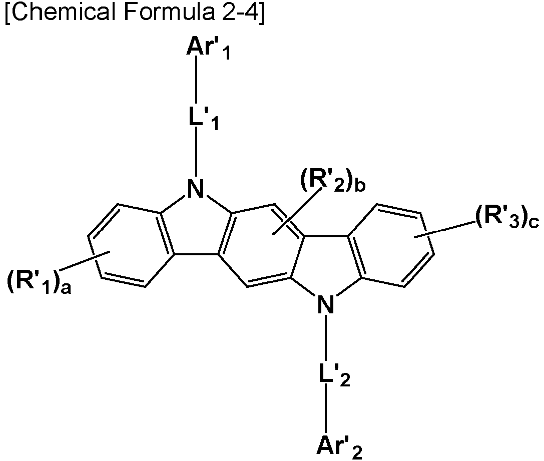 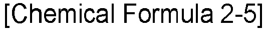 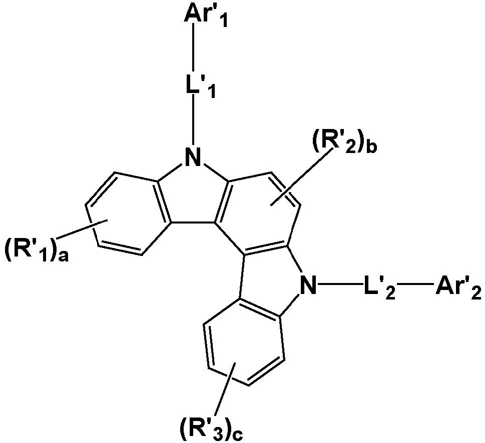 in the Chemical Formulae 2-1 to 2-5,
L'₁, L'₂, Ar'₁, Ar'₂, R'₁, R'₂, R'₃, a, b and c are as describe above.

Preferably, L'₁ and L'₂ are each independently a single bond, phenylene or biphenylylene, and the phenylene or biphenylylene may each independently be unsubstituted or substituted with at least one deuterium.

Preferably, Ar'₁ and Ar'₂ are each independently phenyl, biphenylyl, terphenylyl, naphthyl, phenanthrenyl, triphenylenyl, fluorenyl, dibenzofuranyl, dibenzothiophenyl, carbazole-9-yl, or 9-phenyl-9H-carbazolyl, and Ar'₁ and Ar'₂ may each independently be unsubstituted or substituted with at least one substituent selected from the group consisting of deuterium, C₁₋₁₀ alkyl and C₆₋₂₀ aryl.

Preferably, R'₁, R'₂ and R'₃ are each independently hydrogen, deuterium, phenyl, dibenzofuranyl, dibenzothiophenyl, carbazol-9-yl, or 9-phenyl-9H-carbazolyl, and the phenyl, dibenzofuranyl, carbazol-9-yl, and 9-phenyl-9H-carbazolyl may each independently be unsubstituted or substituted with at least one deuterium.

Herein, a representing the number of R'₁ is 0, 1, 2, 3, or 4, b representing the number of R'₂ is 0, 1, or 2, and c representing the number of R'₃ is 0, 1, 2, 3, or 4.

Representative examples of the second compound represented by the Chemical Formula 2 are as follows:

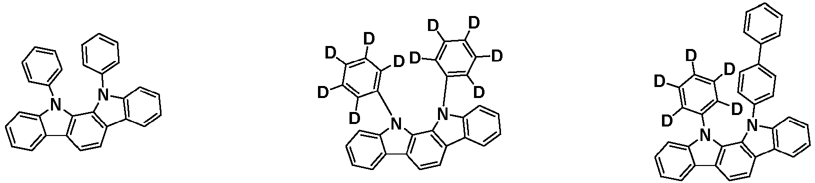

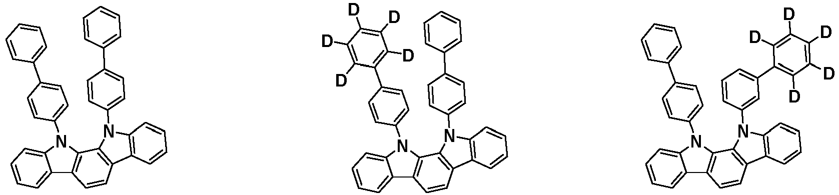

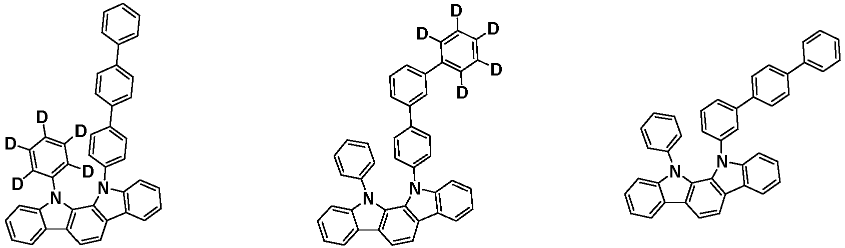

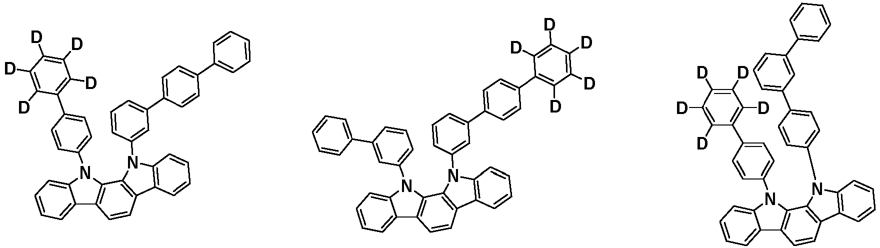

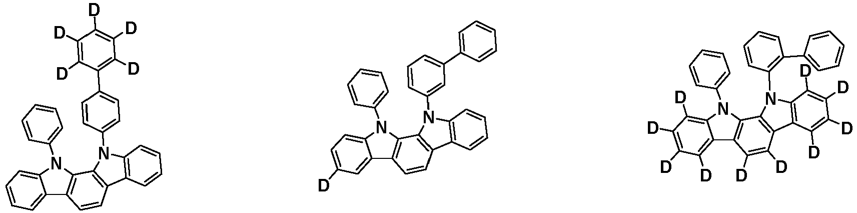

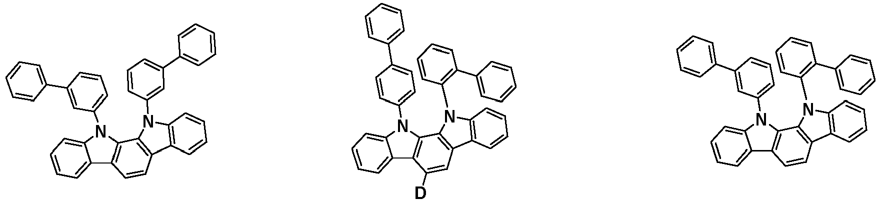

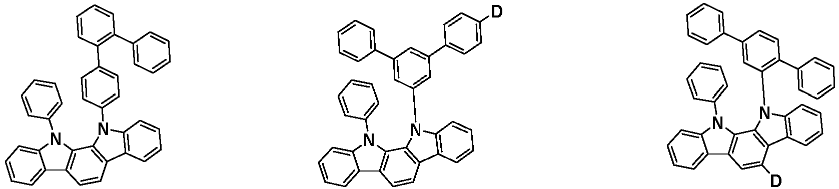

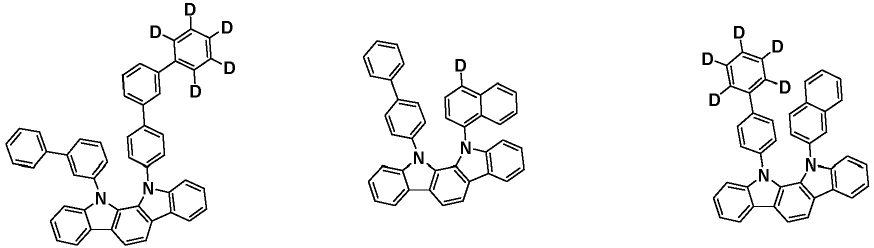

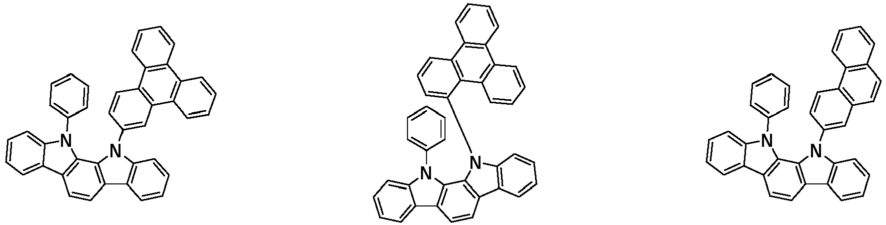

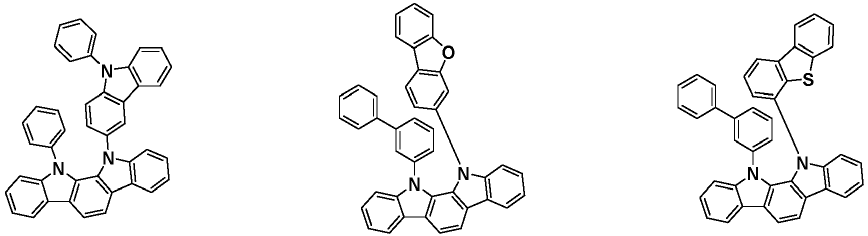

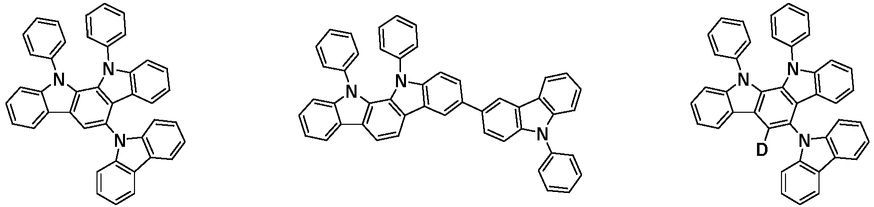

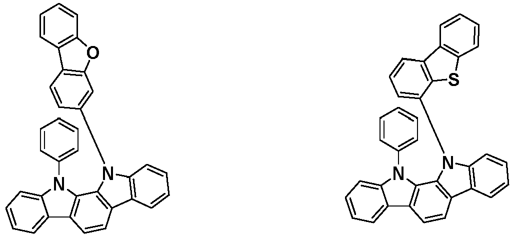

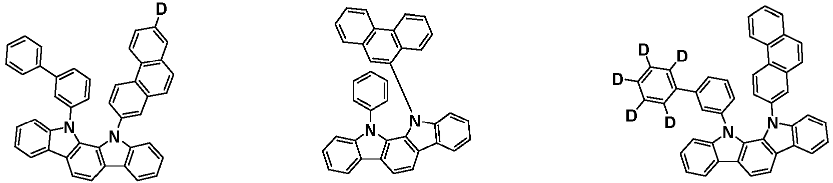

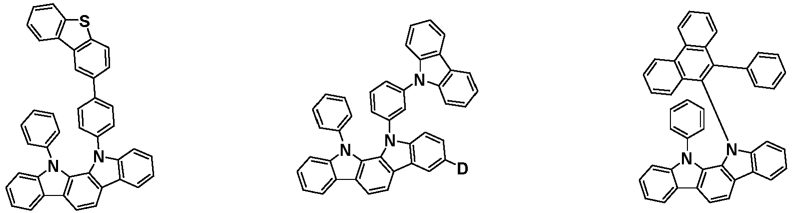

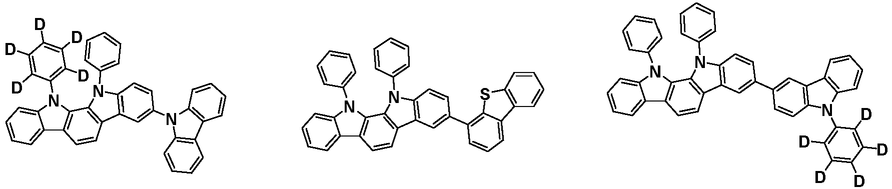

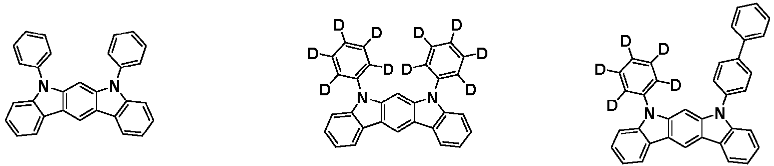

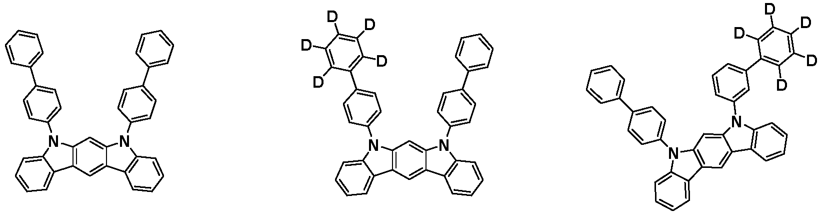

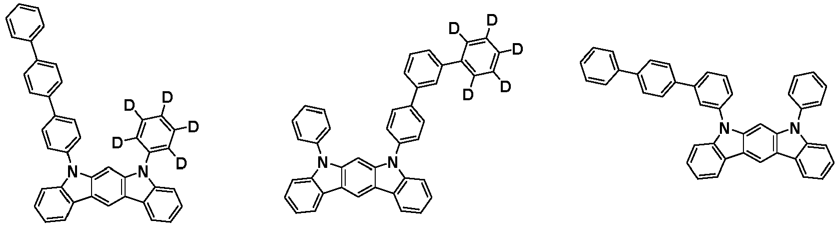

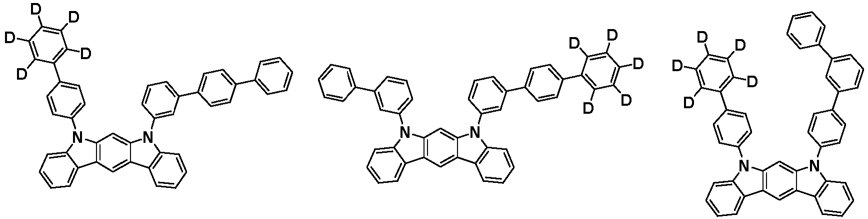

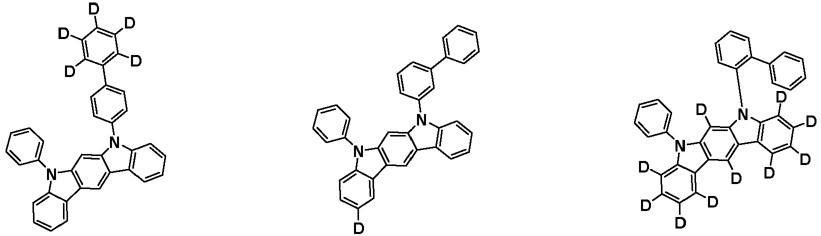

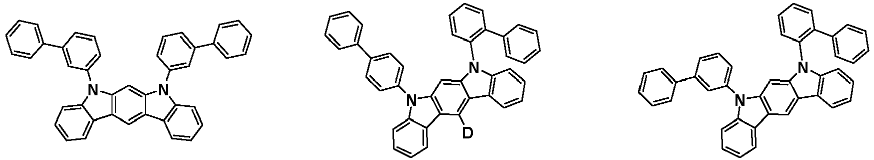

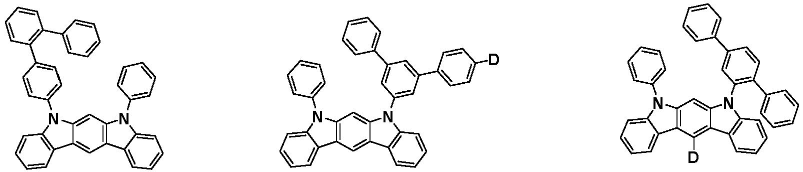

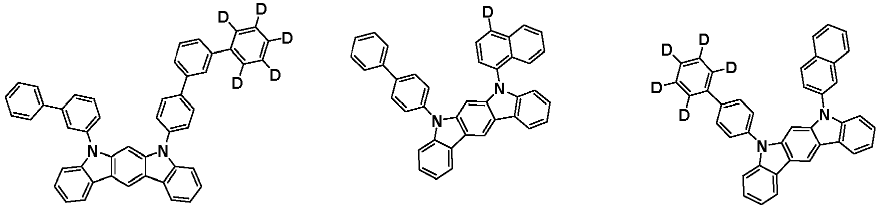

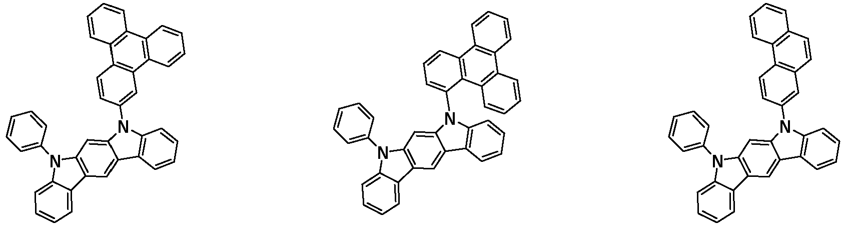

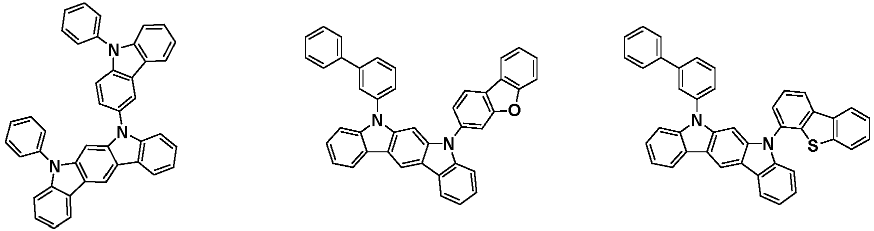

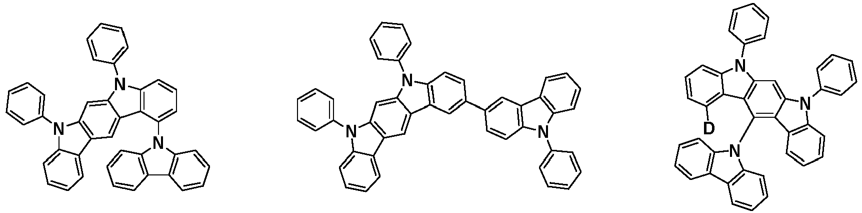

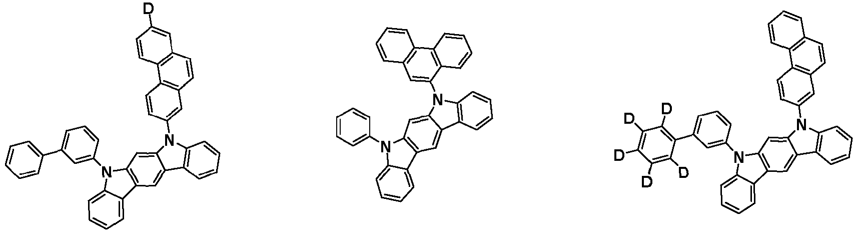

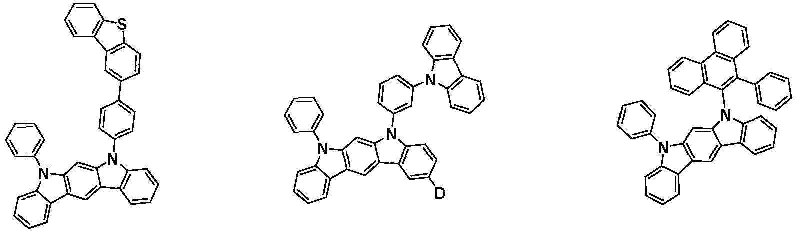

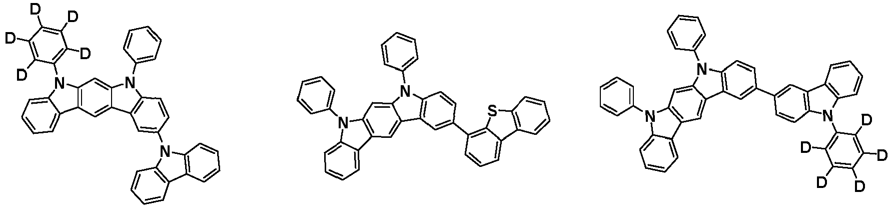

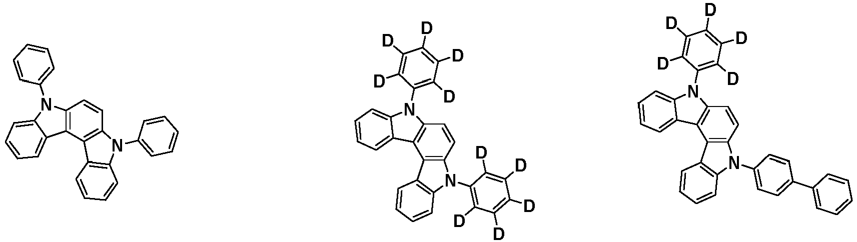

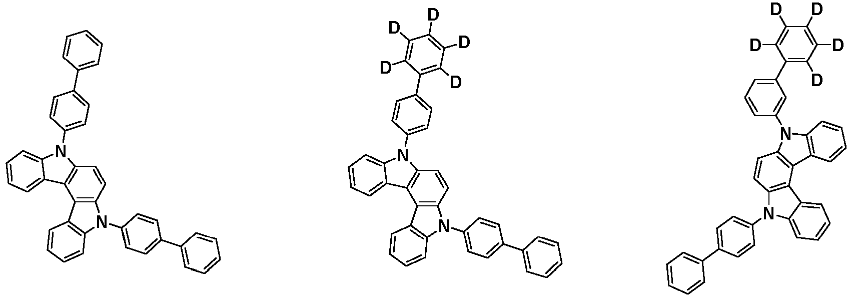

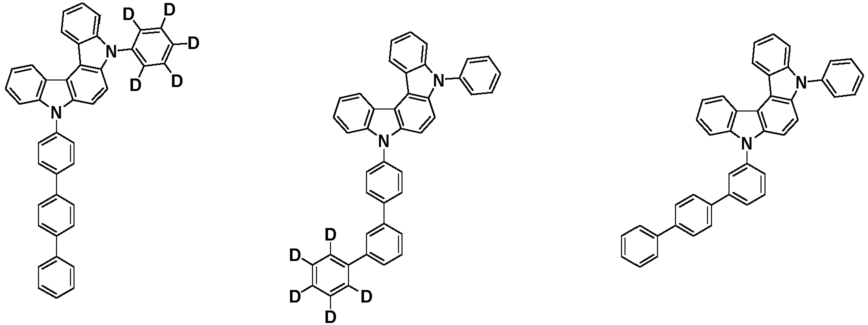

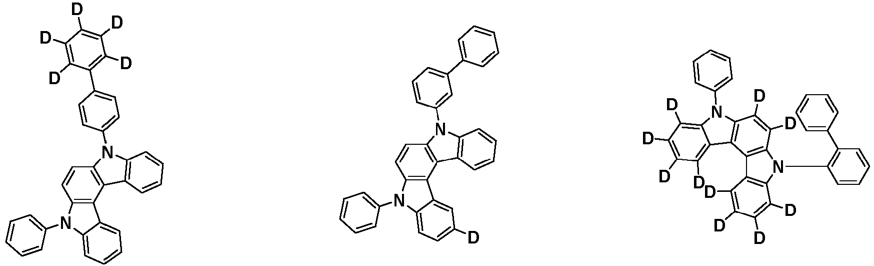

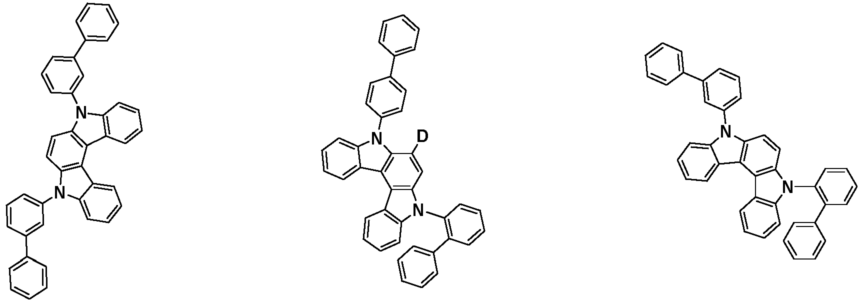

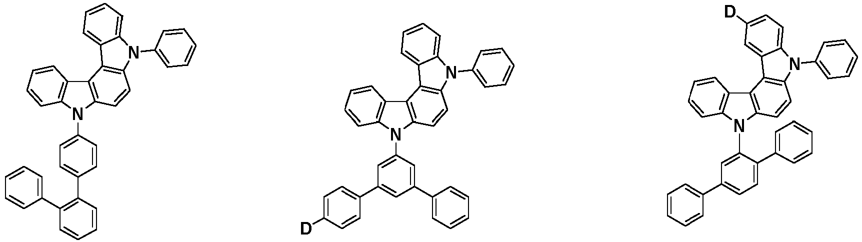

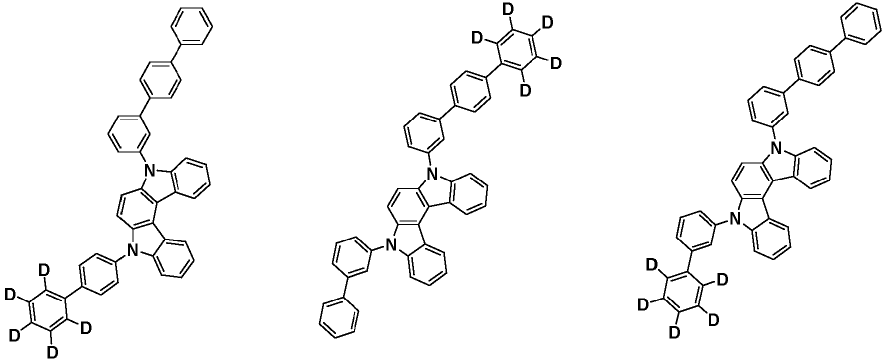

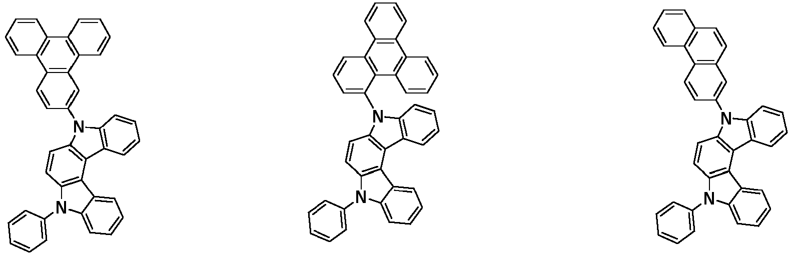

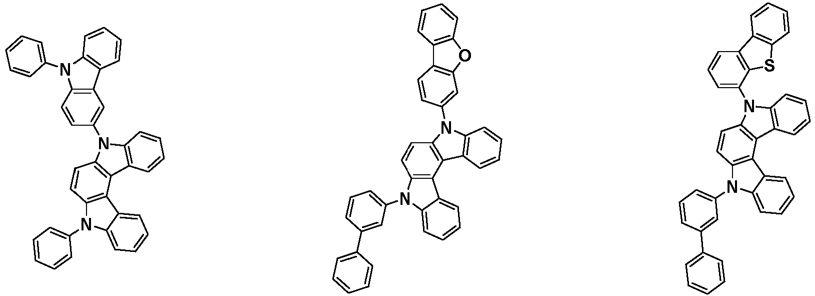

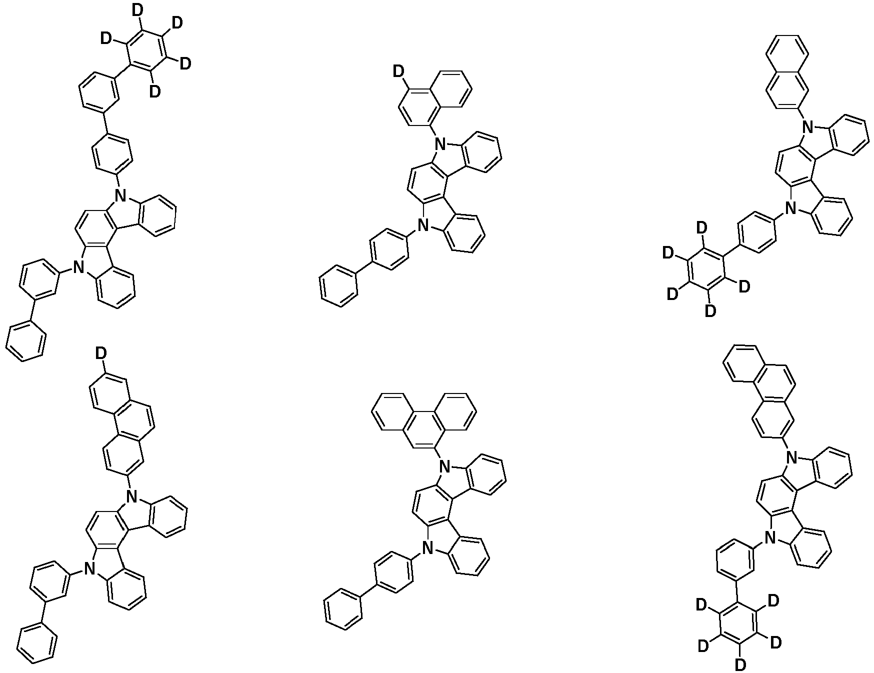

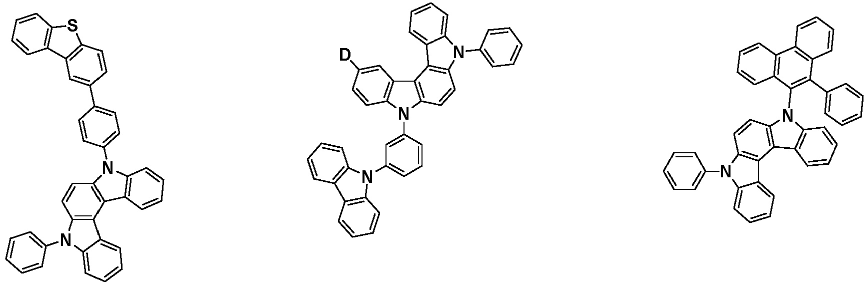

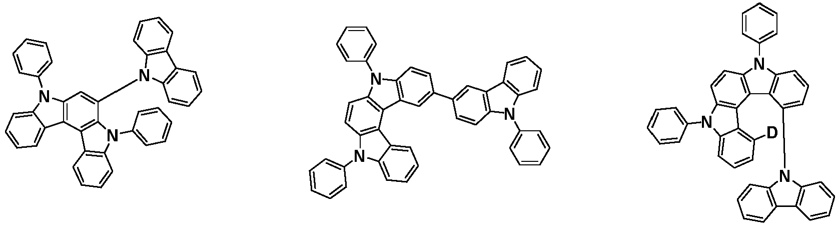

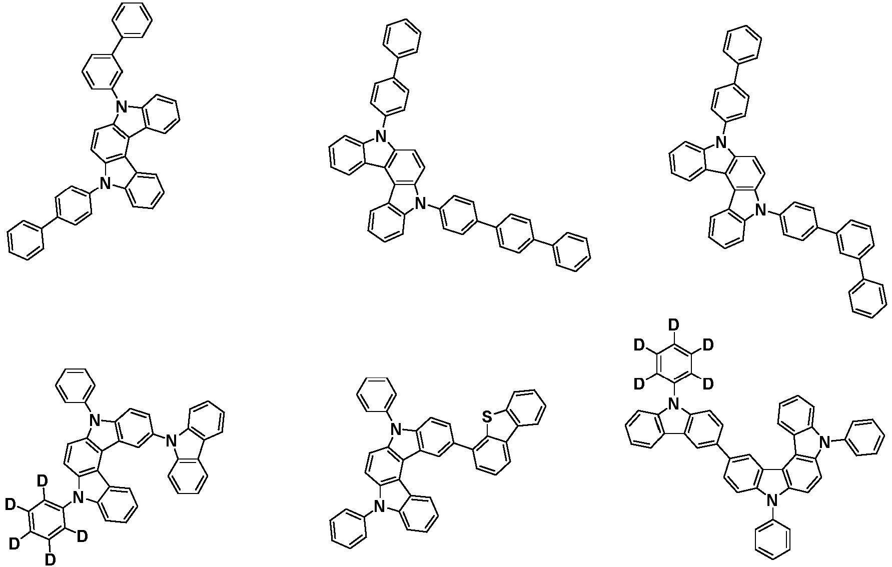

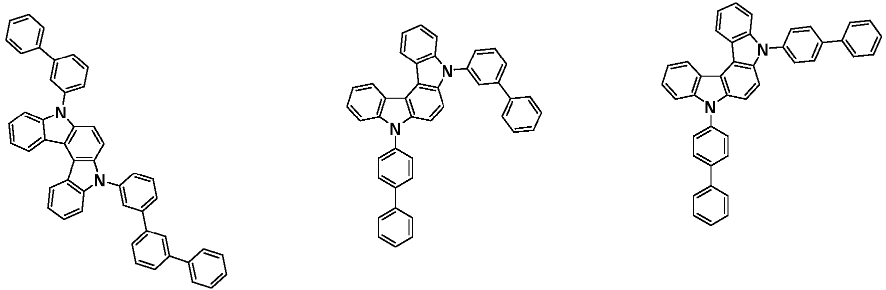

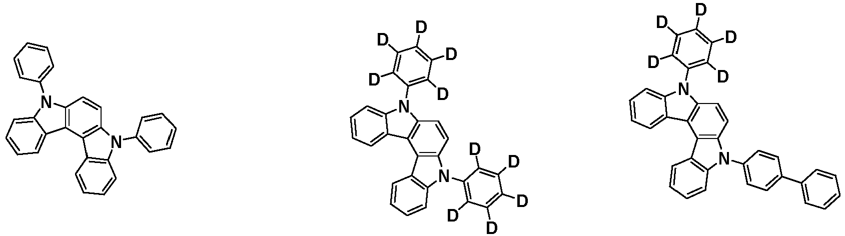

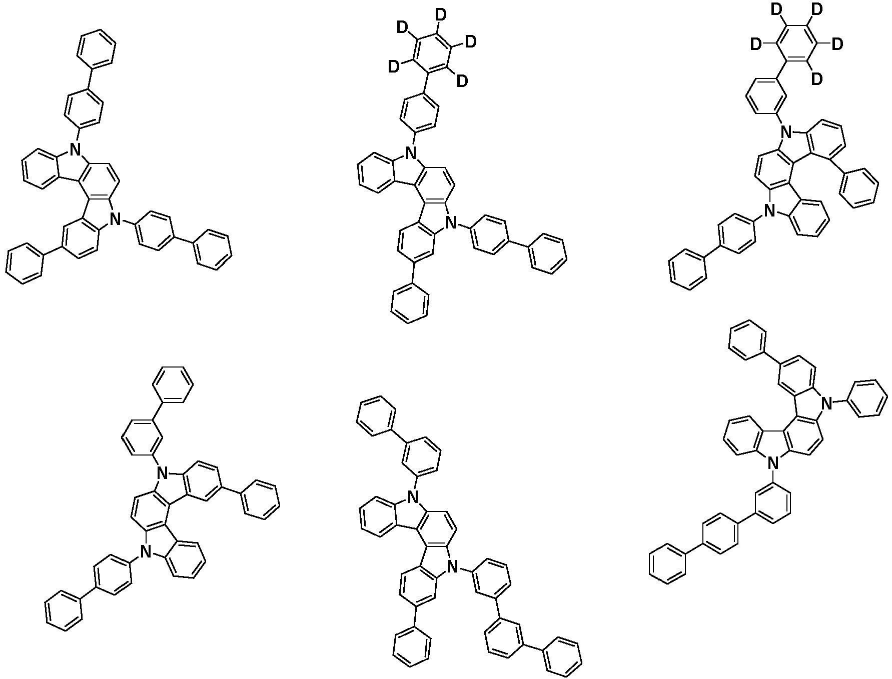

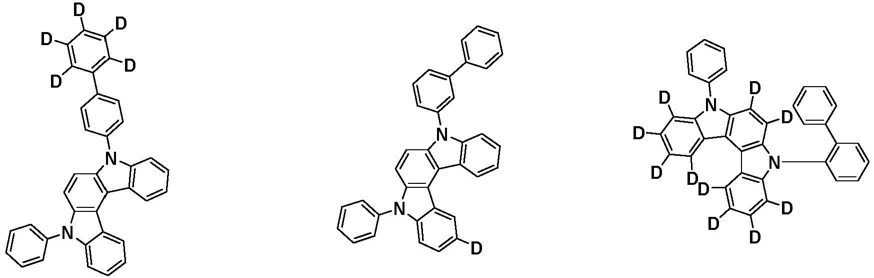

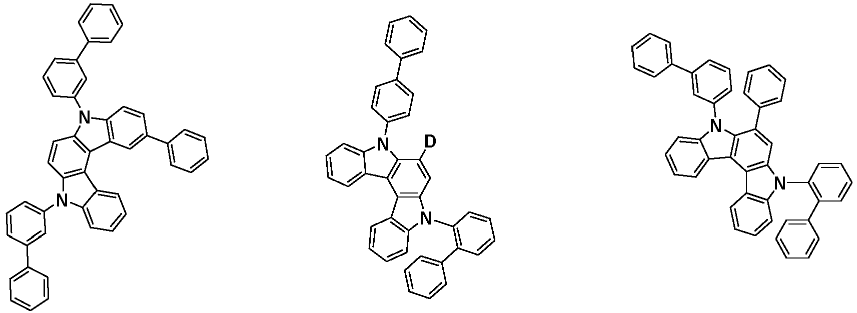

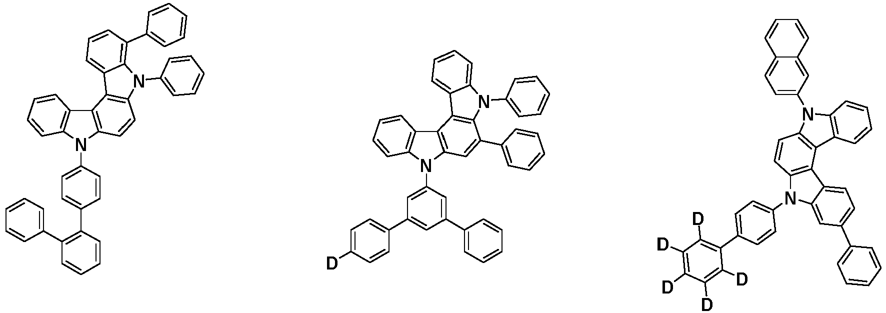

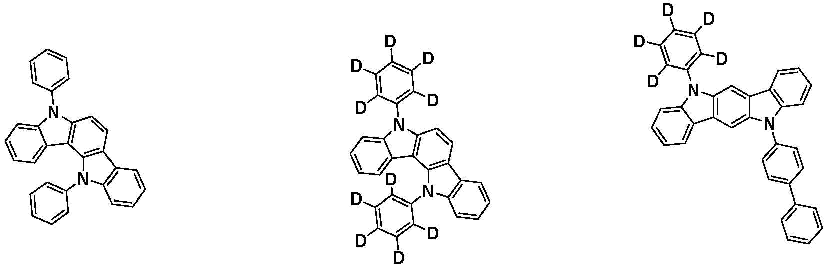

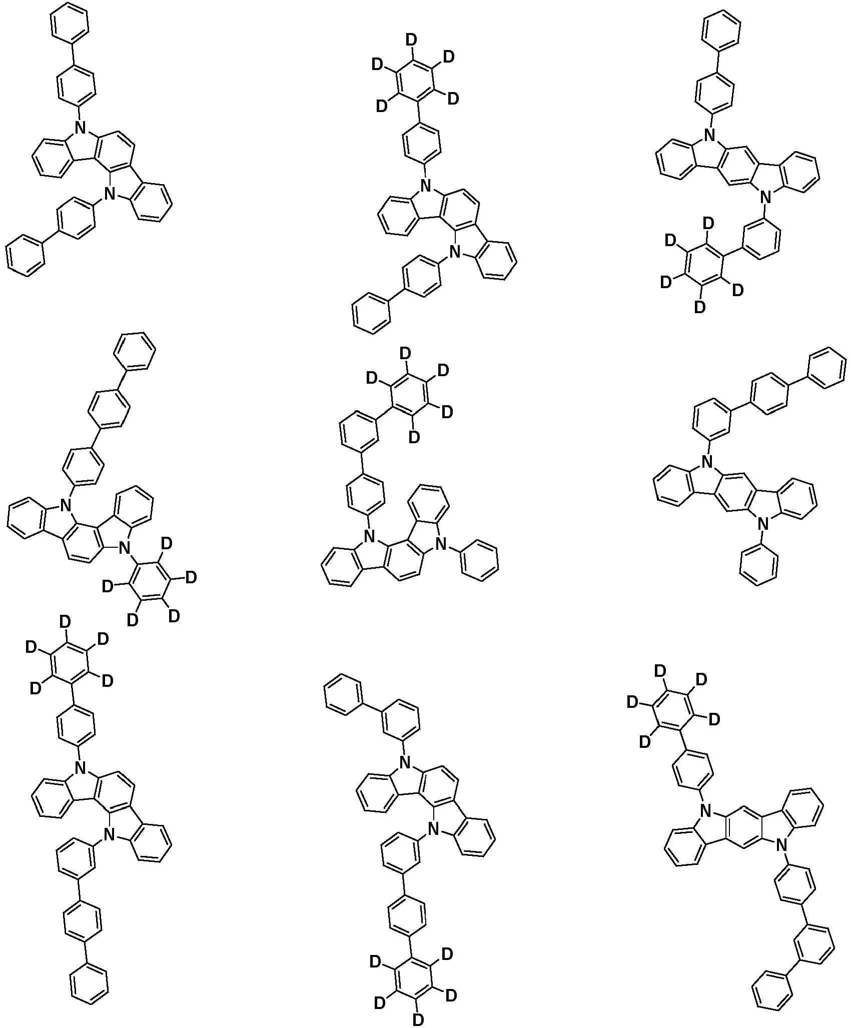

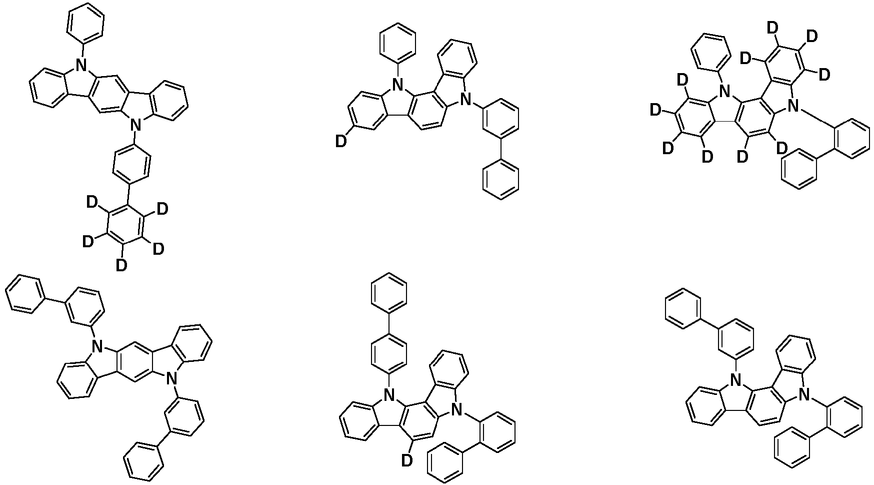

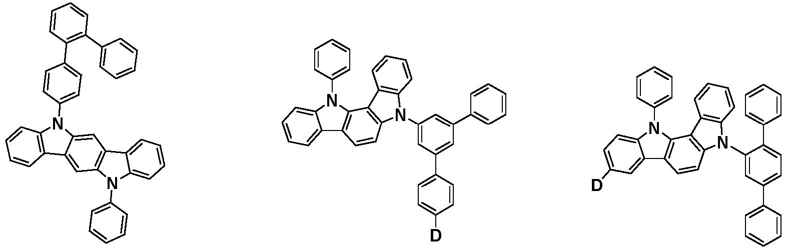

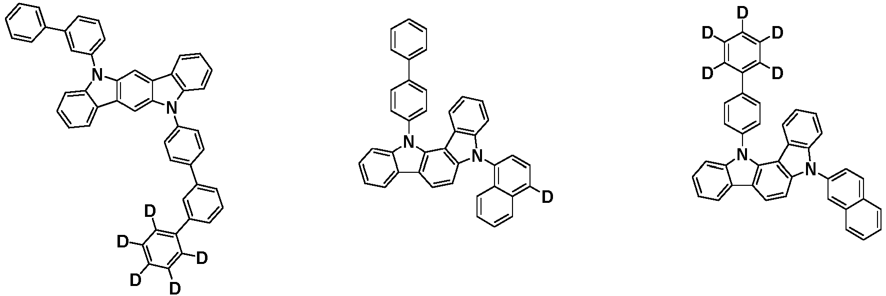

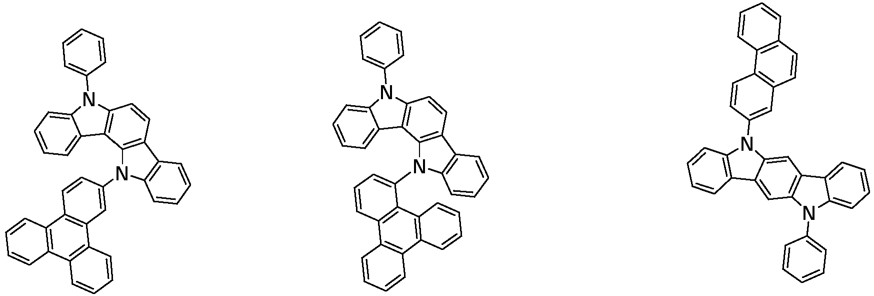

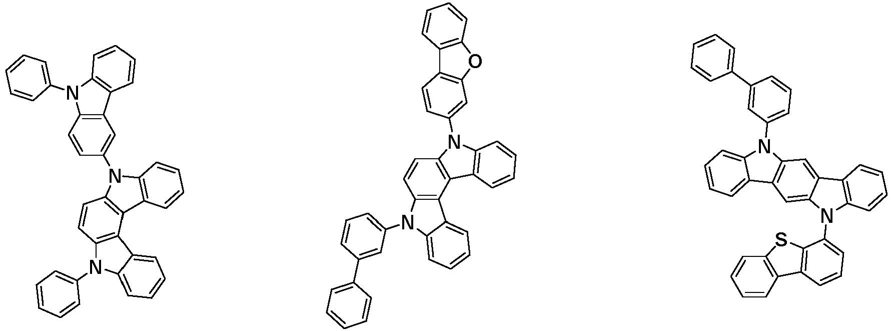

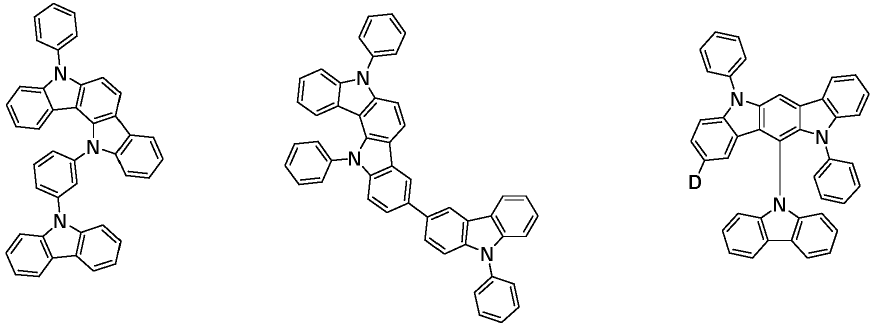

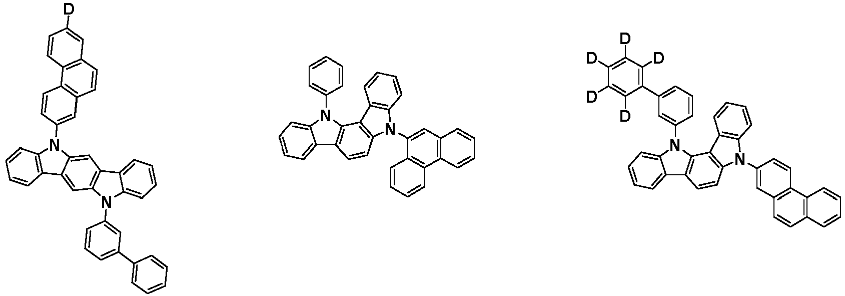

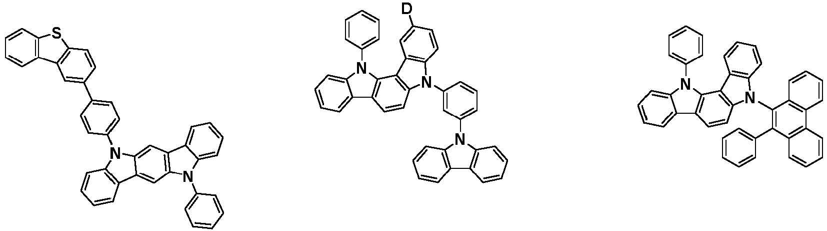

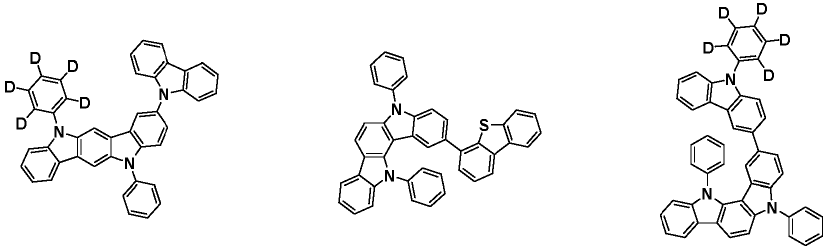

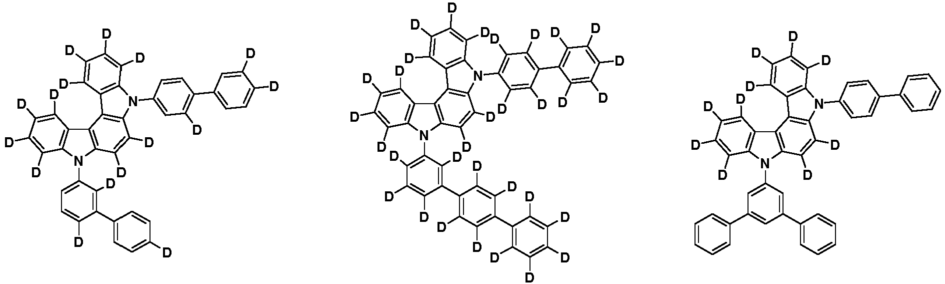

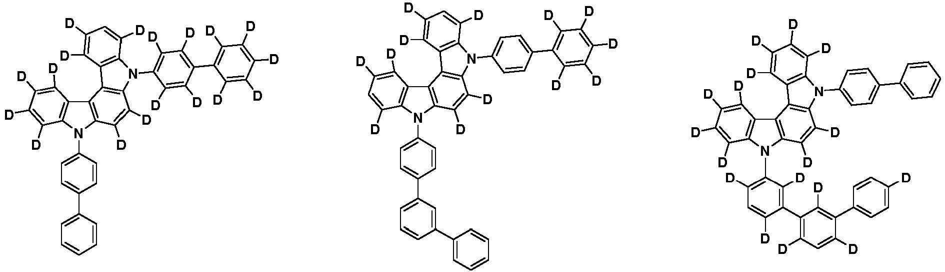

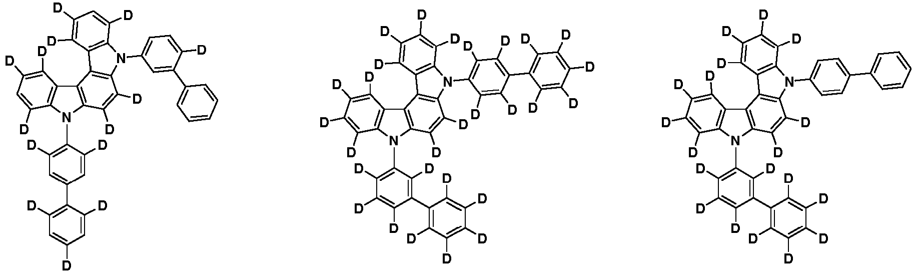

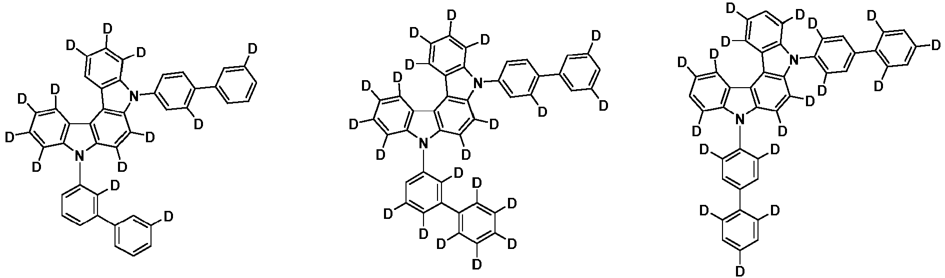

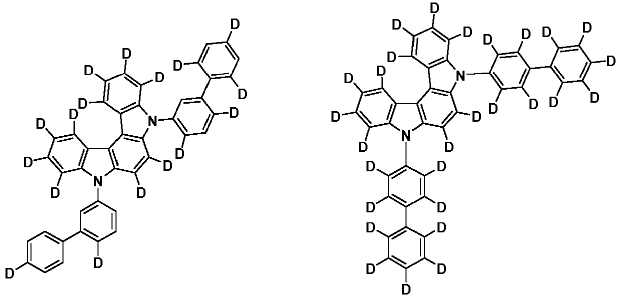

Meanwhile, the second compound may be prepared by, for example, a preparation method as shown in Reaction Scheme 2 below. 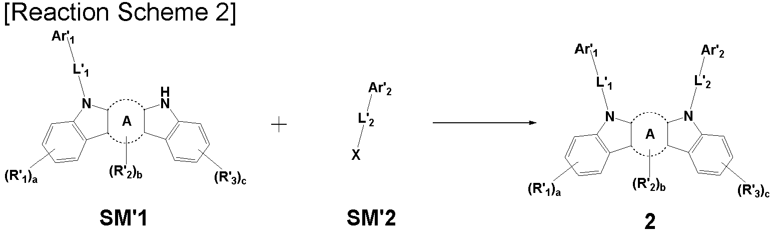 in the Reaction Scheme 2, each X is independently halogen, preferably bromo, or chloro, and the definitions of other substituents are the same as described above.

Specifically, the compound represented by the Chemical Formula 2 is prepared by combining starting materials of SM'1 and SM'2 through an amine substitution reaction. Such an amine substitution reaction is preferably performed in the presence of a palladium catalyst and a base. In addition, the reactive group for the amine substitution reaction may be appropriately changed, and the method for preparing the compound represented by the Chemical Formula 2 may be more specifically described in Synthesis Examples described below.

The first compound and the second compound may be included in the light emitting layer at a weight ratio of 1:9 to 9:1. When the second compound is included in the light emitting layer in an excessively small amount compared to the first compound, electron transport in the light emitting layer is not smooth, and thus holes and electrons are not balanced throughout the device, which may cause problems in voltage, efficiency, and lifespan of the manufactured device. When it is included in an excessively large amount, there may be a problem in that the lifespan is reduced. For example, a weight ratio of the first compound and the second compound in the light emitting layer may be 2:8 to 8:2, 3:7 to 7:3, 4:6 to 6:4, or 4:6 to 5:5.

Meanwhile, the light emitting layer may further include a dopant material other than the two kinds of host materials. Examples of the dopant material include an aromatic amine derivative, a styrylamine compound, a boron complex, a fluoranthene compound, a metal complex, and the like. Specifically, the aromatic amine derivative is a substituted or unsubstituted fused aromatic ring derivative having an arylamino group, and examples thereof include pyrene, anthracene, chrysene, periflanthene and the like, which have an arylamino group. The styrylamine compound is a compound where at least one arylvinyl group is substituted in substituted or unsubstituted arylamine, in which one or two or more substituent groups selected from the group consisting of an aryl group, a silyl group, an alkyl group, a cycloalkyl group, and an arylamino group are substituted or unsubstituted. Specific examples thereof include styrylamine, styryldiamine, styryltriamine, styryltetramine, and the like, but are not limited thereto. Further, the metal complex includes an iridium complex, a platinum complex, and the like, but is not limited thereto.

Herein, the dopant material may be included in the light emitting layer in an amount of 1 to 25 wt% based on a total weight of the host material and the dopant material.

### Hole blocking layer

The organic light emitting device according to the present disclosure may include a hole blocking layer between a light emitting layer and an electron transport layer to be described later, if necessary. The hole blocking layer means a layer which is formed on the light emitting layer, is preferably provided in contact with the light emitting layer, and thus serves to control electron mobility, to prevent excessive movement of holes, and to increase the probability of hole-electron bonding, thereby improving the efficiency of the organic light emitting device. The hole blocking layer includes a hole blocking material, and as an example of such a hole blocking material, compounds having an electron attracting group, such as azine-based derivatives including triazine; triazole derivatives; oxadiazole derivatives; phenanthroline derivatives; phosphine oxide derivatives may be used, but is not limited thereto.

### Electron transport layer

The electron transport layer is formed between the light emitting layer and the cathode to receive electrons from an electron injection layer and transport the electrons to a light emitting layer. The electron transport layer includes an electron transport material, and the electron transport material is suitably a material which can receive electrons well from a cathode and transfer the electrons to a light emitting layer and has large mobility for electrons.

Specific examples of the electron injection and transport material include: an Al complex of 8-hydroxyquinoline; a complex including Alq₃; an organic radical compound; a hydroxyflavone-metal complex; a triazine derivative, and the like, but are not limited thereto. Alternatively, it may be used together with fluorenone, anthraquinodimethane, diphenoquinone, thiopyran dioxide, oxazole, oxadiazole, triazole, imidazole, perylenetetracarboxylic acid, fluorenylidene methane, anthrone, or derivatives thereof, a metal complex compound, a nitrogen-containing 5-membered ring derivative, or the like, but are not limited thereto.

### Electron injection layer

The organic light emitting device according to the present disclosure may include an electron injection layer between an electron transport layer and a cathode, if necessary.

The electron injection layer is located between the electron transport layer and a cathode, and injects electrons from the cathode. The electron injection layer includes an electron injection material, and a material capable of transporting electrons, having an excellent effect of injecting electrons to a light emitting layer or a light emitting material, and excellent in forming a thin film is suitable.

Specific examples of the electron injection material include LiF, NaCl, CsF, Li₂O, BaO, fluorenone, anthraquinodimethane, diphenoquinone, thiopyran dioxide, oxazole, oxadiazole, triazole, imidazole, perylenetetracarboxylic acid, fluorenylidene methane, anthrone, and the like, and derivatives thereof, a metal complex compound, a nitrogen-containing 5-membered ring derivative, and the like, but are not limited thereto.

Examples of the metal complex compound include 8-hydroxyquinolinato lithium, bis(8-hydroxyquinolinato)zinc, bis(8-hydroxyquinolinato)copper, bis(8-hydroxyquinolinato)manganese, tris(8-hydroxyquinolinato)aluminum, tris(2-methyl-8-hydroxyquinolinato)aluminum, tris(8-hydroxyquinolinato)gallium, bis(10-hydroxybenzo[h]quinolinato)beryllium, bis(10-hydroxybenzo[h]quinolinato)zinc, bis(2-methyl-8-quinolinato)chlorogallium, bis(2-methyl-8-quinolinato)(o-cresolato)gallium, bis(2-methyl-8-quinolinato)(1-naphtholato)aluminum, bis(2-methyl-8-quinolinato)(2-naphtholato)gallium, and the like, but are not limited thereto.

### Organic light emitting device

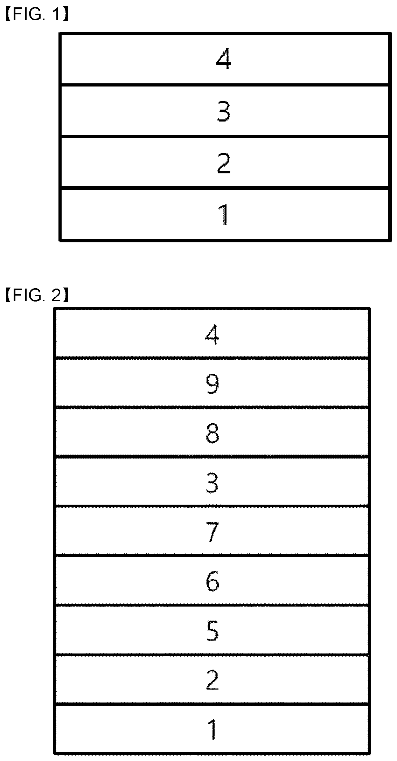

A structure of the organic light emitting device according to the present disclosure is illustrated in FIG. 1. FIG. 1 shows an example of an organic light emitting device including a substrate 1, an anode 2, a light emitting layer 3, and a cathode 4. In such a structure, the first compound and the second compound may be included in the light emitting layer.

FIG. 2 shows an example of an organic light emitting device including a substrate 1, an anode 2, a hole injection layer 5, a hole transport layer 6, an electron blocking layer 7, a light emitting layer 3, a hole blocking layer 8, an electron transport and injection layer 9, and a cathode 4. In such a structure, the first compound and the second compound may be included in the light emitting layer.

The organic light emitting device according to the present disclosure may be manufactured by sequentially laminating the above-mentioned components. In this case, the organic light emitting device may be manufactured by depositing a metal, metal oxides having conductivity, or an alloy thereof on the substrate using a PVD (physical vapor deposition) method such as a sputtering method or an e-beam evaporation method to form an anode, forming the above-mentioned respective layers thereon, and then depositing a material that can be used as the cathode thereon. In addition to such a method, the organic light emitting device may be manufactured by sequentially depositing a cathode material, an organic material layer and an anode material on a substrate. Further, the light emitting layer may be formed using the host and the dopant by a solution coating method as well as a vacuum deposition method. Herein, the solution coating method means a spin coating, a dip coating, a doctor blading, an inkjet printing, a screen printing, a spray method, a roll coating, or the like, but is not limited thereto.

In addition to such a method, the organic light emitting device may be manufactured by sequentially depositing a cathode material, an organic material layer and an anode material on a substrate (International Publication WO2003/012890). However, the manufacturing method is not limited thereto.

Meanwhile, the organic light emitting device according to the present disclosure may be a front side emission type, a backside emission type, or a double-sided emission type according to the used material.

The preparation of the organic light emitting device including the compound represented by the Chemical Formula 1 and the compound represented by the Chemical Formula 2 will be described in detail in the following examples. However, these examples are presented for illustrative purposes only, and are not intended to limit the scope of the present disclosure.

### [Synthesis Examples]

### Synthesis Example 1: Synthesis of Compound 1-1

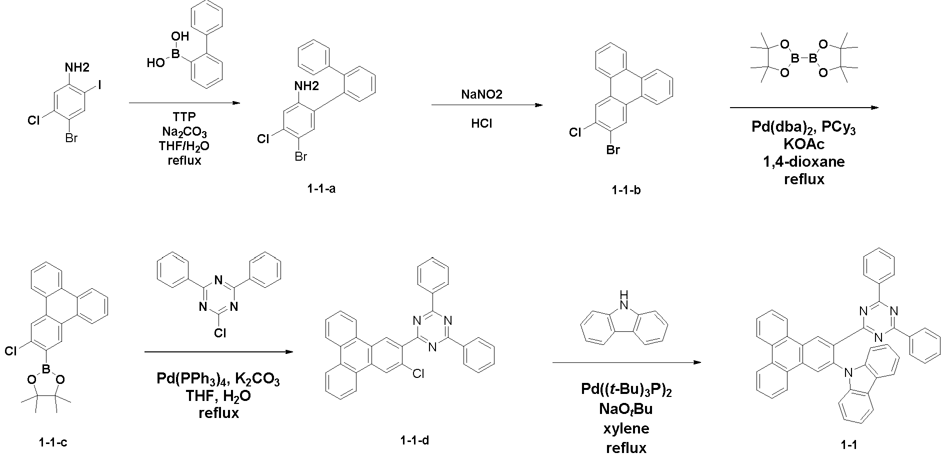

### Step 1) Synthesis of Compound 1-1-a

4-bromo-chloro-2-iodoaniline (50 g, 150.4 mmol) and [1,1'-biphenyl]-2-yl boronic acid (29.8 g, 150.4 mmol) were added to 1000 ml of tetrahydrofuran under a nitrogen atmosphere, and the mixture was stirred and refluxed. Then, sodium carbonate (47.8 g, 451.3 mmol) was dissolved in 48 ml of water, and then added thereto. Thereafter, it was stirred sufficiently, followed by adding tetrakistriphenyl-phosphinopalladium (5.2 g, 4.5mmol). After 2 hours of reaction, cooling was performed to room temperature. Then, the organic layer was separated from the water layer, and then the organic layer was distilled. Then, this was dissolved again in 1079 mL of chloroform corresponding to 20 times, and washed twice with water. Thereafter, the organic layer was separated, treated with anhydrous magnesium sulfate, stirred, then filtered, and the filtrate was distilled under reduced pressure. The concentrated compound was recrystallized using chloroform and hexane to prepare Compound 1-1-a in the form of brown solid (43.2g, 80%, MS: [M+H]⁺= 359.7).

### Step 2) Synthesis of Compound 1-1-b

Compound 1-1-a (40 g, 111.5 mmol) was added to hydrochloric acid (2 M, 700 mL), stirred at 0 °C for about 15 minutes, and then sodium nitrite (8.5 g, 122.6 mmol) was slowly added thereto. After 1 hour, the mixture was heated at 60 °C for 3 hours, and cooling was performed to room temperature. Then, the organic layer was separated from the water layer, and then the organic layer was distilled. Then, this was dissolved again in 800 mL of chloroform corresponding to 20 times, and washed twice with an aqueous sodium hydrogen carbonate solution. Thereafter, the organic layer was separated, treated with anhydrous magnesium sulfate, stirred, then filtered, and the filtrate was distilled under reduced pressure. The concentrated compound was recrystallized using chloroform and hexane to prepare Compound 1-1-b in the form of brown solid (27.4g, 72%, MS: [M+H]⁺= 342.0).

### Step 3) Synthesis of Compound 1-1-c

1-1-b (25 g, 73.3 mmol) and bis(pinacolato)diboron (18.6 g, 73.3mmol) were added to 500 mL of 1,4-dioxane under a nitrogen atmosphere, and the mixture was stirred and refluxed. Thereafter, tripotassium phosphate (46.7 g, 219.9mmol) was added thereto, and sufficiently stirred, followed by adding dibenzylideneacetone palladium (1.3 g, 2.2 mmol) and tricyclohexylphosphine (1.2 g, 4.4 mmol). After 3 hours of reaction, cooling was performed to room temperature. Thereafter, the organic layer was filtered to remove salt, and then the filtered organic layer was distilled. Then, this was dissolved again in 285 mL of chloroform corresponding to 10 times, and washed twice with water. Thereafter, the organic layer was separated, treated with anhydrous magnesium sulfate, stirred, then filtered, and the filtrate was distilled under reduced pressure. The concentrated compound was recrystallized using chloroform and ethanol to prepare Compound 1-1-c in the form of brown solid (22.8g, 80%, MS: [M+H]⁺= 389.7).

### Step 4) Synthesis of Compound 1-1-d

1-1-c (20 g, 51.5 mmol) and 2-chloro-4,6-diphenyl-1,3,5-triazine (13.8 g, 51.5 mmol) were added to 400 ml of tetrahydrofuran under a nitrogen atmosphere, and the mixture was stirred and refluxed. Then, sodium carbonate (16.4 g, 154.4mmol) was dissolved in 16 ml of water, and then added thereto. Thereafter, it was stirred sufficiently, followed by adding tetrakistriphenyl-phosphinopalladium (1.8 g, 1.5mmol). After 1 hour of reaction, cooling was performed to room temperature. Then, the organic layer was separated from the water layer, and then the organic layer was distilled. Then, this was dissolved again in 554 mL of chloroform corresponding to 20 times, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 1-1-d in the form of yellow solid (19.1g, 69%, MS: [M+H]⁺= 494.8).

### Step 5) Synthesis of Compound 1-1

1-1-d (30 g, 55.7 mmol) and 9H-carbazole (9.3 g, 55.7 mmol) were added to 600 ml of xylene under a nitrogen atmosphere, and the mixture was stirred and refluxed. Thereafter, sodium tert-butoxide (16.1 g, 167.1 mmol) was added thereto, and sufficiently stirred, followed by adding bis(tri-tertiary-butylphosphine)palladium (0.9 g, 1.7 mmol). After 4 hours of reaction, cooling was performed to room temperature. Thereafter, the organic layer was filtered to remove salt, and then the filtered organic layer was distilled. Then, this was dissolved again in 351 mL of chloroform corresponding to 10 times, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 1-1 in the form of yellow solid (19g, 54%, MS: [M+H]⁺= 625.7).

### Synthesis Example 2: Synthesis of Compound 1-2

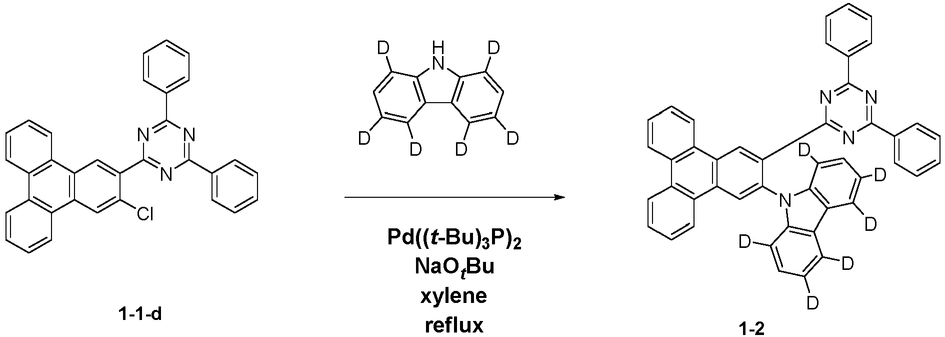

Compound 1-2 (MS: [M+H]⁺= 631.8) was prepared in the same manner as in the preparation method of Compound 1-1, except that 9H-carbazole was changed to 9H-carbazole-1,3,4,5,6,8-d6 in Synthesis Example 1.

### Synthesis Example 3: Synthesis of Compound 1-3

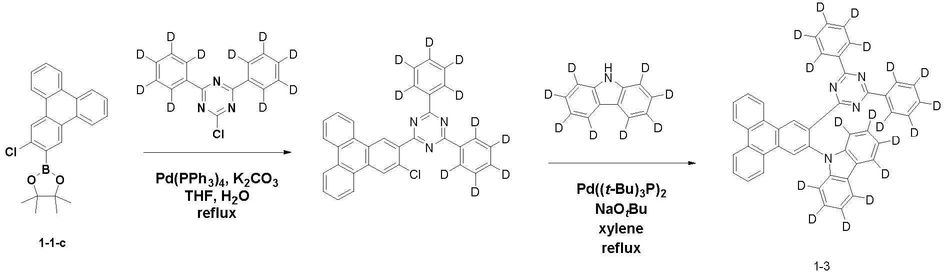

Compound 1-3 (MS[M+H]⁺= 643.8) was prepared in the same manner as in the preparation method of Compound 1-1, except that 2-chloro-4,6-diphenyl-1,3,5-triazine and 9H-carbazole were respectively changed to 2-chloro-4,6-bis(phenyl-d5)-1,3,5-triazine and 9H-carbazole-1,2,3,4,5,6,7,8-d8 in Synthesis Example 1.

### Synthesis Example 4: Synthesis of Compound 1-4

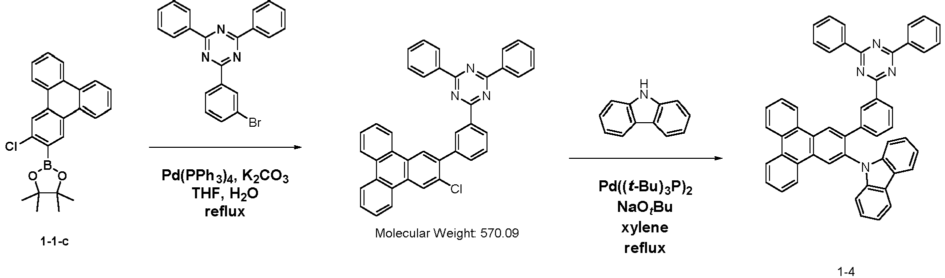

Compound 1-4 (MS[M+H]⁺= 701.8) was prepared in the same manner as in the preparation method of Compound 1-1, except that 2-chloro-4,6-diphenyl-1,3,5-triazine was changed to 2-(3-bromophenyl)-4,6-diphenyl-1,3,5-triazine in Synthesis Example 1.

### Synthesis Example 5: Synthesis of Compound 1-5

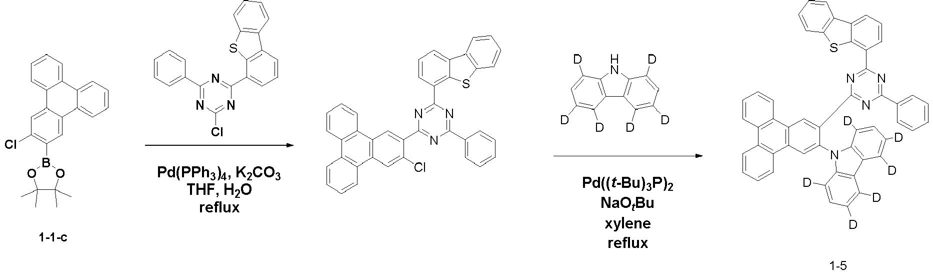

Compound 1-5 (MS[M+H]⁺= 663.2) was prepared in the same manner as in the preparation method of Compound 1-1, except that 2-chloro-4,6-diphenyl-1,3,5-triazine and 9H-carbazole were respectively changed to 2-chloro-4-(dibenzo[b,d]thiophen-4-yl)-6-phenyl-1,3,5-triazine and 9H-carbazole-1,3,4,5,6,8-d6 in Synthesis Example 1.

### Synthesis Example 6: Synthesis of Compound 1-6

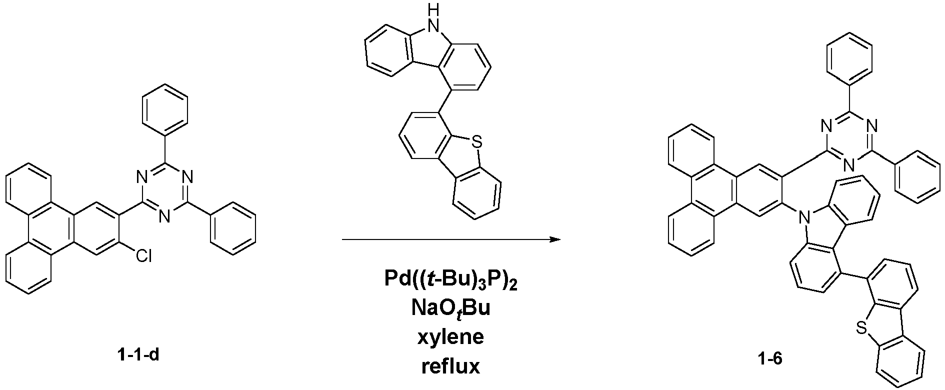

Compound 1-6 (MS[M+H]⁺= 807.8) was prepared in the same manner as in the preparation method of Compound 1-1, except that 9H-carbazole was changed to 4-(dibenzo[b,d]thiophen-4-yl)-9H-carbazole in Synthesis Example 1.

### Synthesis Example 7: Synthesis of Compound 1-7

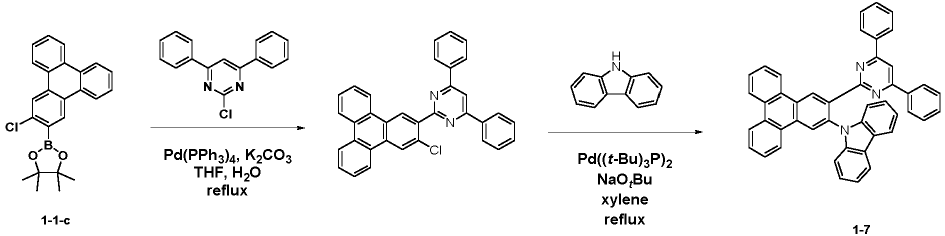

Compound 1-7 (MS[M+H]⁺= 624.7) was prepared in the same manner as in the preparation method of Compound 1-1, except that 2-chloro-4,6-diphenyl-1,3,5-triazine was changed to 2-chloro-4,6-diphenylpyrimidine in Synthesis Example 1.

### Synthesis Example 8: Synthesis of Compound 1-8

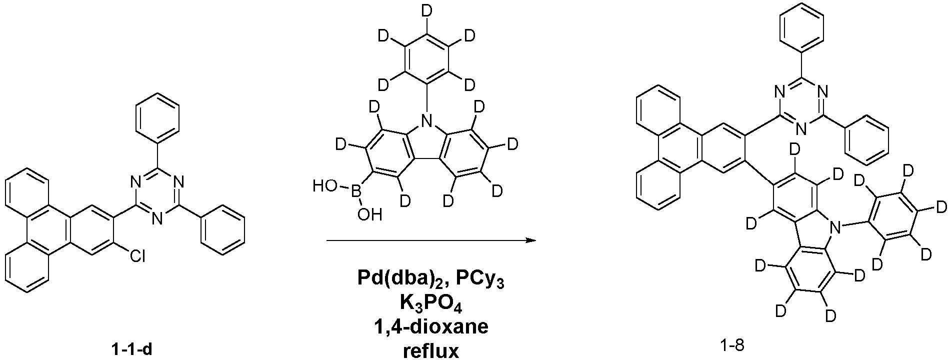

1-1-d (30 g, 60.7 mmol) and (9-(phenyl-d5)-9H-carbazol-3-yl-1,2,4,5,6,7,8-d7)boronic acid (18.2 g, 60.7 mmol) were added to 600 mL of 1,4-dioxane under a nitrogen atmosphere, and the mixture was stirred and refluxed. Thereafter, tripotassium phosphate (38.7 g, 182.2mmol) was dissolved in 39 ml of water, and then added thereto. Thereafter, it was stirred sufficiently, followed by adding dibenzylideneacetonepalladium (1 g, 1.8 mmol) and tricyclohexylphosphine (1 g, 3.6 mmol). After 9 hours of reaction, it was cooled to room temperature and the resulting solid was filtered. Then, the resulting solid was dissolved in 1299 mL of chloroform corresponding to 30 times, and washed twice with water. Thereafter, the organic layer was separated, treated with anhydrous magnesium sulfate, stirred, then filtered, and the filtrate was distilled under reduced pressure. The concentrated compound was recrystallized using chloroform and ethyl acetate to prepare Compound 1-8 in the form of yellow solid (28.1g, 65%, MS: [M+H]⁺= 713.9).

### Synthesis Example 9: Synthesis of Compound 1-9

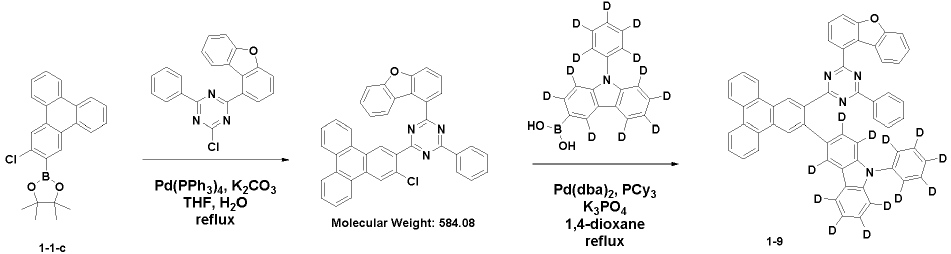

Compound 1-9 (MS[M+H]⁺= 804.2) was prepared in the same manner as in the preparation methods of Compound 1-1 and Compound 1-8, except that 2-chloro-4,6-diphenyl-1,3,5-triazine was changed to 2-chloro-4-(dibenzo[b,d]furan-1-yl)-6-phenyl-1,3,5-triazine in Synthesis Examples 1 and 8.

### Synthesis Example 10: Synthesis of Compound 1-10

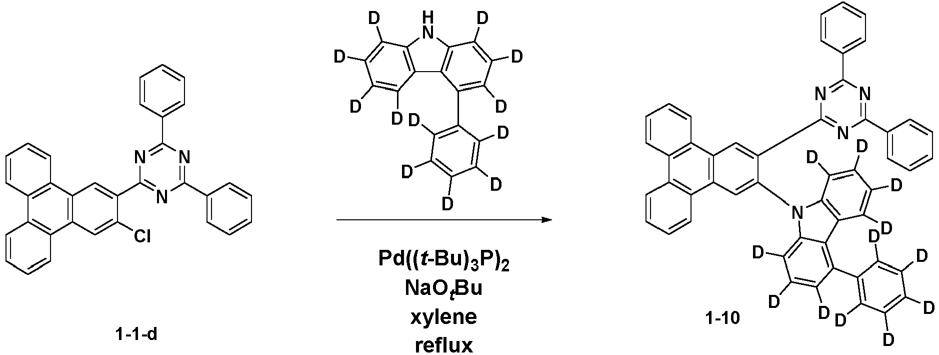

Compound 1-10 (MS[M+H]⁺= 713.9) was prepared in the same manner as in the preparation method of Compound 1-1, except that 9H-carbazole was changed to 4-(phenyl-d5)-9H-carbazole-1,2,3,5,6,7,8-d7 in Synthesis Example 1.

### Synthesis Example 11: Synthesis of Compound 2-1

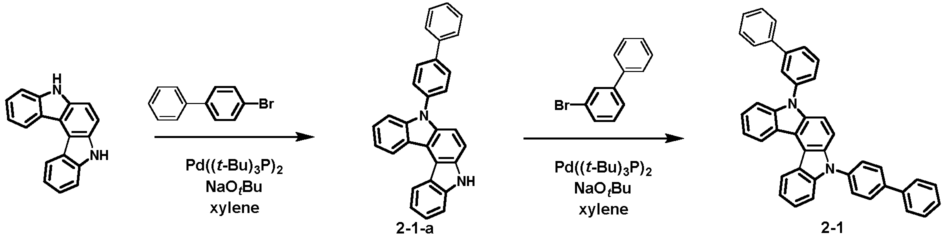

### Step 1) Synthesis of Compound 2-1-a

5,8-dihydroindolo[2,3-c]carbazole (30 g, 117 mmol) and 4-bromo-1,1'-biphenyl (27.3 g, 117 mmol) were added to 600 ml of xylene under a nitrogen atmosphere, and the mixture was stirred and refluxed. Thereafter, sodium tert-butoxide (33.8 g, 351.1 mmol) was added thereto, and sufficiently stirred, followed by adding bis(tri-tertiary-butylphosphine)palladium (1.8 g, 3.5mmol). After 2 hours of reaction, cooling was performed to room temperature. Thereafter, the organic layer was filtered to remove salt, and then the filtered organic layer was distilled. Then, this was dissolved again in 478 mL of toluene corresponding to 10 times, and washed twice with water. Thereafter, the organic layer was separated, treated with anhydrous magnesium sulfate, stirred, then filtered, and the filtrate was distilled under reduced pressure. The concentrated compound was purified by silica column using toluene and ethyl acetate to prepare Compound 2-1-a in the form of white solid (31.6g, 66%, MS: [M+H]⁺= 409.5).

### Step 2) Synthesis of Compound 2-1

2-1-a (30 g, 73.4 mmol) and 3-bromo-1,1'-biphenyl (17.1 g, 73.4 mmol) were added to 600 ml of xylene under a nitrogen atmosphere, and the mixture was stirred and refluxed. Thereafter, sodium tert-butoxide (21.2 g, 220.3mmol) was added thereto, and sufficiently stirred, followed by adding bis(tri-tertiary-butylphosphine)palladium (1.1 g, 2.2mmol). After 4 hours of reaction, cooling was performed to room temperature. Thereafter, the organic layer was filtered to remove salt, and then the filtered organic layer was distilled. Then, this was dissolved again in 412 mL of toluene corresponding to 10 times, and washed twice with water. Thereafter, the organic layer was separated, treated with anhydrous magnesium sulfate, stirred, then filtered, and the filtrate was distilled under reduced pressure. The concentrated compound was purified by silica column using toluene and ethyl acetate to prepare Compound 2-1 in the form of white solid (31.3g, 76%, MS: [M+H]⁺= 561.7).

### Synthesis Example 12: Synthesis of Compound 2-2

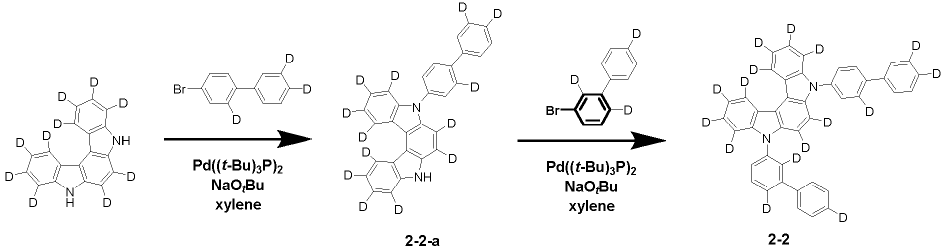

Compound 2-2 (MS[M+H]⁺= 577.8) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,8-dihydroindolo[2,3-c]carbazole-1,2,3,4,6,7,9,10,11,12-d10, 4-bromo-1,1'-biphenyl-2,3',4'-d3 and 3-bromo-1,1'-biphenyl-2,4',6-d3 in Synthesis Example 11.

### Synthesis Example 13: Synthesis of Compound 2-3

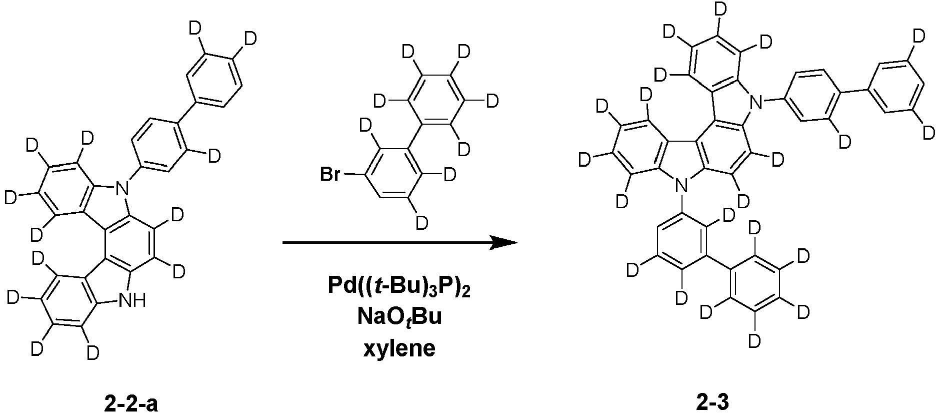

Compound 2-3 (MS[M+H]⁺= 582.8) was prepared in the same manner as in the preparation method of Compound 2-1, except that Compound 2-1-a was changed to Compound 2-2-a and 3-bromo-1,1'-biphenyl was changed to 3-bromo-1,1'-biphenyl-2,2',3',4',5,5',6,6'-d8 in Synthesis Example 11.

### Synthesis Example 14: Synthesis of Compound 2-4

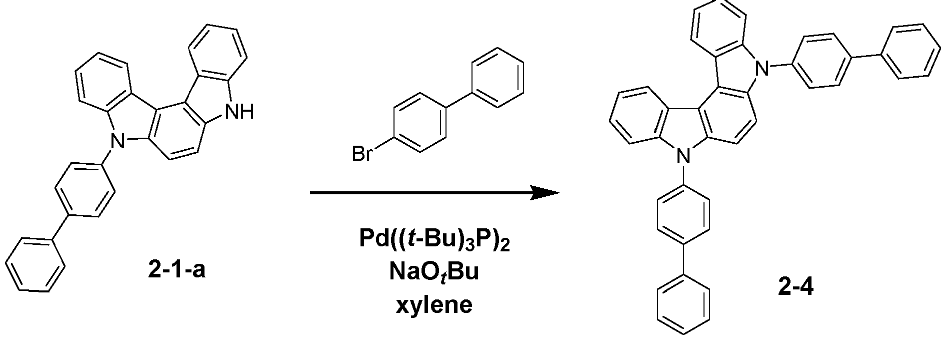

Compound 2-4 (MS[M+H]⁺= 561.7) was prepared in the same manner as in the preparation method of Compound 2-1, except that 3-bromo-1,1'-biphenyl was changed to 4-bromo-1,1'-biphenyl in Synthesis Example 11.

### Synthesis Example 15: Synthesis of Compound 2-5

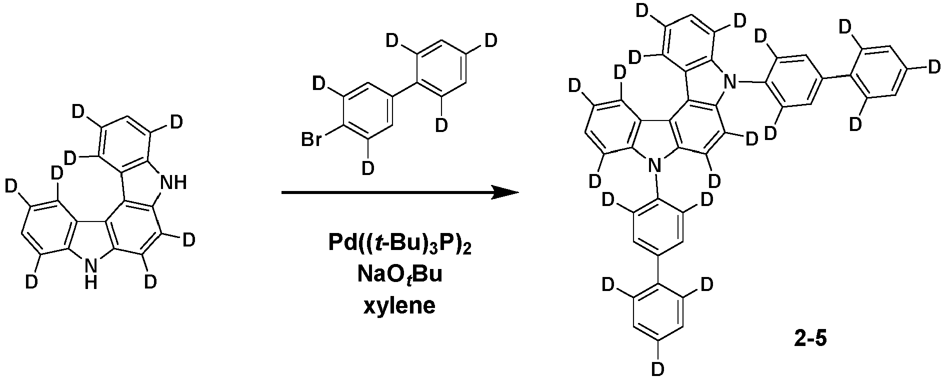

Compound 2-5 (MS[M+H]⁺= 579.8) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,8-dihydroindolo[2,3-c]carbazole-1,2,4,6,7,9,11,12-d8 and 4-bromo-1,1'-biphenyl-2',3,4',5,6'-d5 in Synthesis Example 11.

### Synthesis Example 16: Synthesis of Compound 2-6

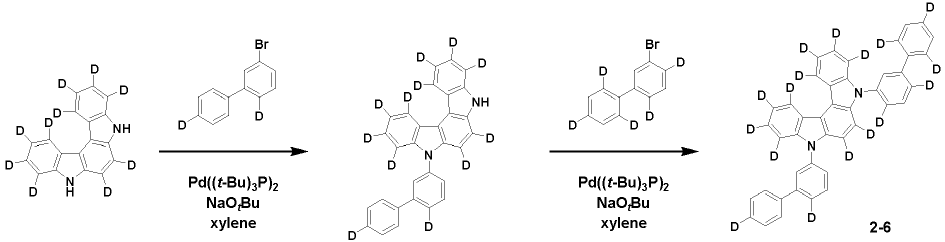

Compound 2-6 (MS[M+H]⁺= 578.8) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,8-dihydroindolo[2,3-c]carbazole-1,2,3,4,6,7,9,10,11,12-d10, 3-bromo-1,1'-biphenyl-4',6-d2 and 3-bromo-1,1'-biphenyl-2',4,4',6,6'-d5 in Synthesis Example 11.

### Synthesis Example 17: Synthesis of Compound 2-7

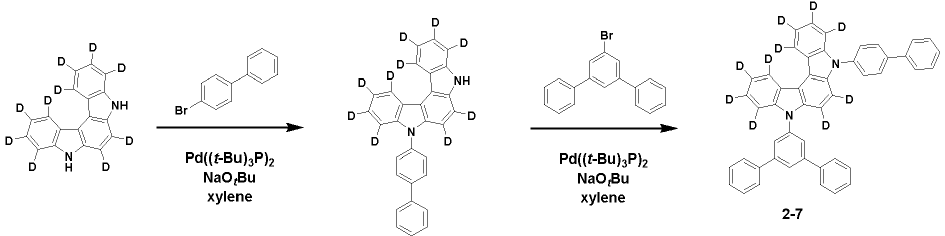

Compound 2-7 (MS[M+H]⁺= 647.8) was prepared in the same manner as in the preparation method of Compound 2-1, except that 5,8-dihydroindolo[2,3-c]carbazole, and 3-bromo-1,1'-biphenyl were respectively changed to 5,8-dihydroindolo[2,3-c]carbazole-1,2,3,4,6,7,9,10,11,12-d10, and 5'-bromo-1,1':3',1"-terphenyl in Synthesis Example 11.

### Synthesis Example 18: Synthesis of Compound 2-8

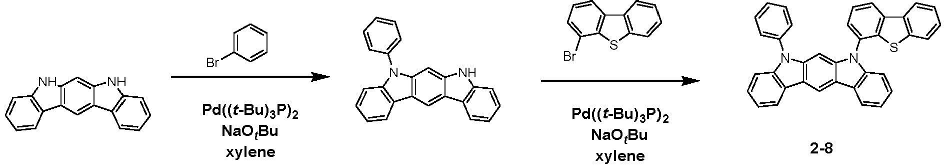

Compound 2-8 (MS[M+H]⁺= 515.6) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,7-dihydroindolo[2,3-b]carbazole, bromobenzene and 4-bromodibenzo[b,d]thiophene in Synthesis Example 11.

### Synthesis Example 19: Synthesis of Compound 2-9

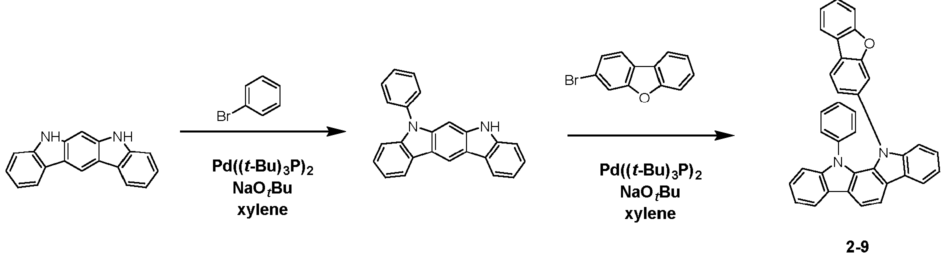

Compound 2-9 (MS[M+H]⁺= 499.6) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,7-dihydroindolo[2,3-b]carbazole, bromobenzene and 4-bromodibenzo[b,d]furan in Synthesis Example 11.

### Synthesis Example 20: Synthesis of Compound 2-10

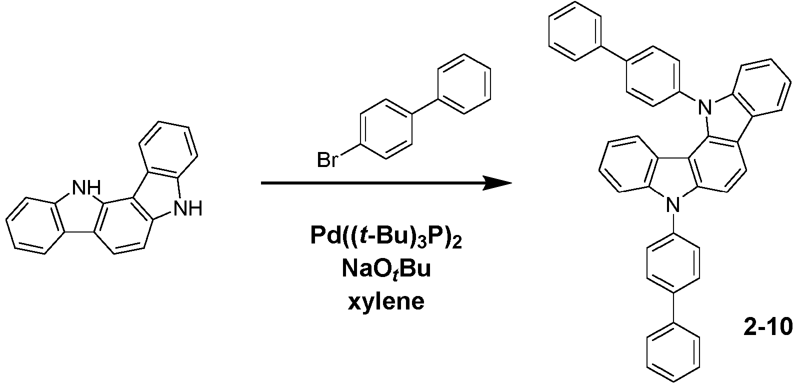

Compound 2-10 (MS[M+H]⁺= 561.7) was prepared in the same manner as in the preparation method of Compound 2-1, except that 5,8-dihydroindolo[2,3-c]carbazole, 4-bromo-1,1'-biphenyl and 3-bromo-1,1'-biphenyl were respectively changed to 5,12-dihydroindolo[3,2-a]carbazole and 4-bromo-1,1'-biphenyl in Synthesis Example 11.

### [Examples]

### Example 1

A glass substrate on which ITO (Indium Tin Oxide) was coated as a thin film to a thickness of 1,400 Å was put into distilled water in which a detergent was dissolved, and ultrasonically cleaned. At this time, a product manufactured by Fischer Co. was used as the detergent, and distilled water filtered twice using a filter manufactured by Millipore Co. was used as the distilled water. After the ITO was cleaned for 30 minutes, ultrasonic cleaning was repeated twice using distilled water for 10 minutes. After the cleaning with distilled water was completed, the substrate was ultrasonically cleaned with solvents of isopropyl alcohol, acetone, and methanol, dried, and then transferred to a plasma cleaner. In addition, the substrate was cleaned for 5 minutes using oxygen plasma and then transferred to a vacuum depositor.

A hole injection layer was formed by thermally vacuum-depositing the following Compound HT-A and the following Compound PD to a thickness of 100 Å at a weight ratio of 95:5 on the prepared ITO transparent electrode. Then, only the following Compound HT-A was deposited to a thickness of 1150 Å to form a hole transport layer. The following Compound HT-B was thermally vacuum-deposited thereon to a thickness of 450 Å to form an electron blocking layer.

On the electron blocking layer, the Compound 1-1 and the Compound 2-1 prepared above as host compounds and the following Compound GD as a dopant compound were vacuum-deposited to a thickness of 400 Å at a weight ratio of 85:15 to form a light emitting layer. Herein, a weight ratio of Compound 1-1 and Compound 2-1 was 1:1.

On the light emitting layer, the following Compound ET-A was vacuum-deposited to a thickness of 50 Å to form a hole blocking layer. On the hole blocking layer, the following Compound ET-B and Liq were thermally vacuum-deposited to a thickness of 250 Å at a weight ratio of 2:1, and LiF and magnesium were vacuum-deposited to a thickness of 30 Å at a weight ratio of 1:1 to form an electron transport and injection layer. On the electron transport and injection layer, magnesium and silver were deposited to a thickness of 160 Å at a weight ratio of 1:4 to form a cathode, thereby manufacturing an organic light emitting device.

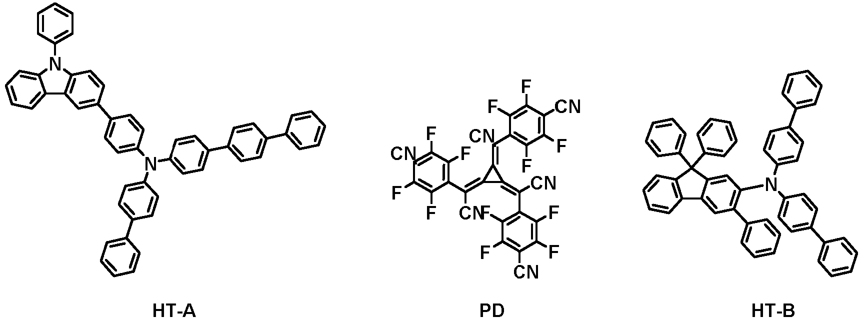

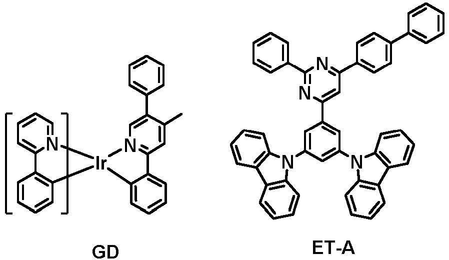

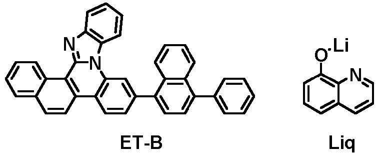

In the above process, the deposition rate of the organic material was maintained at 0.4 to 0.7 Å/sec, the deposition rate of lithium fluoride of the cathode was maintained at 0.3Å/sec, and the deposition rate of silver and magnesium was maintained at 2 Å/sec. In addition, the degree of vacuum during the deposition was maintained at 2x10⁻⁷ to 5x10⁻⁶ torr, thereby manufacturing an organic light emitting device.

### Examples 2 to 10

An organic light emitting device was manufactured in the same manner as in Example 1, except that the compounds shown in Table 1 were used instead of Compound 1-1 and Compound 2-1 in Example 1.

At this time, the structures of the first compound and the second compound used in Examples are summarized as follows.

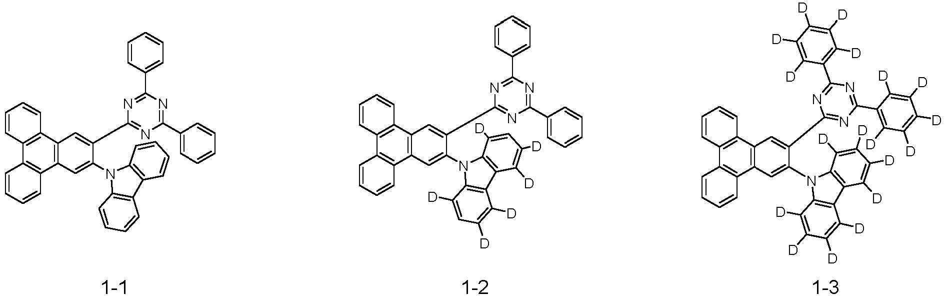

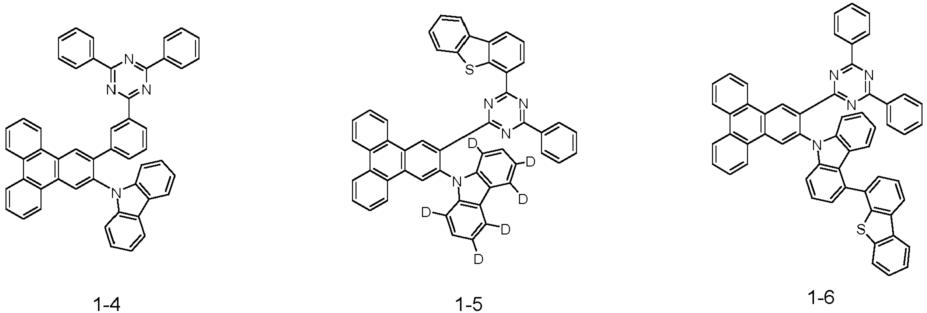

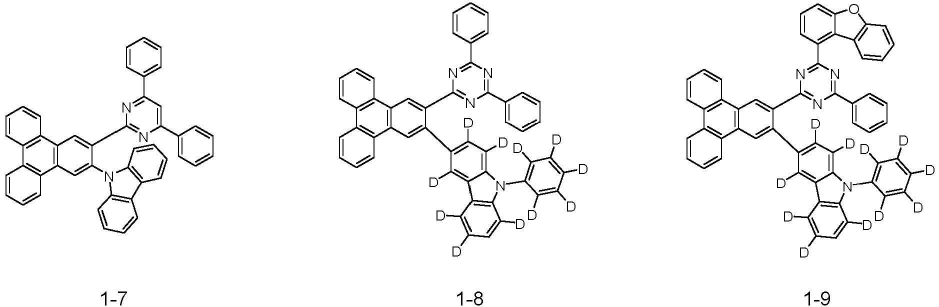

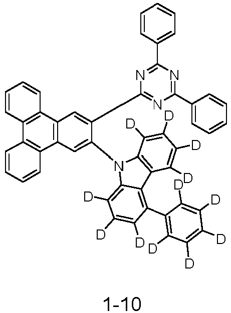

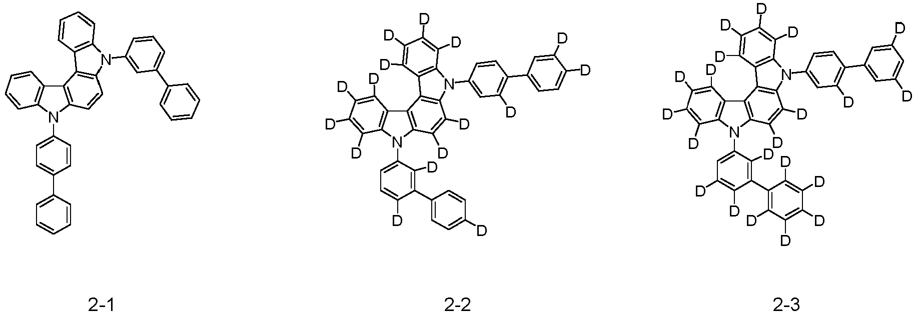

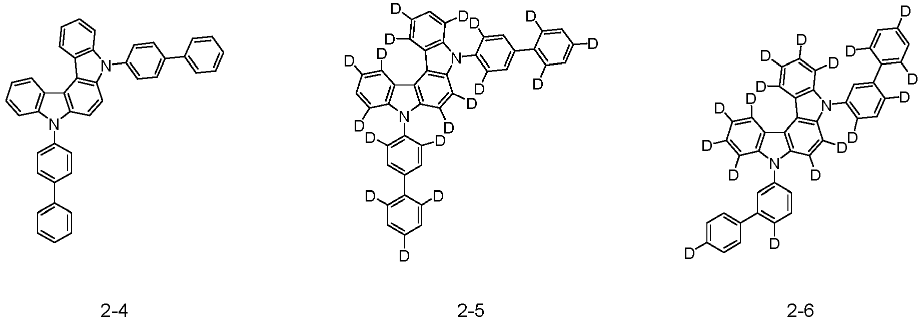

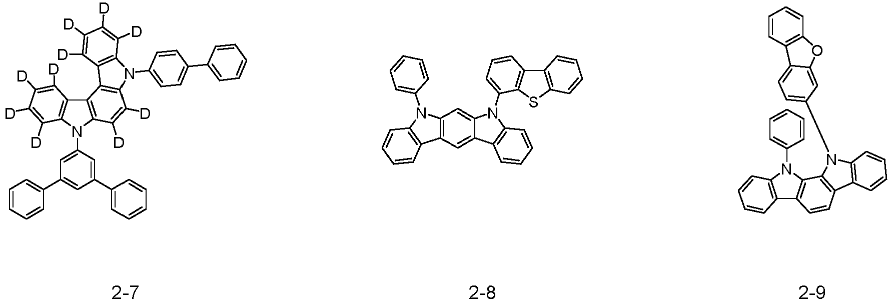

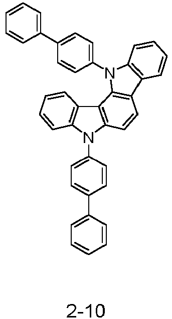

### Comparative Examples 1 to 9

An organic light emitting device was manufactured in the same manner as in Example 1, except that the compound shown in Table 1 was used instead of Compound 1-1 and Compound 2-1 in Example 1.

At this time, Compounds H-2 and C1 to C3 listed in Table 1 are as follows.

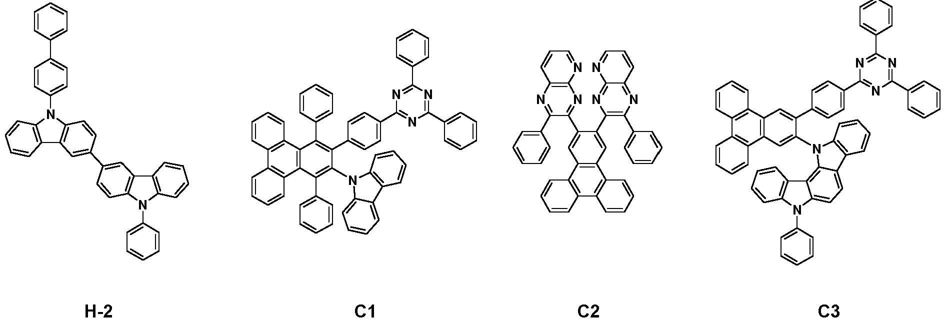

### Experimental Examples

For the organic light emitting devices prepared in Examples and Comparative Examples, the voltage, efficiency, and lifespan (T95) were measured by applying a current, and the results are shown in Table 1 below. Herein, the voltage and efficiency were measured by applying a current density of 10 mA/cm². In addition, T95 in Table 1 means the time taken until the initial luminance decreases to 95% at a current density of 20 mA/cm².

**[Table 1]**

| Category | Compound in light emitting layer | Voltage (V) (@10mA/cm²) | Efficiency (cd/A) (@10mA/cm²) | Emission color | T95(hr) (@20mA/cm²) |
|---|---|---|---|---|---|
| Example 1 | Compound 1-1, Compound 2-1 | 3.02 | 69.8 | Green | 80 |
| Example 2 | Compound 1-2, Compound 2-1 | 3.02 | 69.8 | Green | 86 |
| Example 3 | Compound 1-3, Compound 2-4 | 3.01 | 70.0 | Green | 90 |
| Example 4 | Compound 1-3, Compound 2-5 | 3.01 | 70.0 | Green | 101 |
| Example 5 | Compound 1-3, Compound 2-1 | 3.00 | 72.1 | Green | 81 |
| Example 6 | Compound 1-4, Compound 2-2 | 3.01 | 71.1 | Green | 85 |
| Example 7 | Compound 1-5, Compound 2-6 | 3.04 | 72.3 | Green | 89 |
| Example 8 | Compound 1-6, Compound 2-5 | 3.08 | 71.5 | Green | 84 |
| Example 9 | Compound 1-6, Compound 2-6 | 3.09 | 70.2 | Green | 81 |
| Example 10 | Compound 1-7, Compound 2-7 | 2.98 | 70.5 | Green | 82 |
| Example 11 | Compound 1-8, Compound 2-8 | 3.07 | 73.5 | Green | 80 |
| Example 12 | Compound 1-9, Compound 2-9 | 3.02 | 72.1 | Green | 87 |
| Example 13 | Compound 1-10, Compound 2-10 | 3.03 | 71.3 | Green | 86 |
| Comparative Example 1 | Compound 1-2 | 3.15 | 62.1 | Green | 50 |
| Comparative Example 2 | Compound 1-8 | 3.26 | 59.1 | Green | 40 |
| Comparative Example 3 | Compound 1-10 | 3.12 | 62.0 | Green | 53 |
| Comparative Example 4 | Compound C1 | 3.51 | 55.1 | Green | 41 |
| Comparative Example 5 | Compound C2 | 3.48 | 53.9 | Green | 29 |
| Comparative Example 6 | Compound C3 | 3.26 | 51.0 | Green | 32 |
| Comparative Example 7 | Compound C1, Compound H-2 | 3.30 | 59.5 | Green | 55 |
| Comparative Example 8 | Compound C1, Compound 2-1 | 3.20 | 68.2 | Green | 70 |
| Comparative Example 9 | Compound 1-10, Compound H-2 | 3.25 | 69.6 | Green | 75 |

As shown in Table 1, it was confirmed that the organic light emitting devices of Examples using both the first compound and the second compound of the present disclosure as hosts exhibited superior characteristics in terms of efficiency and lifespan compared to the organic light emitting devices of Comparative Examples 1 to 3 using only the first compound and the organic light emitting devices of Comparative Examples 4 and 7 in which neither the first compound nor the second compound was used.

In addition, the organic light emitting devices of Examples exhibited higher efficiency and superior lifespan compared to the organic light emitting devices of Comparative Examples 8 and 9 using two kinds of hosts but employing a combination of other hosts instead of the combination of the first compound and the second compound.

This means that the organic light emitting devices employing the compound of the present disclosure exhibit significantly improved device characteristics compared to the devices of Comparative Examples, considering that the luminous efficiency and lifespan of the organic light emitting device generally have a trade-off relationship.

**[DESCRIPTION OF SYMBOLS]**

| | | | |
|---|---|---|---|
| 1: | Substrate | 2: | Anode |
| 3: | Light emitting layer | 4: | Cathode |
| 5: | Hole injection layer | 6: | Hole transport layer |
| 7: | Electron blocking layer | 8: | Hole blocking layer |
| 9: | Electron transport and injection layer | | |

## Claims

1. An organic light emitting device comprising:
an anode (2);
a cathode (4) that is provided opposite to the anode; and
a light emitting layer (3) that is provided between the anode and the cathode
wherein the light emitting layer comprises a first compound represented by the following Chemical Formula 1 and a second compound represented by the following Chemical Formula 2: 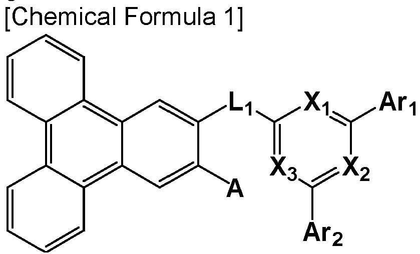 in the Chemical Formula 1,
A is substituted or unsubstituted carbazolyl,
L₁ is a single bond, or substituted or unsubstituted C₆₋₆₀ arylene,
X₁, X₂ and X₃ are each independently N, or CH, provided that at least one of X₁, X₂ and X₃ is N, and
Ar₁ and Ar₂ are each independently substituted or unsubstituted C₆₋₆₀ aryl, or substituted or unsubstituted C₂₋₆₀ heteroaryl containing at least one heteroatom selected from the group consisting of N, O and S, 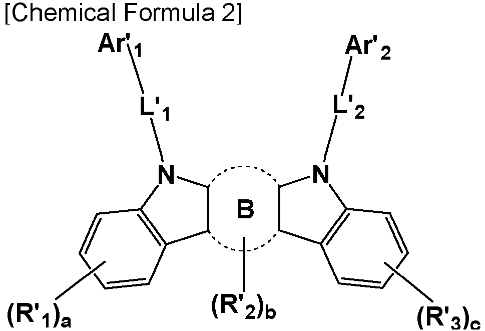 in the Chemical Formula 2,
B is a benzene ring fused with two adjacent pentagonal rings,
L'₁ and L'₂ are each independently a single bond, or substituted or unsubstituted C₆₋₆₀ arylene,
Ar'₁ and Ar'₂ are each independently substituted or unsubstituted C₆₋₆₀ aryl, or substituted or unsubstituted C₂₋₆₀ heteroaryl containing at least one heteroatom selected from the group consisting of N, O and S,
R'₁, R'₂ and R'₃ are each independently hydrogen, deuterium, substituted or unsubstituted C₆₋₆₀ aryl, or substituted or unsubstituted C₂₋₆₀ heteroaryl containing at least one heteroatom selected from the group consisting of N, O and S,
a is an integer of 0 to 4,
b is an integer of 0 to 2, and
c is an integer of 0 to 4.

2. The organic light emitting device of Claim 1,
wherein the first compound is represented by the following Chemical Formula 1-1 or Chemical Formula 1-2: 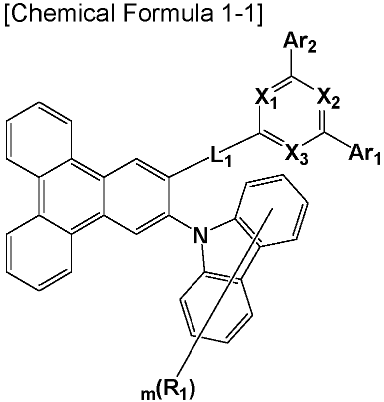 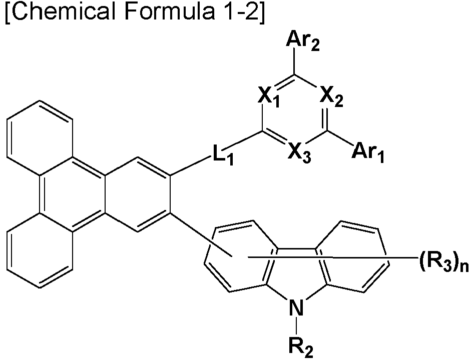 in the Chemical Formula 1-1 or Chemical Formula 1-2,
L₁, X₁, X₂, X₃, Ar₁, and Ar₂ are as defined in Claim 1,
R₁ are each independently hydrogen, deuterium, substituted or unsubstituted C₁₋₆₀ alkyl, substituted or unsubstituted C₆₋₆₀ aryl, or substituted or unsubstituted C₂₋₆₀ heteroaryl containing at least one heteroatom selected from the group consisting of N, O and S,
R₂ is substituted or unsubstituted C₁₋₆₀ alkyl, or substituted or unsubstituted C₆₋₆₀ aryl,
R₃ are each independently hydrogen, deuterium, substituted or unsubstituted C₁₋₆₀ alkyl, substituted or unsubstituted C₆₋₆₀ aryl, or substituted or unsubstituted C₅₋₆₀ heteroaryl containing at least one heteroatom selected from the group consisting of N, O and S,
m is an integer of 0 to 8, and
n is an integer of 0 to 7.

3. The organic light emitting device of Claim 1,
wherein L₁ is a single bond, phenylene or biphenylylene, and
the phenylene and biphenylylene are each independently unsubstituted or substituted with at least one deuterium.

4. The organic light emitting device of Claim 1,
wherein Ar₁ and Ar₂ are each independently phenyl, biphenylyl, terphenylyl, dimethylfluorenyl, naphthyl, phenanthrenyl, triphenylenyl, dibenzofuranyl, dibenzothiophenyl, carbazol-9-yl, 9-phenyl-9H-carbazolyl, benzoxazolyl, benzothiazolyl, 2-phenylbenzoxazolyl or 2-phenylbenzothiazolyl, and
they are each independently unsubstituted or substituted with at least one deuterium.

5. The organic light emitting device of Claim 2,
wherein R₁ are each independently hydrogen, deuterium, phenyl, dibenzofuranyl, dibenzothiophenyl, or carbazolyl, and
the phenyl, dibenzofuranyl, dibenzothiophenyl, and carbazolyl are each independently unsubstituted or substituted with at least one deuterium.

6. The organic light emitting device of Claim 2,
wherein R₂ are each independently phenyl unsubstituted or substituted with at least one deuterium.

7. The organic light emitting device of Claim 2,
wherein R₃ are each independently hydrogen, deuterium, phenyl, dibenzofuranyl, dibenzothiophenyl, or carbazolyl, and
the phenyl, dibenzofuranyl, dibenzothiophenyl, and carbazolyl are each independently unsubstituted or substituted with at least one deuterium.

8. The organic light emitting device of Claim 1,
wherein the first compound is any one selected from the group consisting of the following compounds:

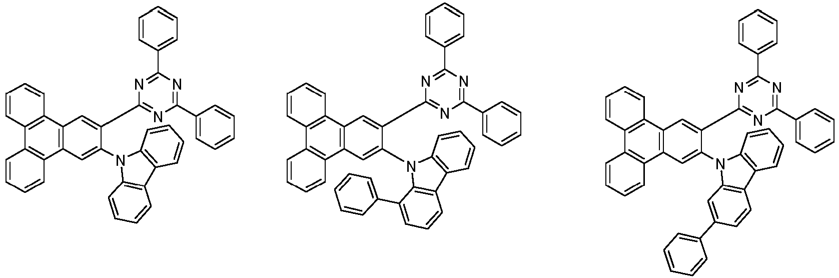

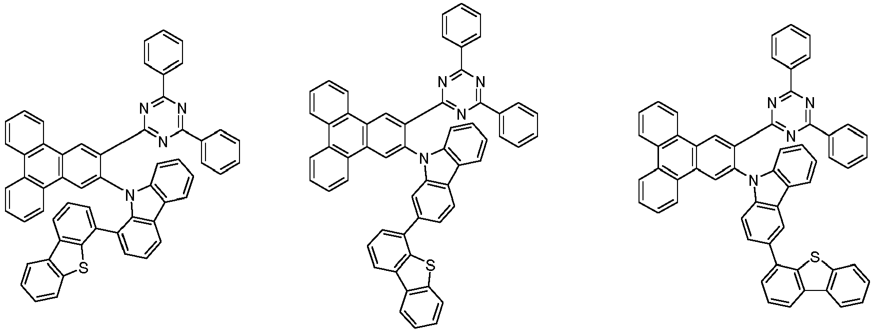

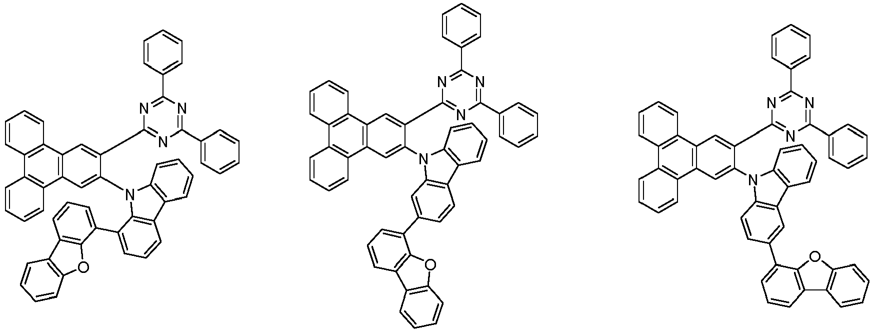

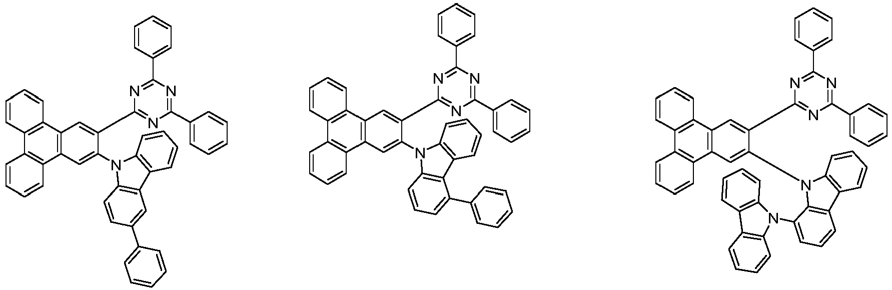

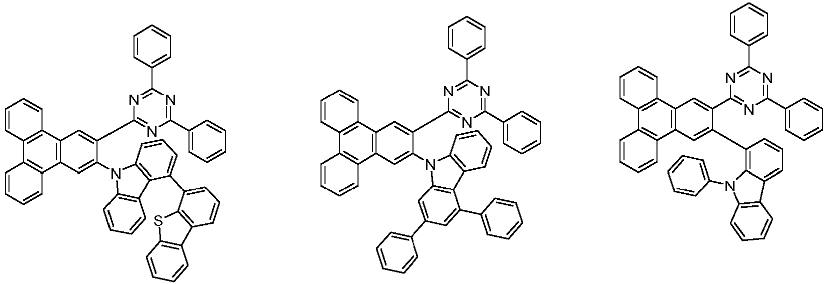

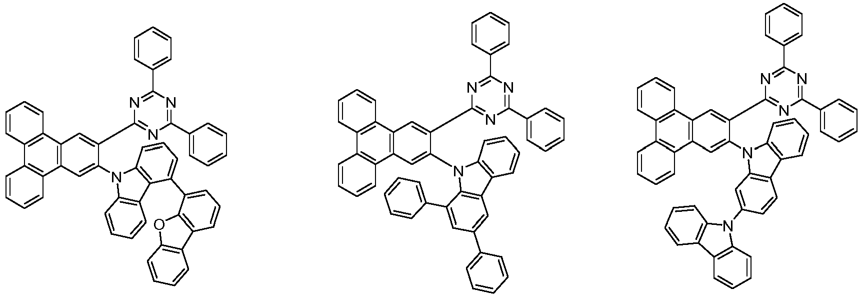

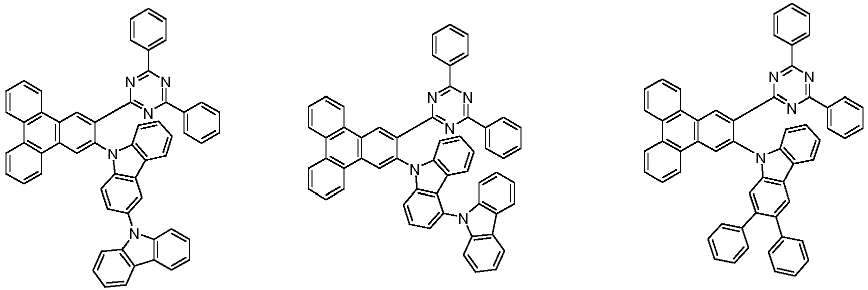

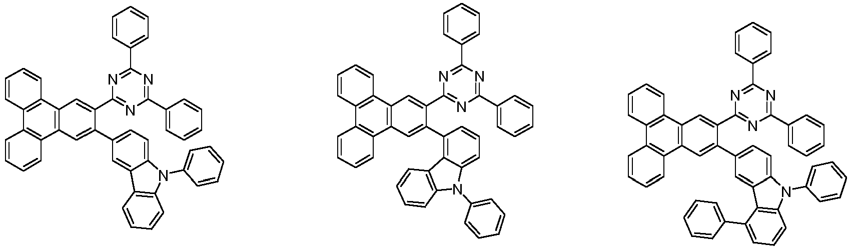

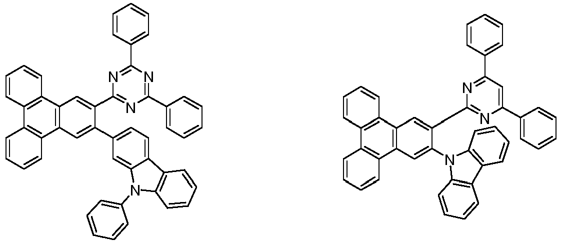

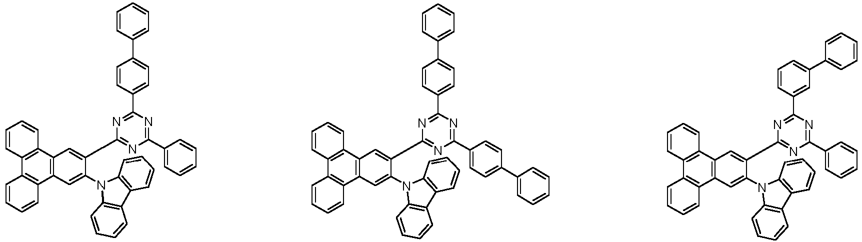

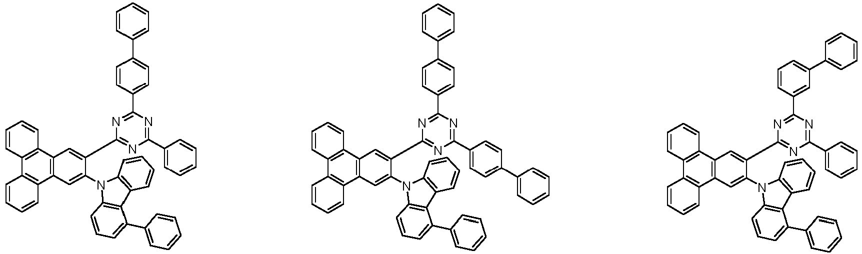

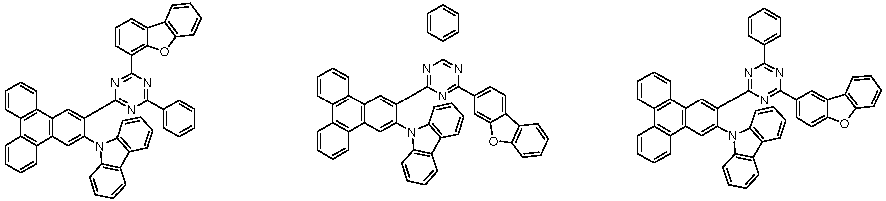

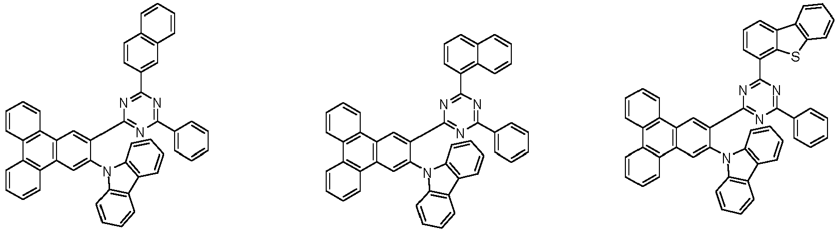

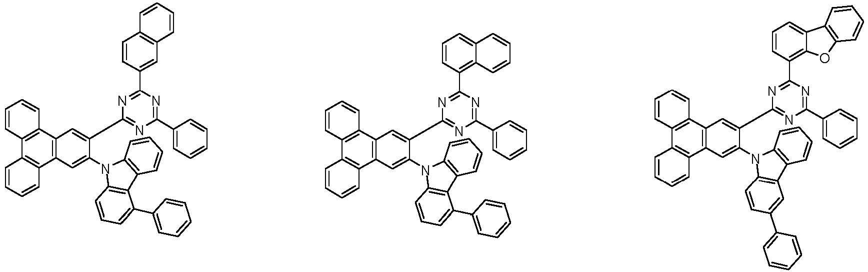

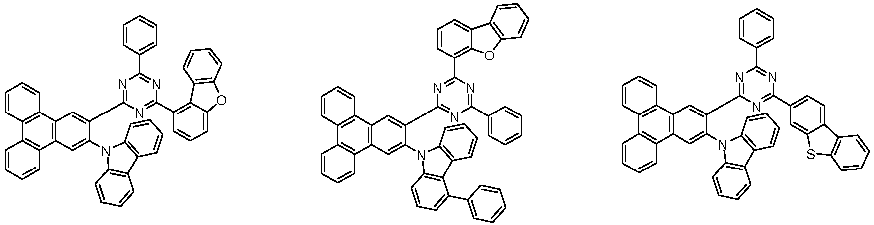

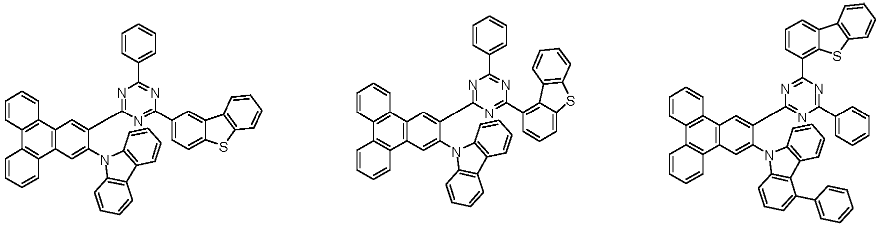

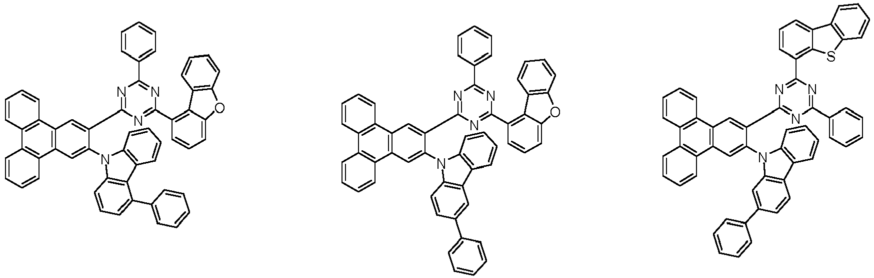

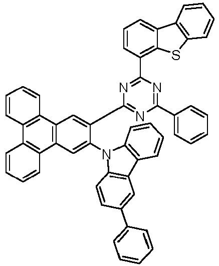

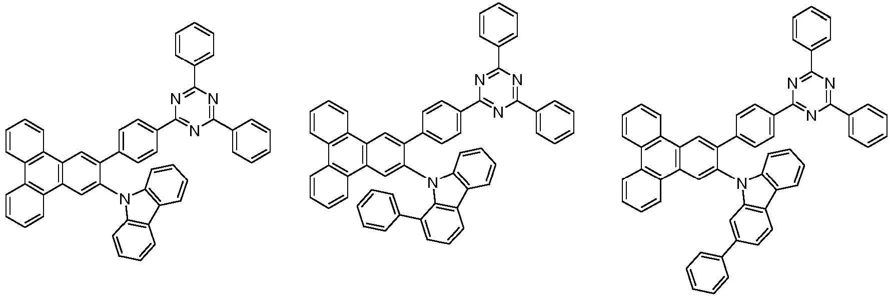

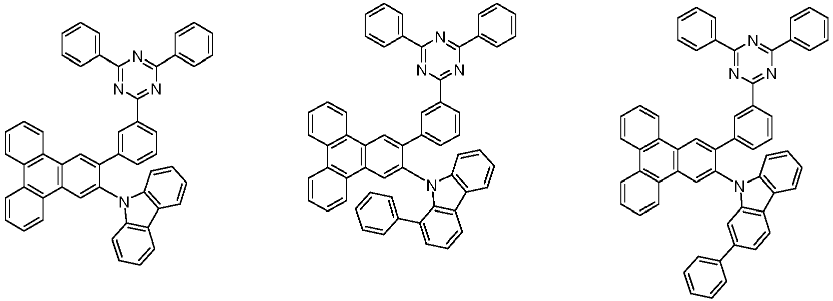

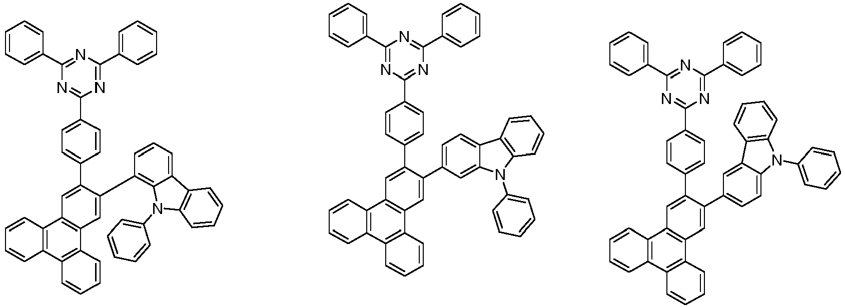

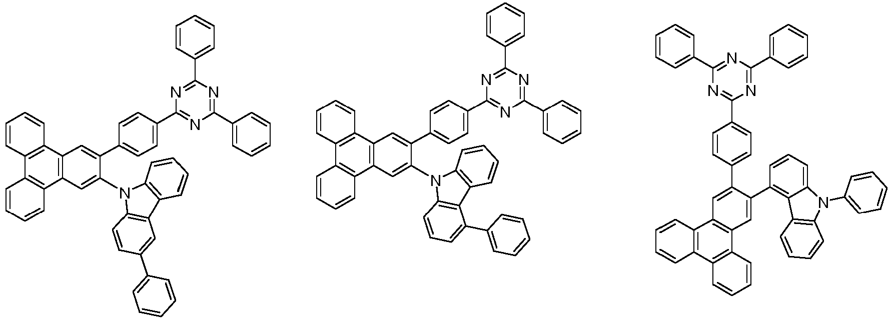

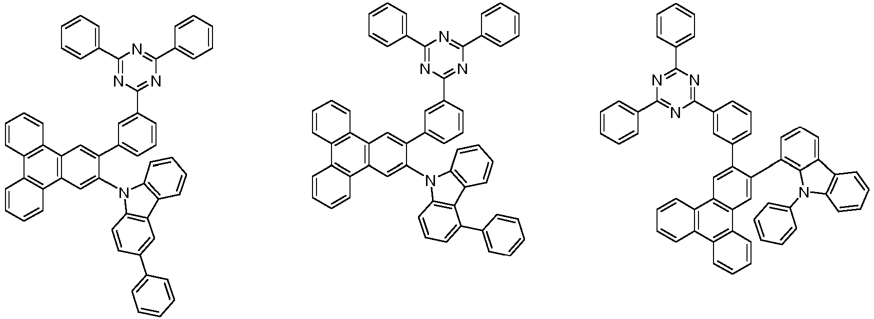

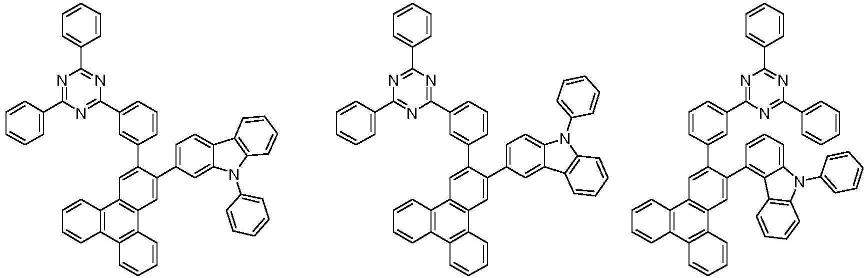

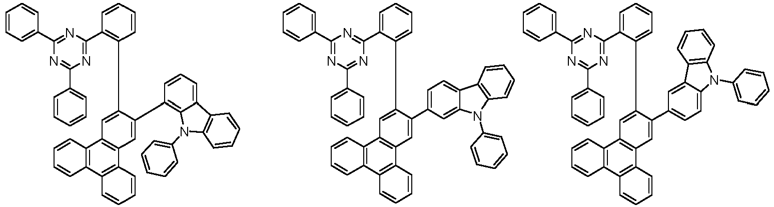

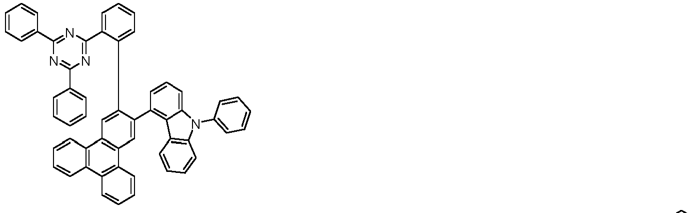

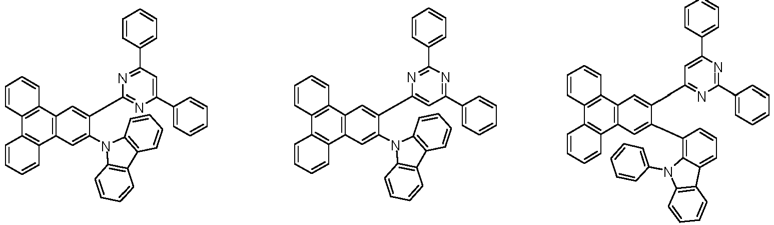

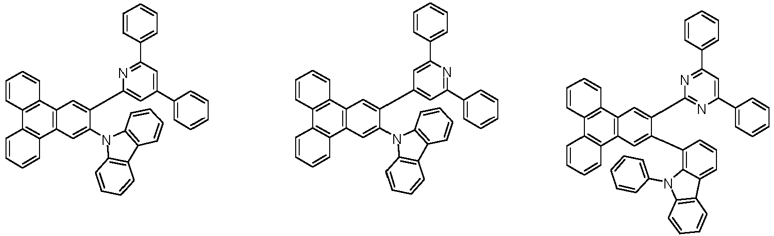

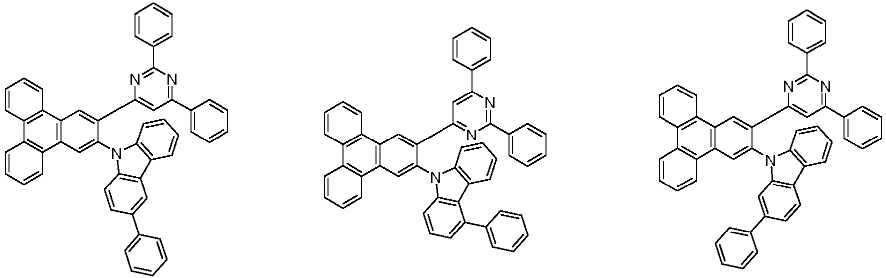

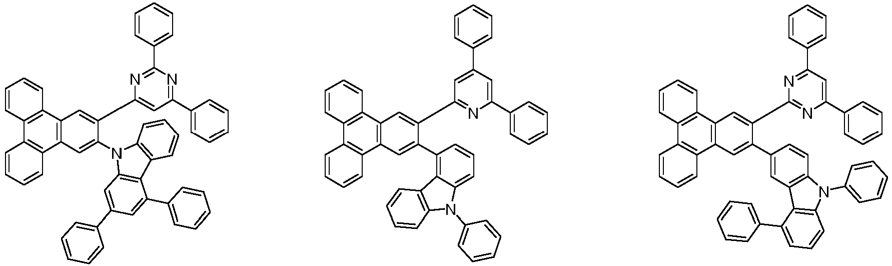

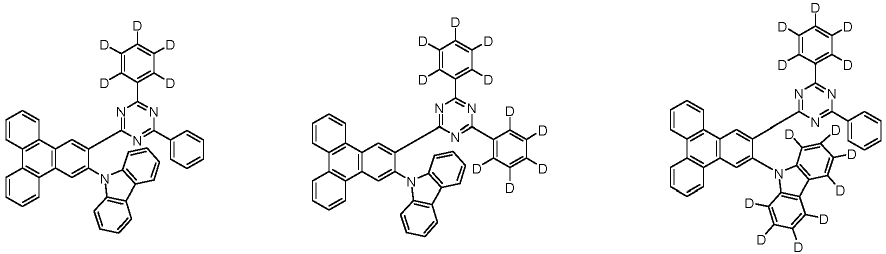

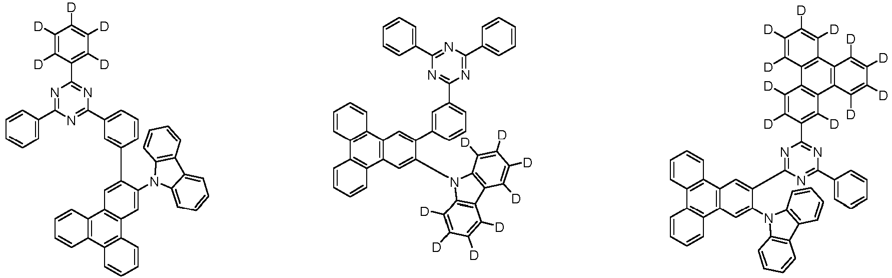

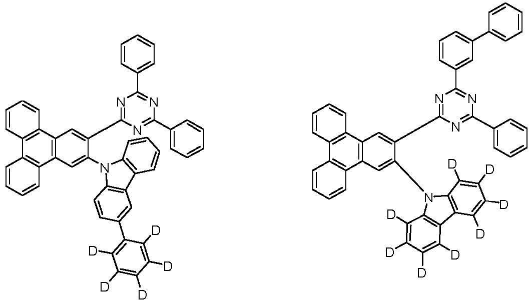

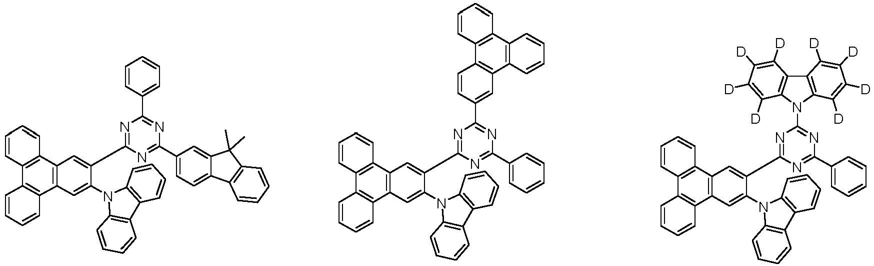

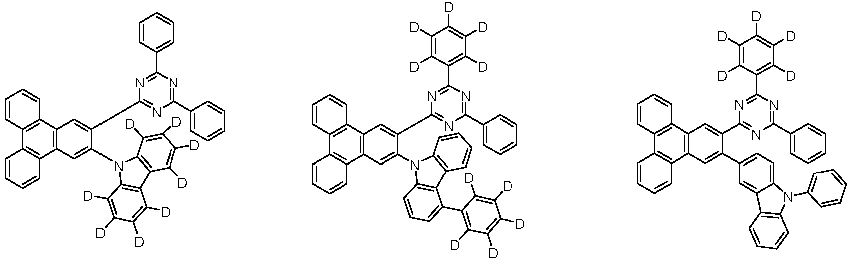

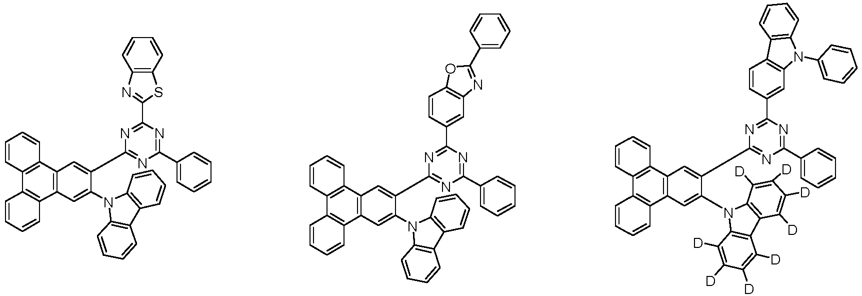

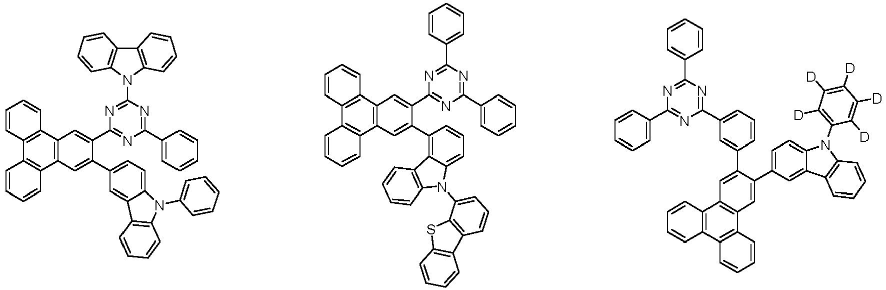

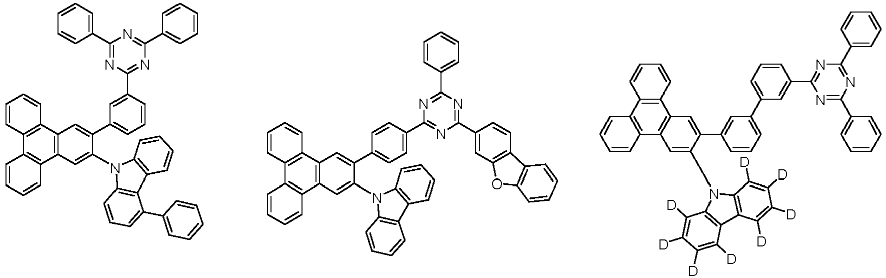

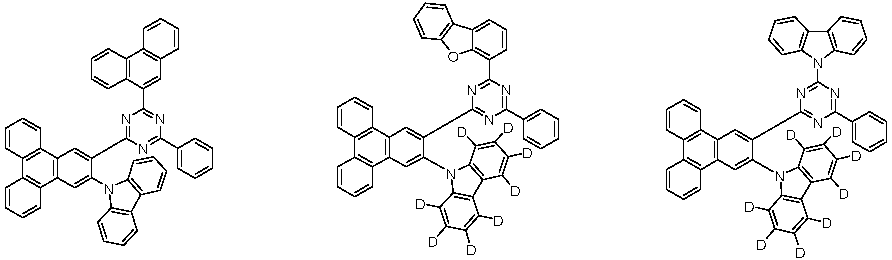

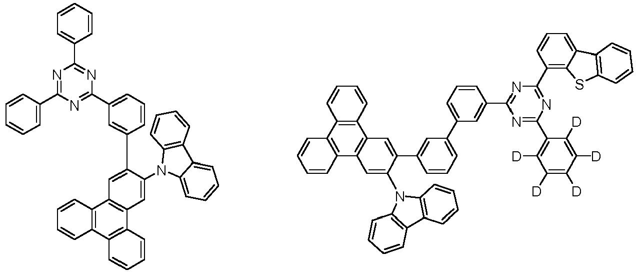

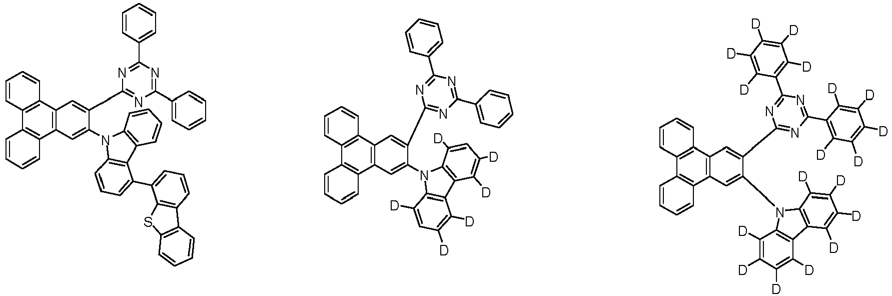

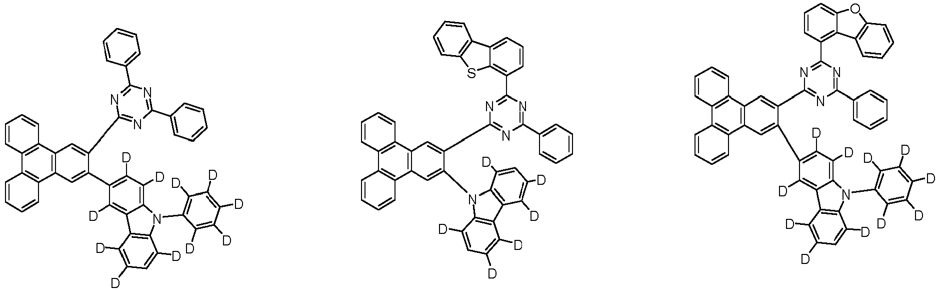

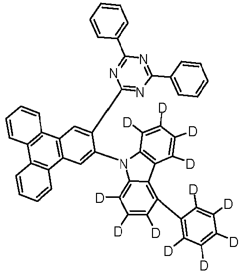

9. The organic light emitting device of Claim 1,
wherein the second compound is represented by any one of the following Chemical Formulae 2-1 to 2-5: 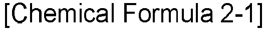 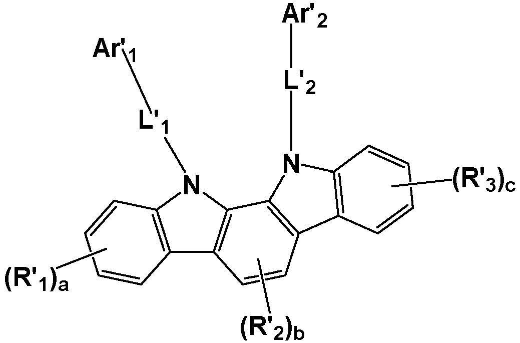 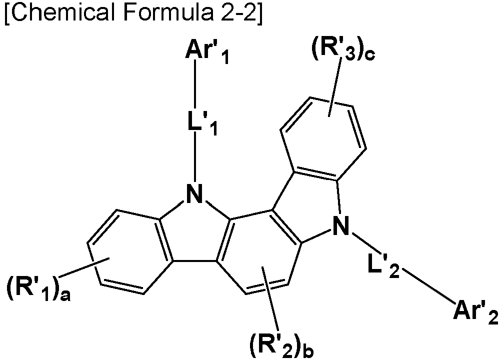 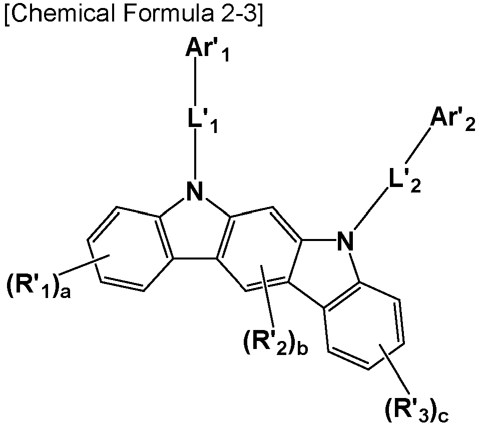 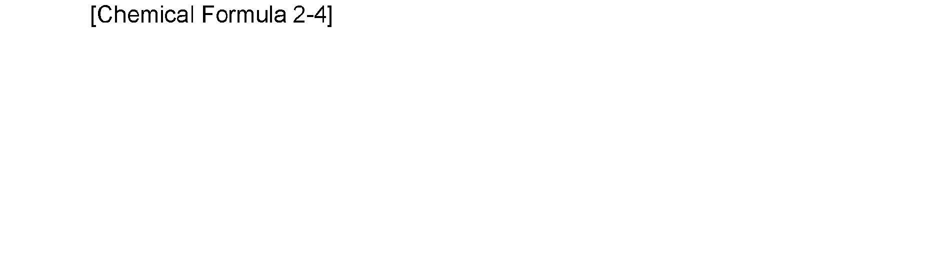 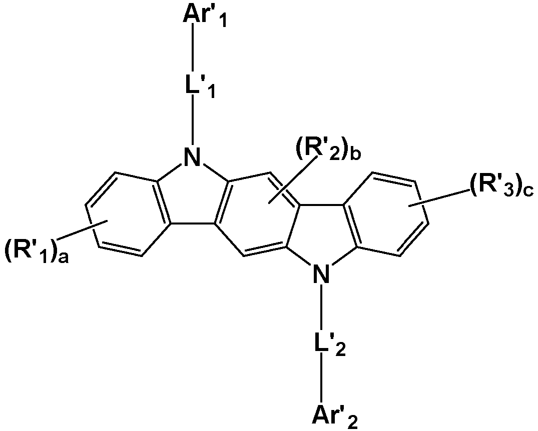 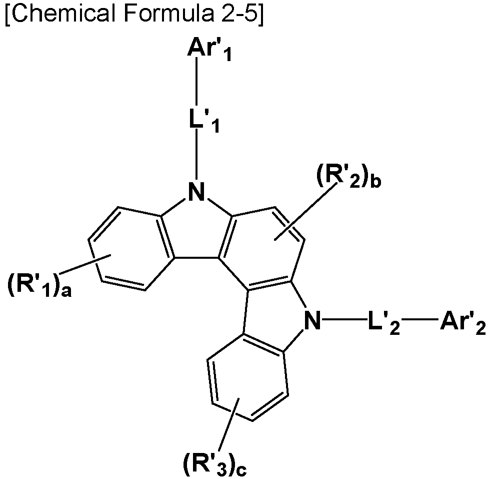 in the Chemical Formulae 2-1 to 2-5,
L'₁, L'₂, Ar'₁, Ar'₂, R'₁, R'₂, R'₃, a, b and c are as defined in Claim 1.

10. The organic light emitting device of Claim 1,
wherein L'₁ and L'₂ are each independently a single bond, phenylene or biphenylylene, and
the phenylene and biphenylylene are each independently unsubstituted or substituted with at least one deuterium.

11. The organic light emitting device of Claim 1,
wherein Ar'₁ and Ar'₂ are each independently phenyl, biphenylyl, terphenylyl, naphthyl, phenanthrenyl, triphenylenyl, fluorenyl, dibenzofuranyl, dibenzothiophenyl, carbazole-9-yl, or 9-phenyl-9H-carbazolyl, and
Ar'₁ and Ar'₂ are each independently unsubstituted or substituted with at least one substituent selected from the group consisting of deuterium, C₁₋₁₀ alkyl and C₆₋₂₀ aryl.

12. The organic light emitting device of Claim 1,
wherein R'₁, R'₂ and R'₃ are each independently hydrogen, deuterium, phenyl, dibenzofuranyl, dibenzothiophenyl, carbazol-9-yl, or 9-phenyl-9H-carbazolyl, and
the phenyl, dibenzofuranyl, dibenzothiophenyl, carbazol-9-yl, and 9-phenyl-9H-carbazolyl are each independently unsubstituted or substituted with at least one deuterium.

13. The organic light emitting device of Claim 1,
wherein the second compound is any one selected from the group consisting of the following compounds:

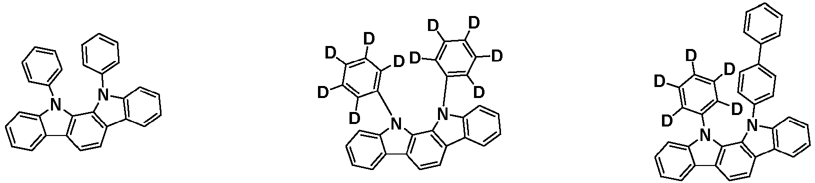

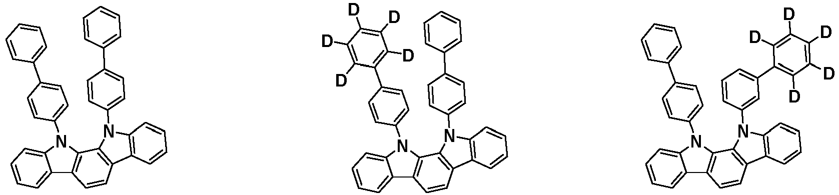

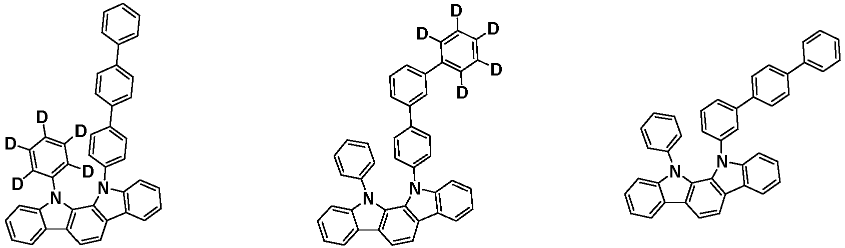

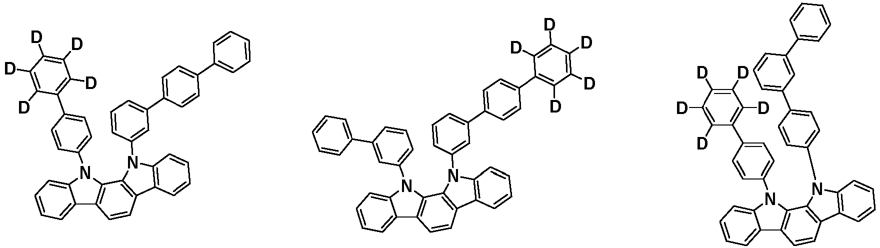

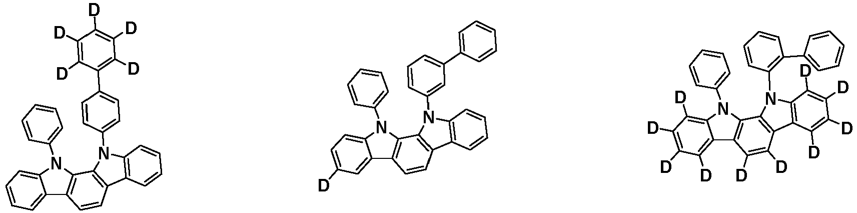

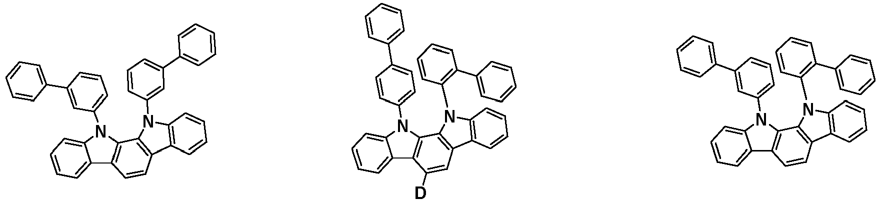

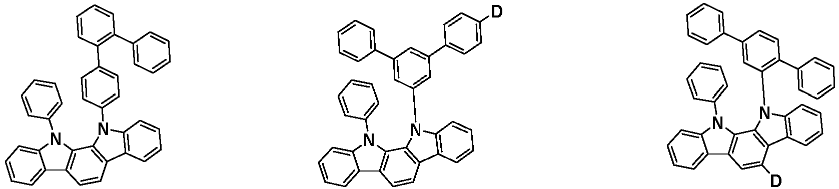

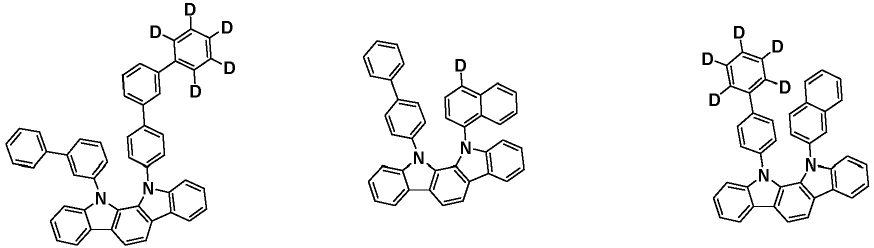

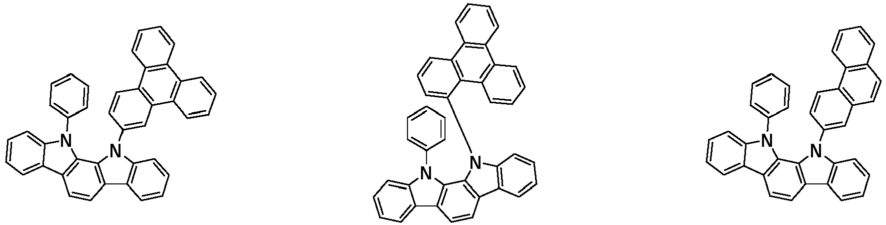

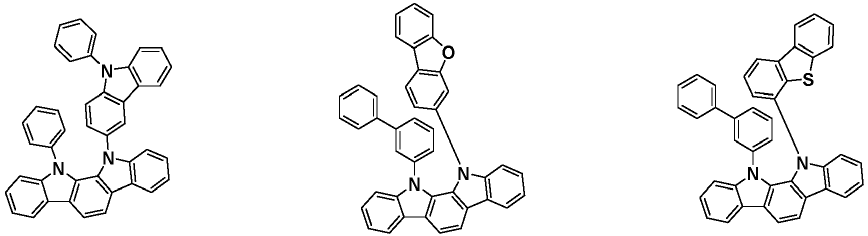

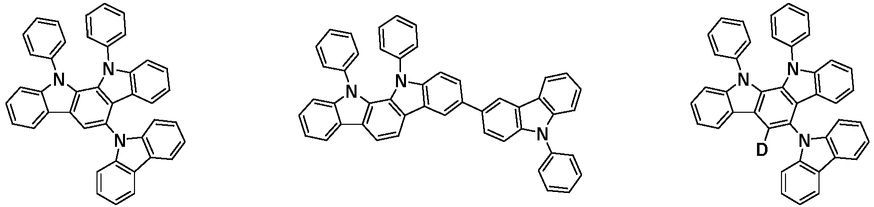

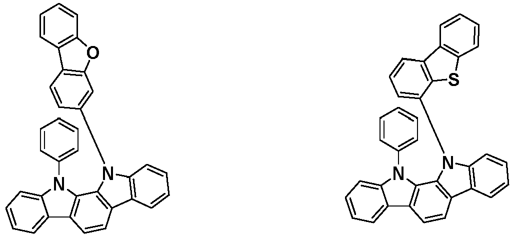

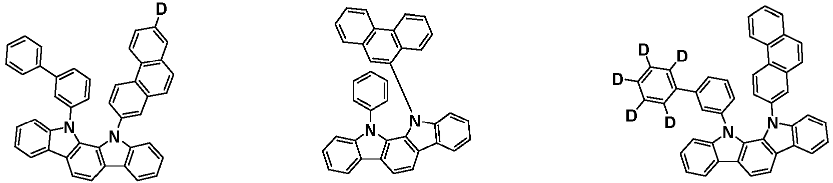

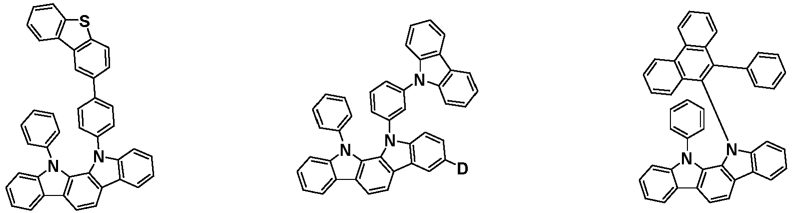

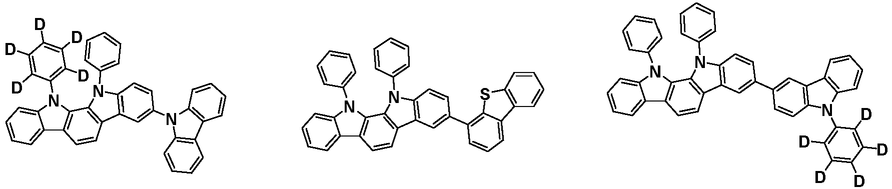

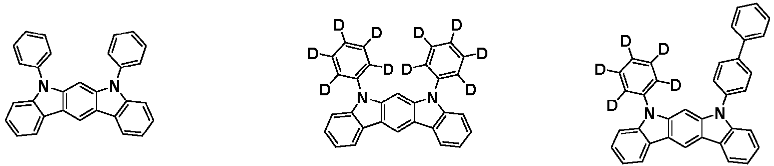

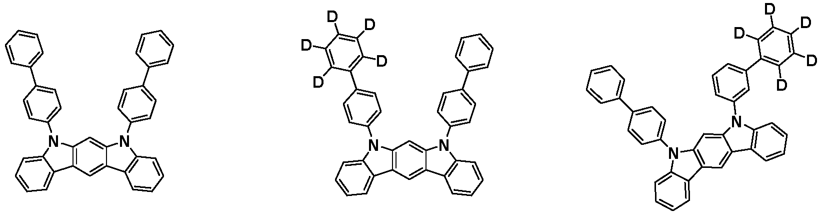

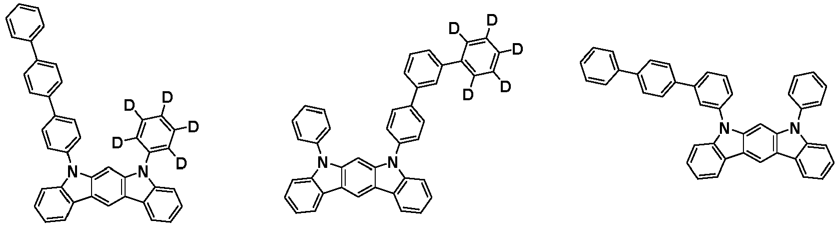

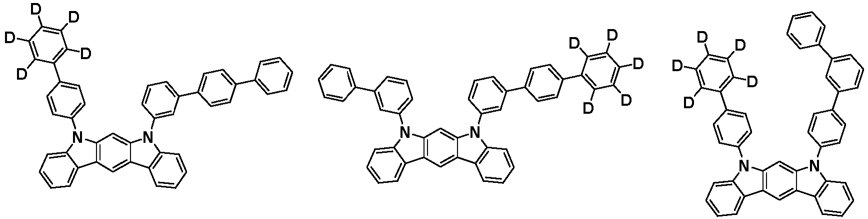

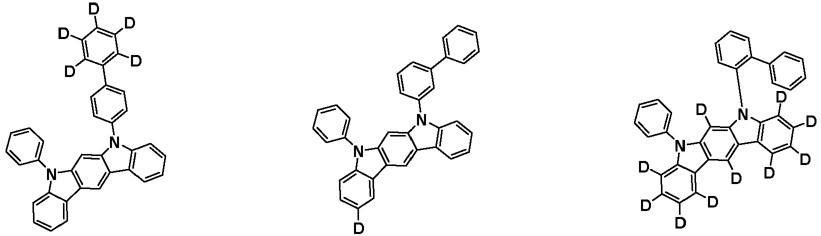

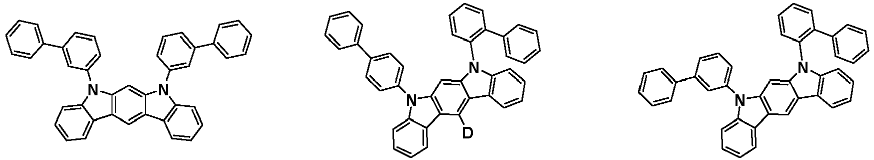

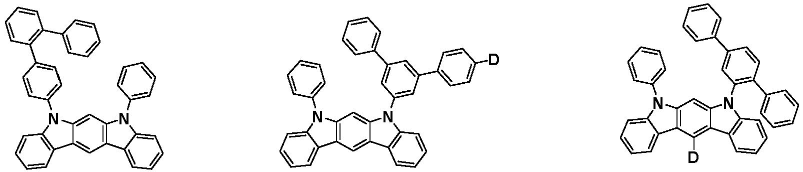

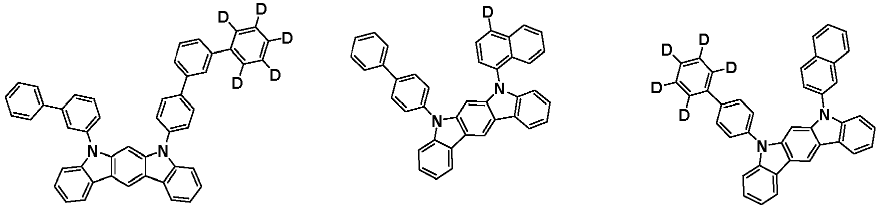

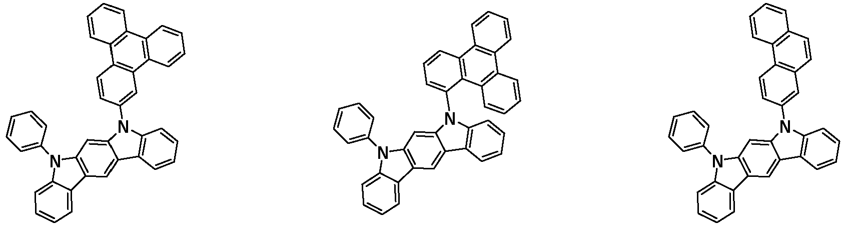

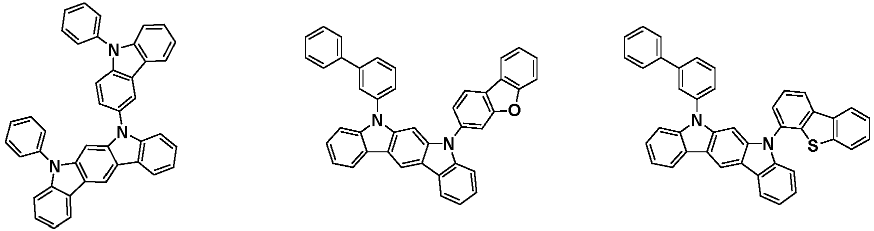

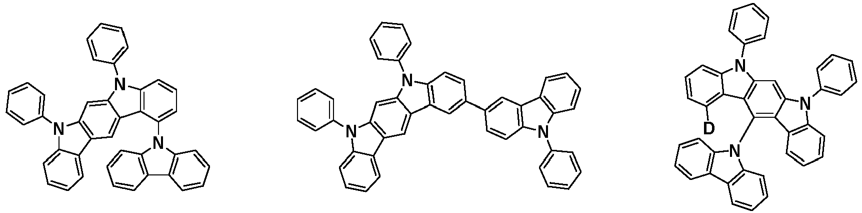

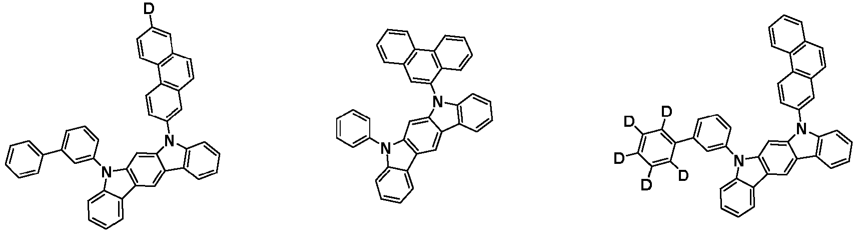

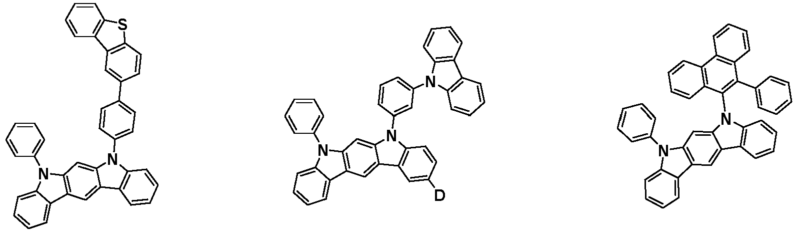

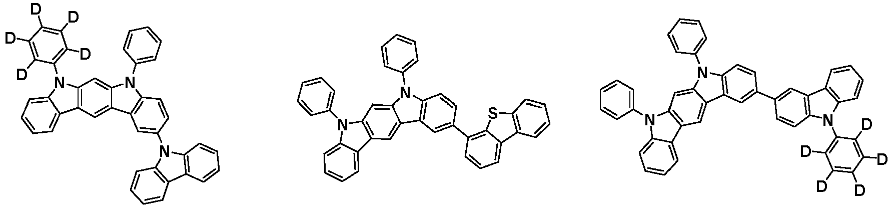

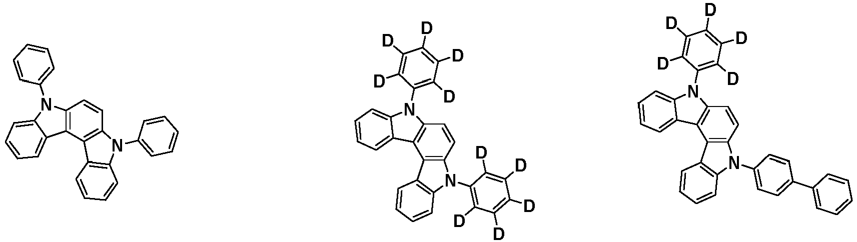

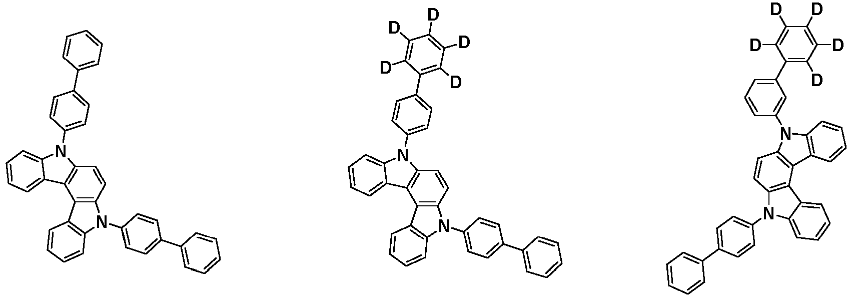

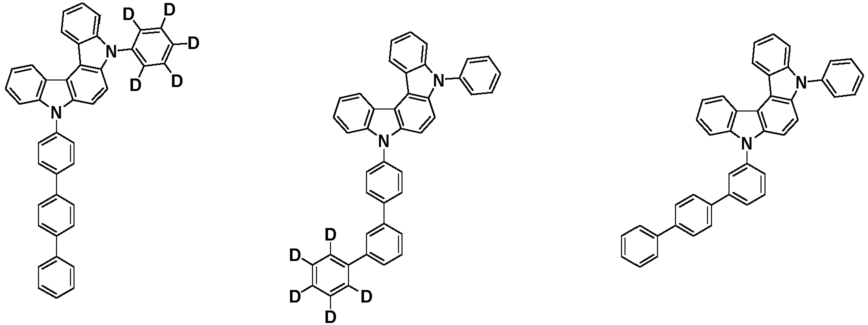

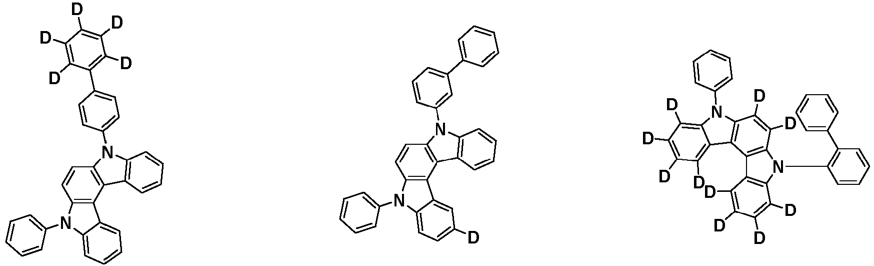

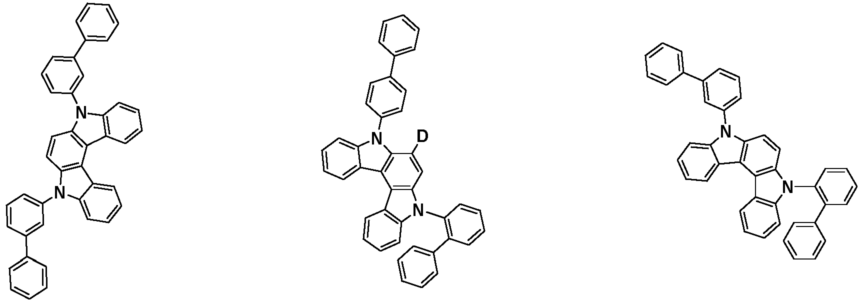

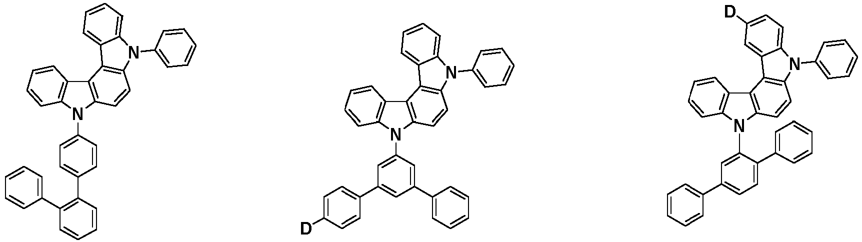

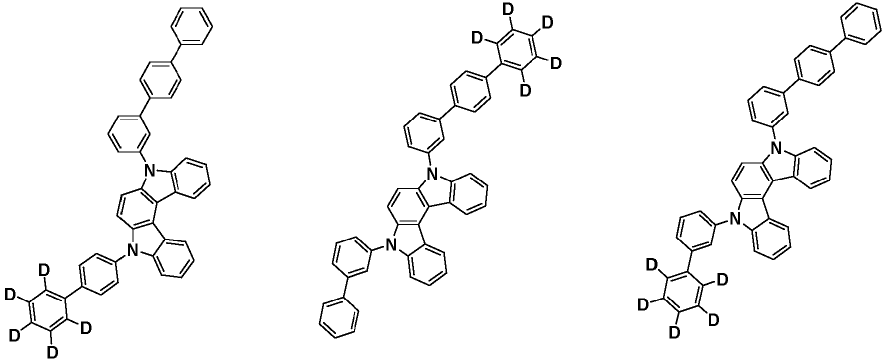

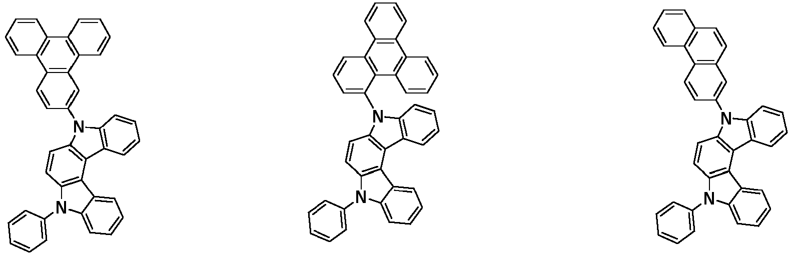

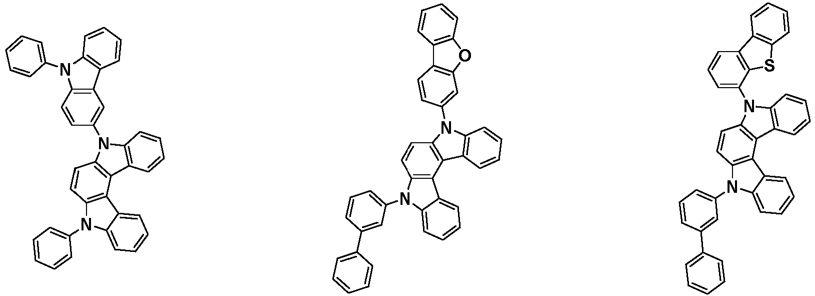

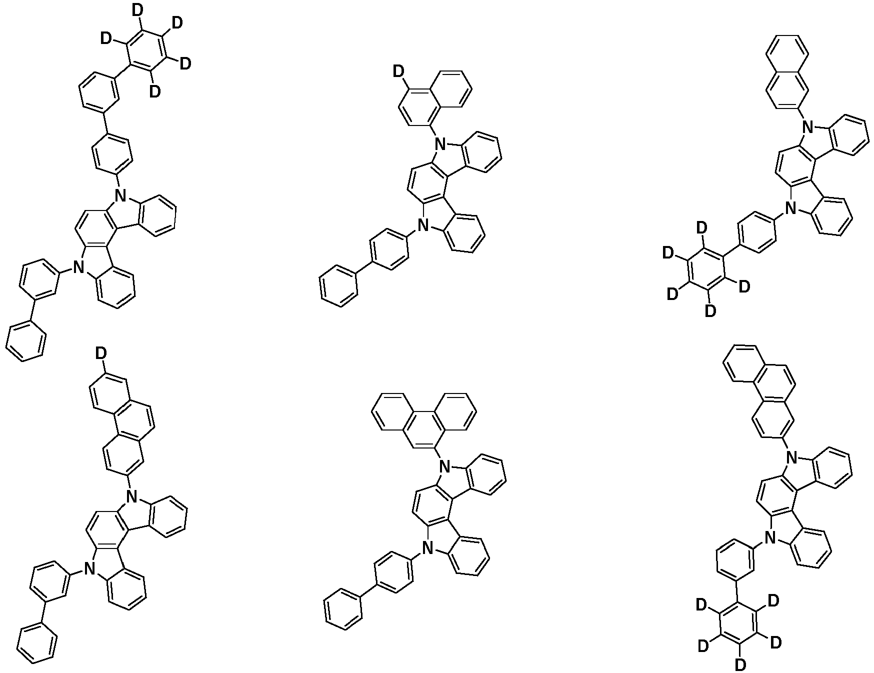

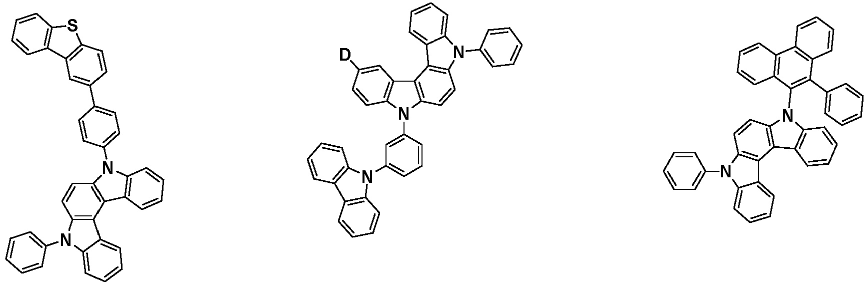

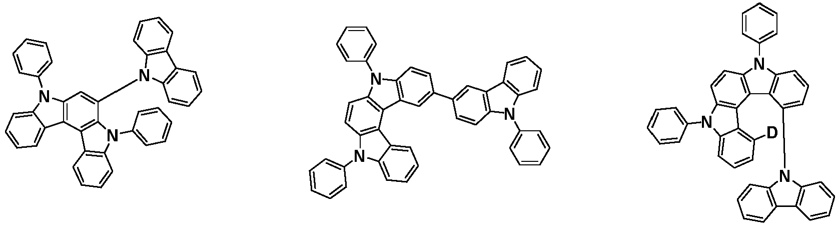

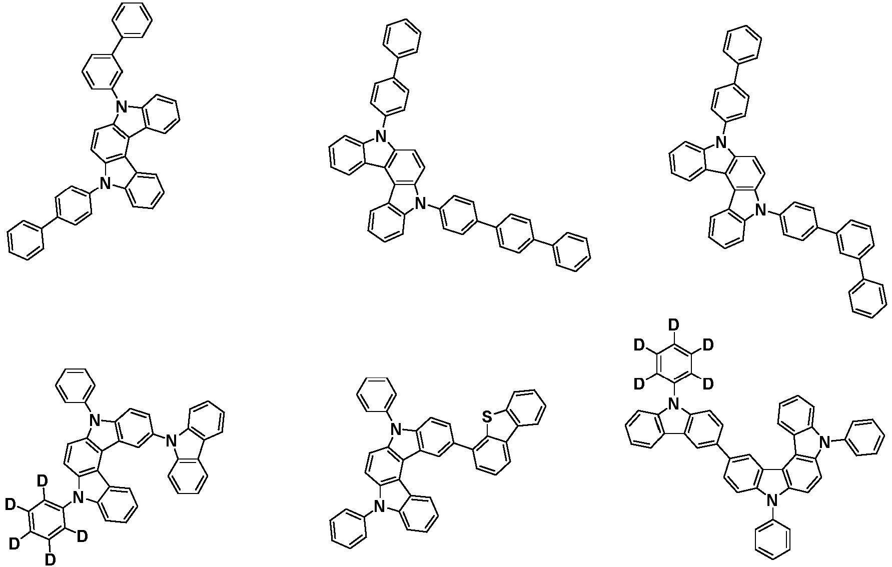

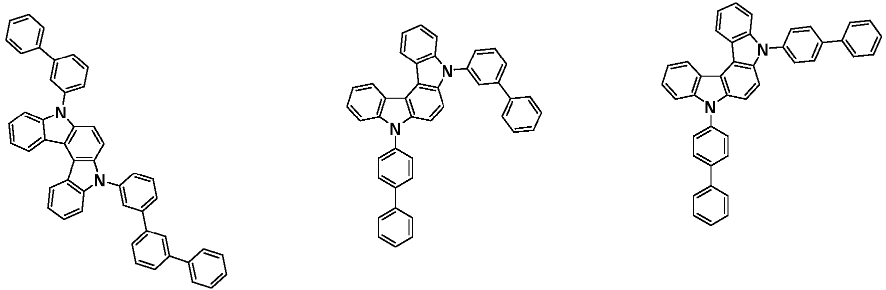

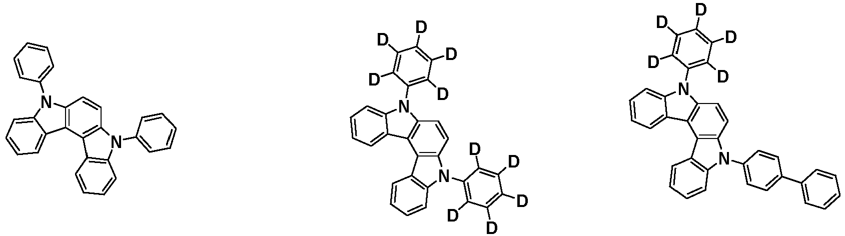

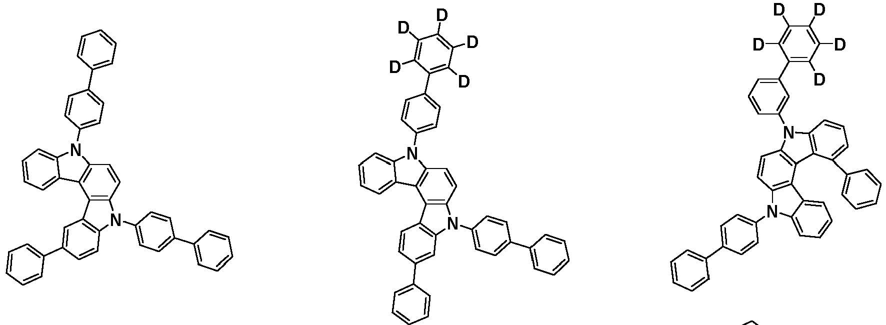

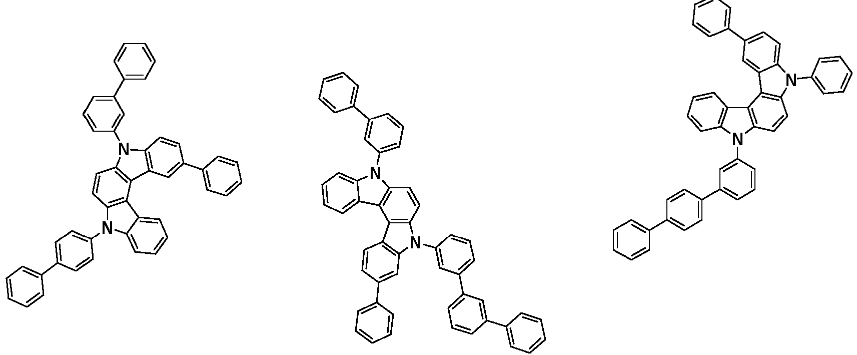

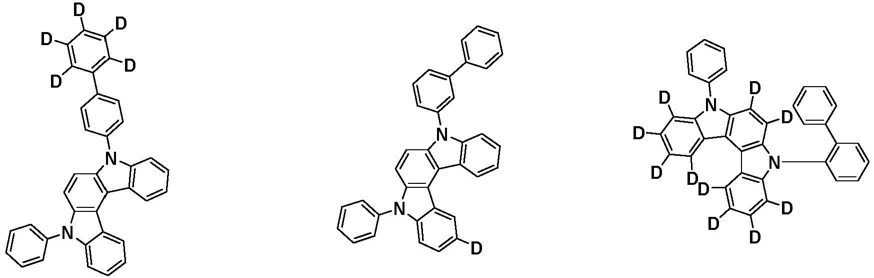

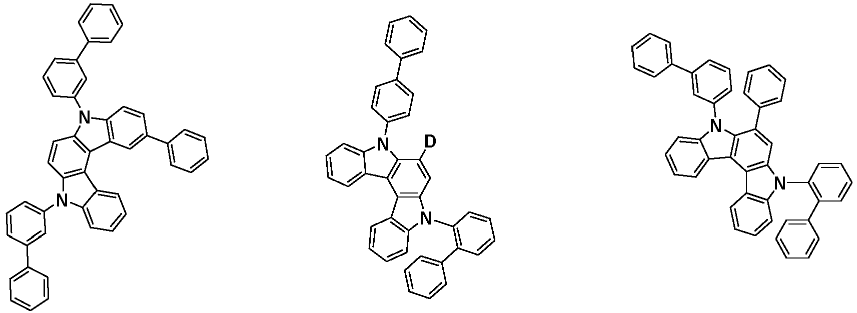

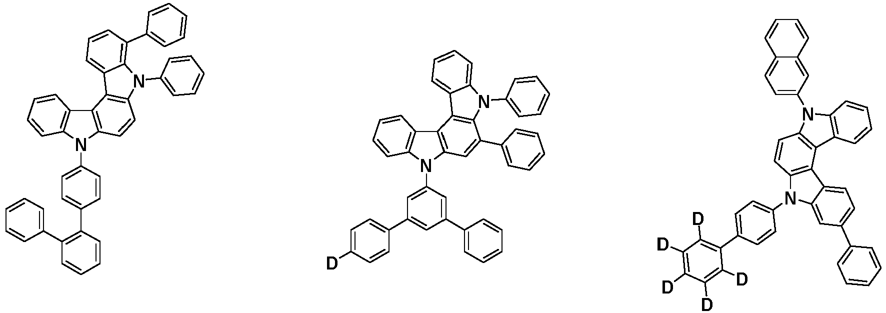

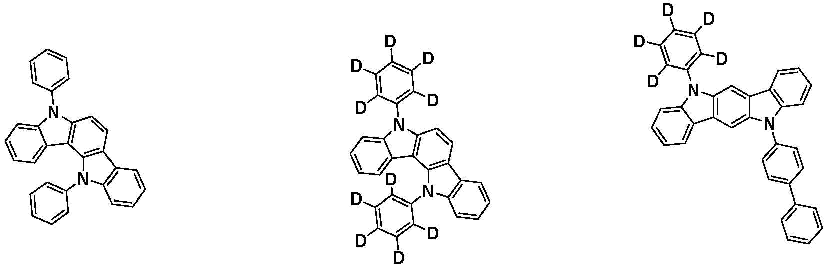

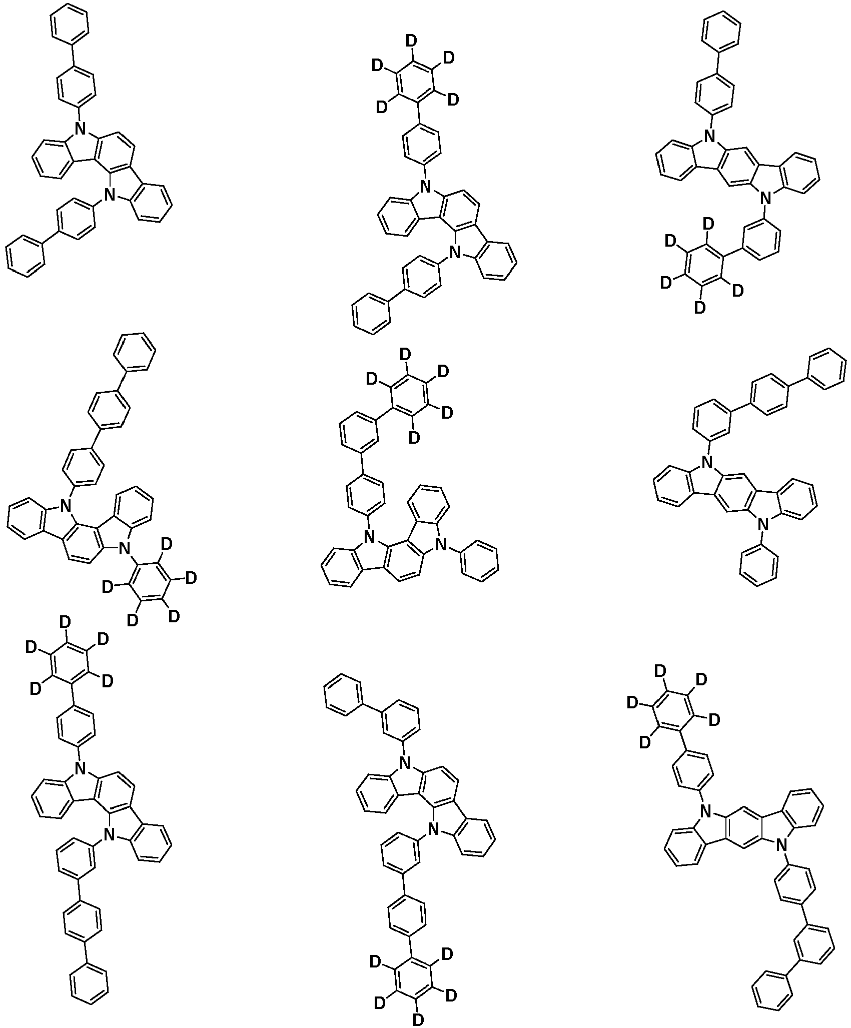

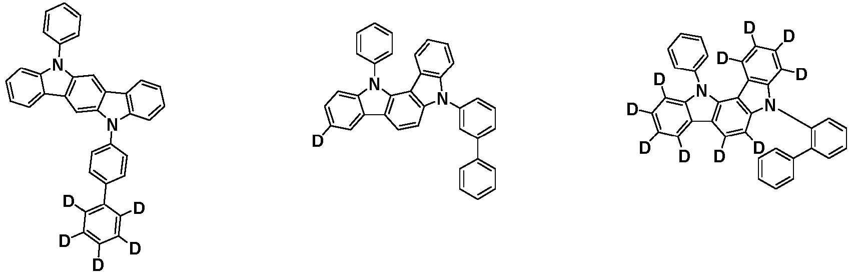

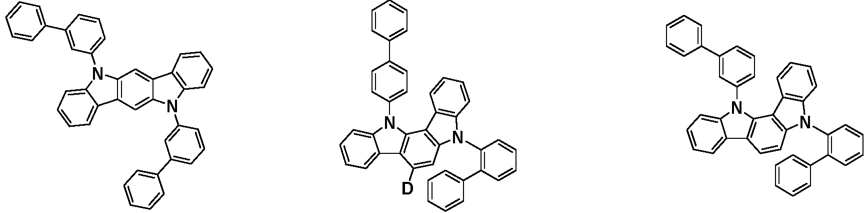

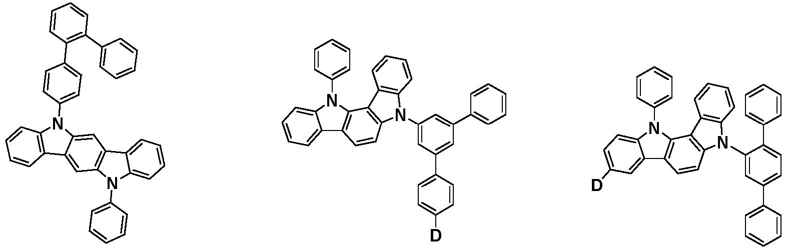

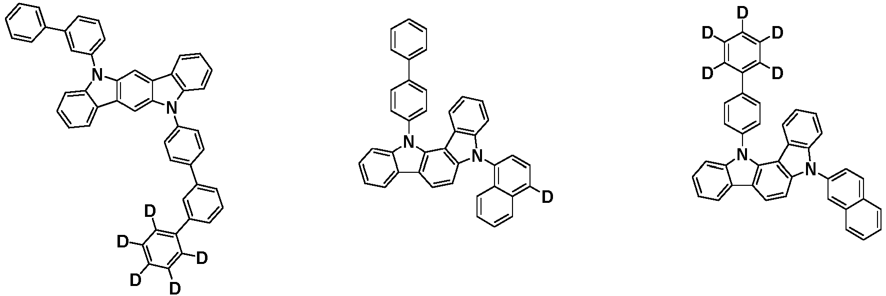

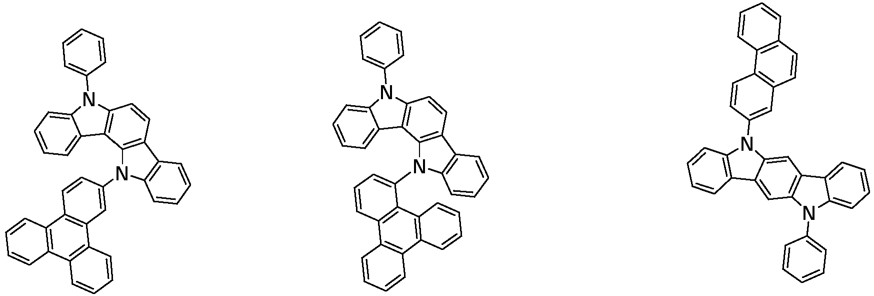

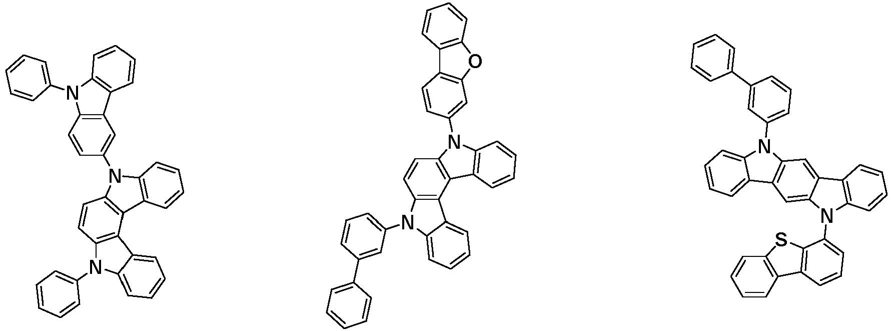

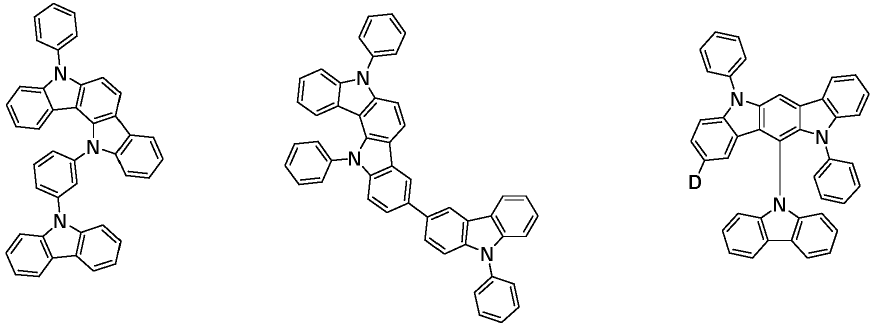

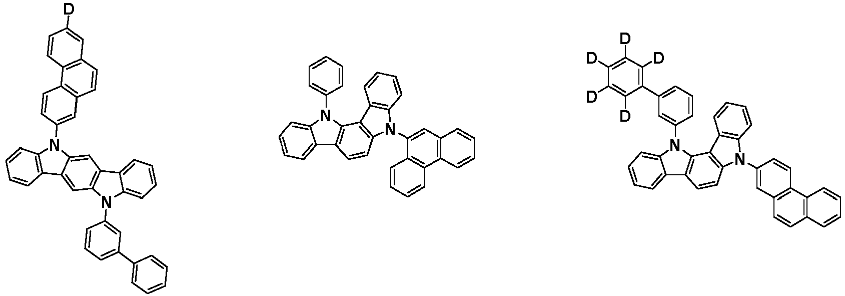

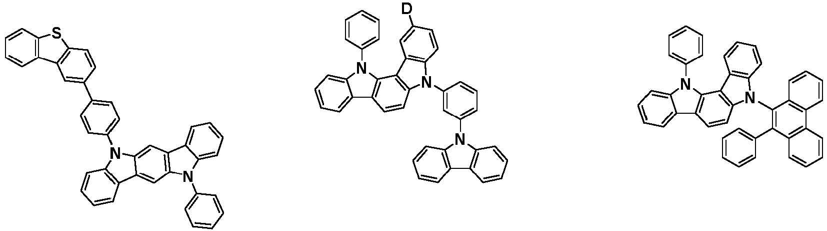

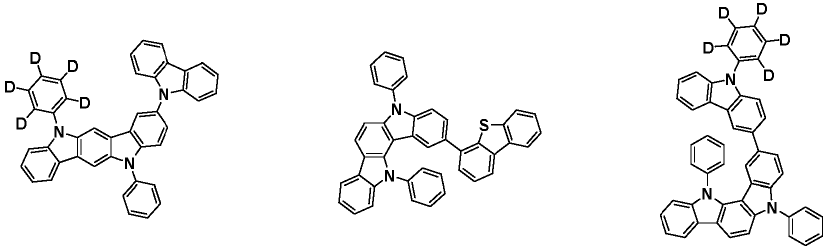

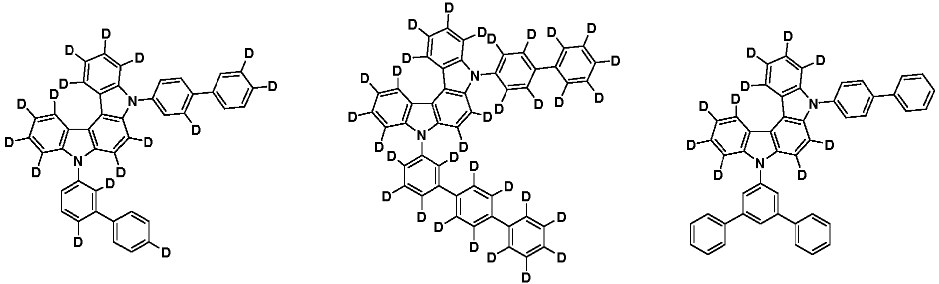

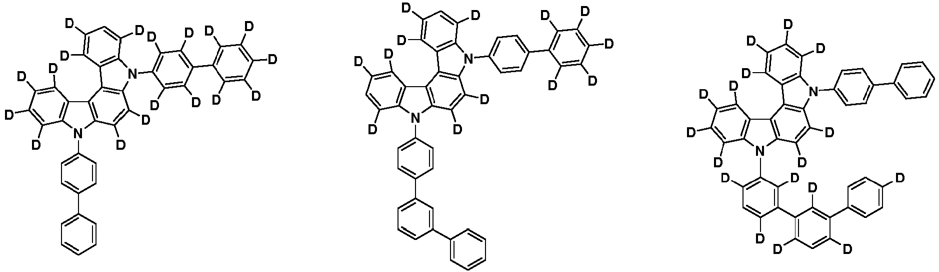

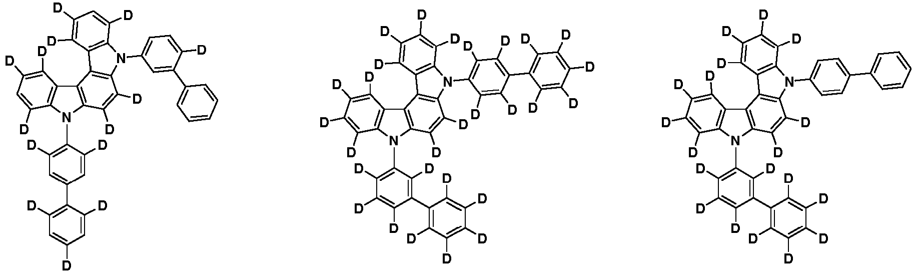

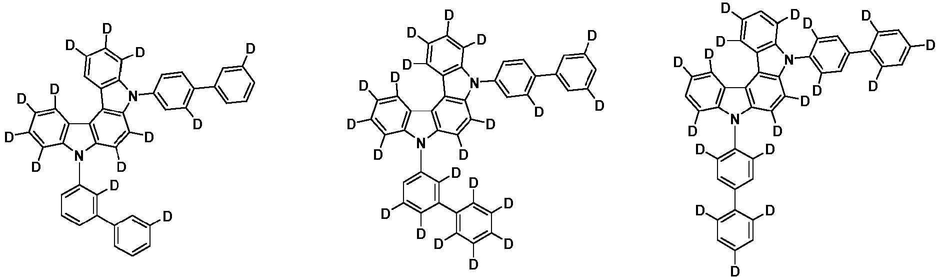

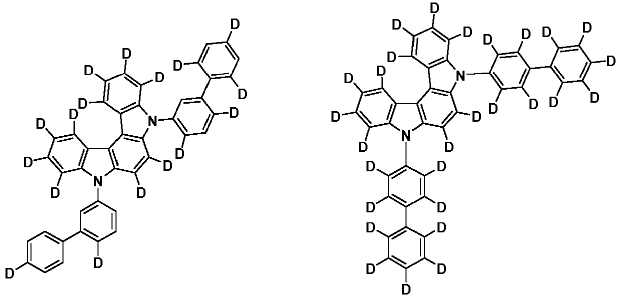

## Patentansprüche

1. Organische lichtemittierende Vorrichtung, umfassend:
eine Anode (2);
eine Kathode (4), die gegenüber der Anode bereitgestellt ist; und
eine lichtemittierende Schicht (3), die zwischen der Anode und der Kathode bereitgestellt ist,
wobei die lichtemittierende Schicht eine erste Verbindung, dargestellt durch die folgende chemische Formel 1, und eine zweite Verbindung, dargestellt durch die folgende chemische Formel 2, umfasst: 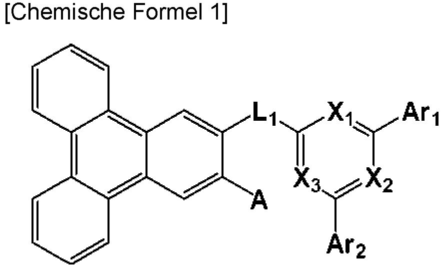 in der chemischen Formel 1,
ist A substituiertes oder unsubstituiertes Carbazolyl,
ist L₁ eine Einfachbindung oder ein substituiertes oder unsubstituiertes C₆₋₆₀-Arylen,
sind X₁, X₂ und X₃ jeweils unabhängig N oder CH, mit der Maßgabe, dass mindestens eines von X₁, X₂ und X₃ N ist, und
sind Ar₁ und Ar₂ jeweils unabhängig substituiertes oder unsubstituiertes C₆₋₆₀-Aryl oder substituiertes oder unsubstituiertes C₂₋₆₀-Heteroaryl, das mindestens ein Heteroatom aufweist, das aus der Gruppe ausgewählt ist, die aus N, O und S besteht, 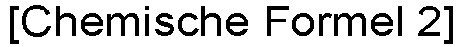 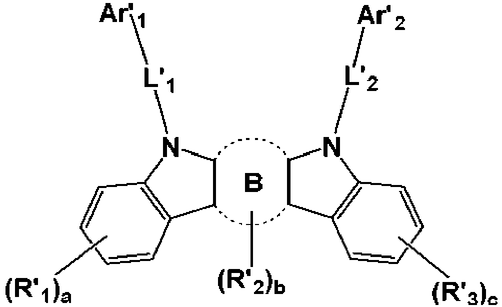 in der chemischen Formel 2,
ist B ein Benzolring, der mit zwei benachbarten fünfeckigen Ringen verschmolzen ist,
sind L'₁ und L'₂ jeweils unabhängig eine Einfachbindung oder substituiertes oder unsubstituiertes C₆₋₆₀Arylen,
sind Ar'₁ und Ar'₂ jeweils unabhängig substituiertes oder unsubstituiertes C₆₋₆₀-Aryl oder substituiertes oder unsubstituiertes C₂₋₆₀-Heteroaryl, das mindestens ein Heteroatom aufweist, das aus der Gruppe ausgewählt ist, die aus N, O und S besteht,
sind R'₁, R'₂ und R'₃ jeweils unabhängig Wasserstoff, Deuterium, substituiertes oder unsubstituiertes C₆₋₆₀-Aryl oder substituiertes oder unsubstituiertesC₂₋₆₀-Heteroaryl, das mindestens ein Heteroatom aufweist, das aus der Gruppe ausgewählt ist, die aus N, O und S besteht,
ist a eine ganze Zahl von 0 bis 4,
ist b eine ganze Zahl von 0 bis 2, und
ist c eine ganze Zahl von 0 bis 4.

2. Organische lichtemittierende Vorrichtung nach Anspruch 1,
wobei die erste Verbindung durch die folgende chemische Formel 1-1 oder chemische Formel 1-2 dargestellt wird: 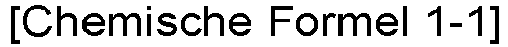 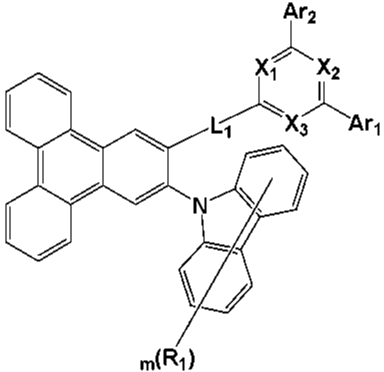 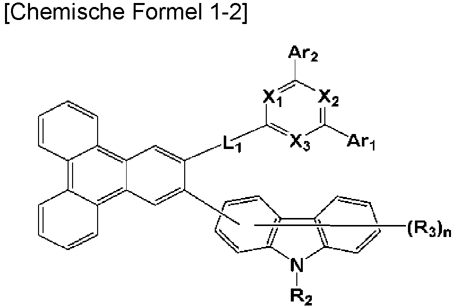 in der chemischen Formel 1-1 oder der chemischen Formel 1-2,
sind L₁, X₁, X₂, X₃, Ar₁, und Ar₂ wie in Anspruch 1 definiert,
ist R₁ jeweils unabhängig Wasserstoff, Deuterium, substituiertes oder unsubstituiertes C₁₋₆₀-Alkyl, substituiertes oder unsubstituiertes C₆₋₆₀-Aryl oder substituiertes oder unsubstituiertes C₂₋₆₀-Heteroaryl, das mindestens ein Heteroatom aufweist, das aus der Gruppe ausgewählt ist, die aus N, O und S besteht,
ist R₂ substituiertes oder unsubstituiertes C₁₋₆₀-Alkyl oder ein substituiertes oder unsubstituiertes C₆₋₆₀-Aryl,
ist R₃ jeweils unabhängig Wasserstoff, Deuterium, substituiertes oder unsubstituiertes C₁₋₆₀-Alkyl, substituiertes oder unsubstituiertes C₆₋₆₀-Aryl oder substituiertes oder unsubstituiertes C₅₋₆₀-Heteroaryl, das mindestens ein Heteroatom aufweist, das aus der Gruppe ausgewählt ist, die aus N, O und S besteht,
ist m eine ganze Zahl von 0 bis 8, und
ist n eine ganze Zahl von 0 bis 7.

3. Organische lichtemittierende Vorrichtung nach Anspruch 1,
wobei L₁ eine Einfachbindung, Phenylen oder Biphenylylen ist, und
das Phenylen und das Biphenylylen jeweils unabhängig unsubstituiert oder mit mindestens einem Deuterium substituiert sind.

4. Organische lichtemittierende Vorrichtung nach Anspruch 1,
wobei Ar₁ und Ar₂ jeweils unabhängig Phenyl, Biphenylyl, Terphenylyl, Dimethylfluorenyl, Naphthyl, Phenanthrenyl, Triphenylenyl, Dibenzofuranyl, Dibenzothiophenyl, Carbazol-9-yl, 9-Phenyl-9H-carbazolyl, Benzoxazolyl, Benzothiazolyl, 2-Phenylbenzoxazolyl oder 2-Phenylbenzothiazolyl sind, und
sie jeweils unabhängig unsubstituiert oder mit mindestens einem Deuterium substituiert sind.

5. Organische lichtemittierende Vorrichtung nach Anspruch 2,
wobei R₁ jeweils unabhängig Wasserstoff, Deuterium, Phenyl, Dibenzofuranyl, Dibenzothiophenyl oder Carbazolyl sind, und
das Phenyl, Dibenzofuranyl, Dibenzothiophenyl und Carbazolyl jeweils unabhängig unsubstituiert oder mit mindestens einem Deuterium substituiert sind.

6. Organische lichtemittierende Vorrichtung nach Anspruch 2,
wobei R₂ jeweils unabhängig unsubstituiertes oder mit mindestens einem Deuterium substituiertes Phenyl sind.

7. Organische lichtemittierende Vorrichtung nach Anspruch 2,
wobei R₃ jeweils unabhängig Wasserstoff, Deuterium, Phenyl, Dibenzofuranyl, Dibenzothiophenyl oder Carbazolyl sind, und
das Phenyl, Dibenzofuranyl, Dibenzothiophenyl und Carbazolyl jeweils unabhängig unsubstituiert oder mit mindestens einem Deuterium substituiert sind.

8. Organische lichtemittierende Vorrichtung nach Anspruch 1,
wobei die erste Verbindung eine beliebige Verbindung ist, ausgewählt aus der Gruppe, bestehend aus den folgenden Verbindungen:

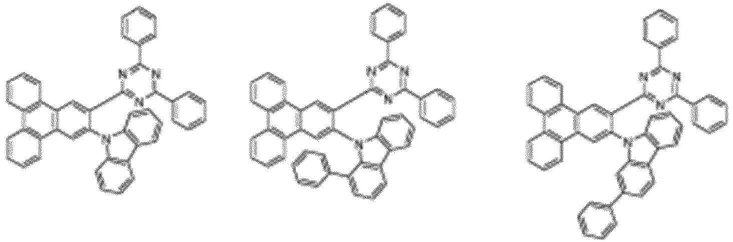

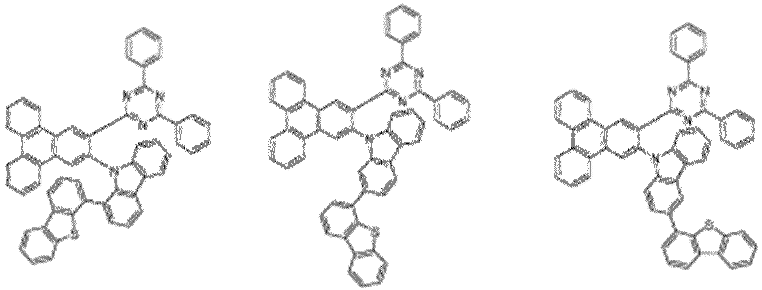

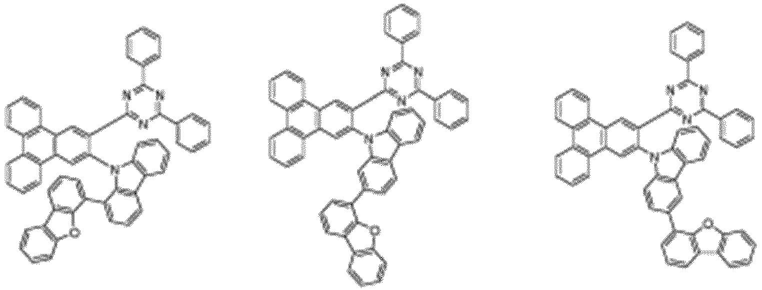

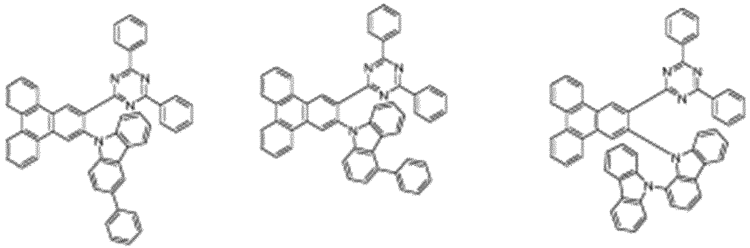

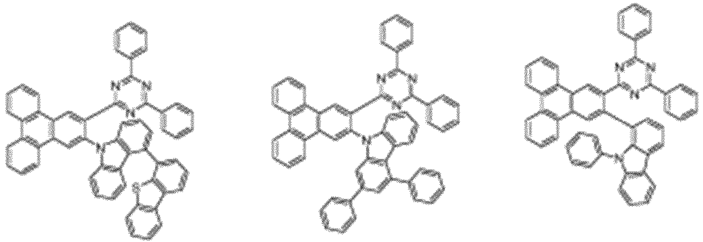

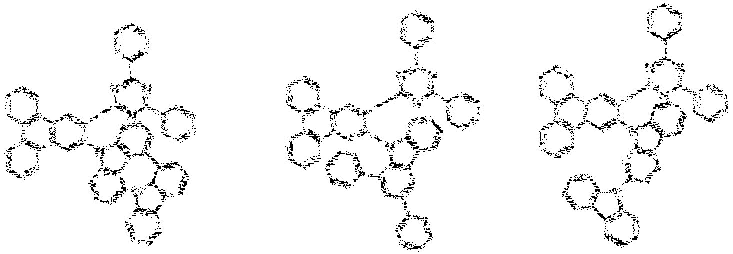

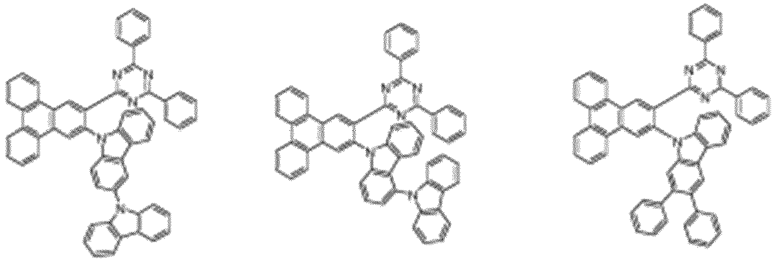

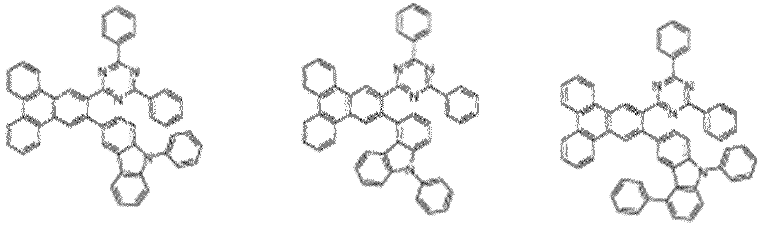

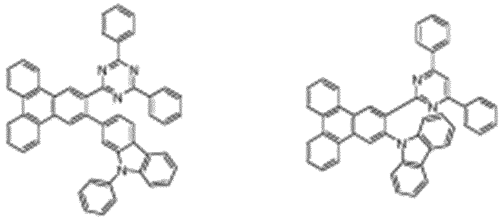

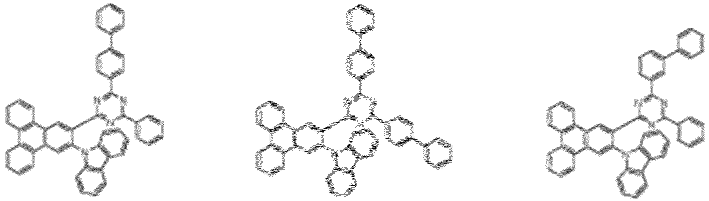

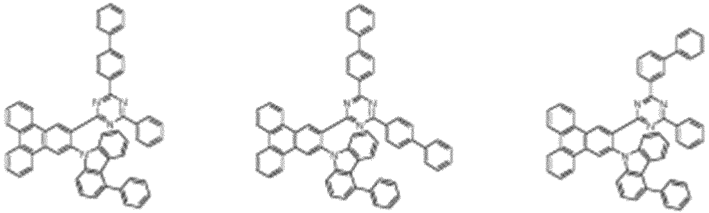

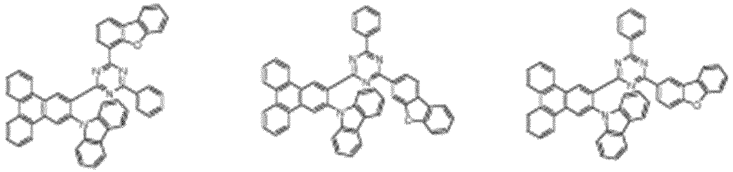

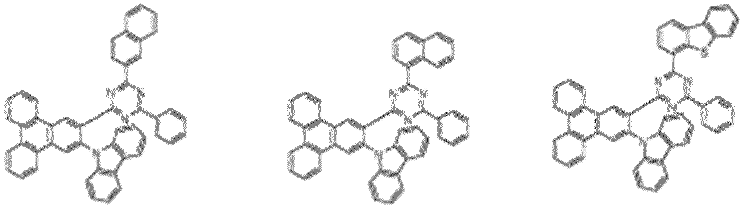

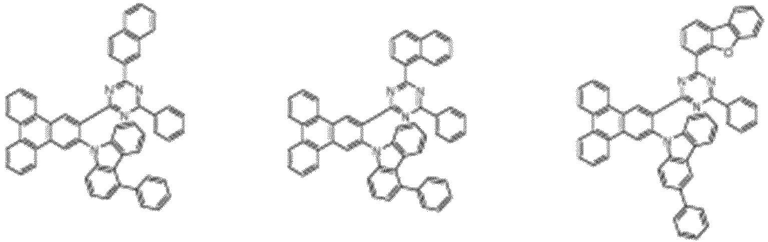

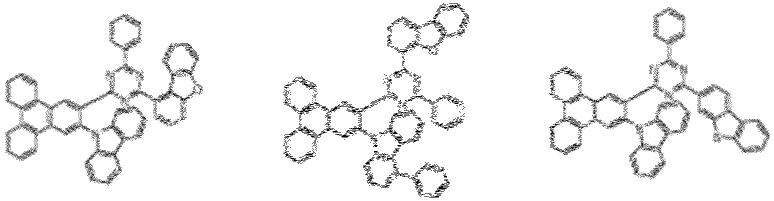

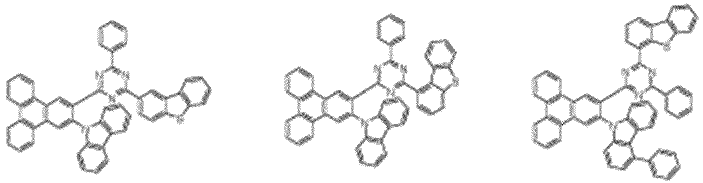

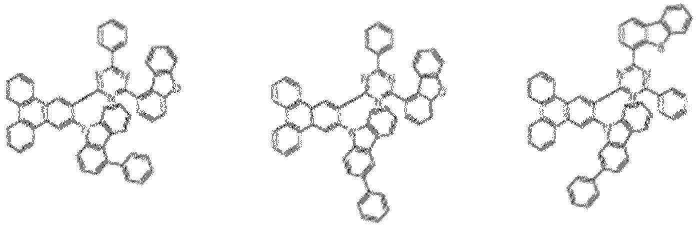

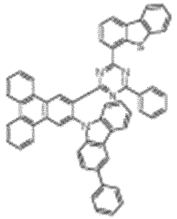

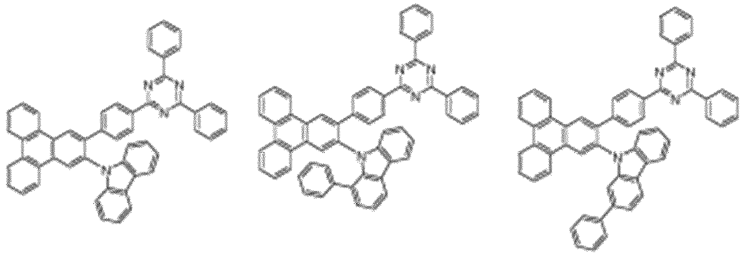

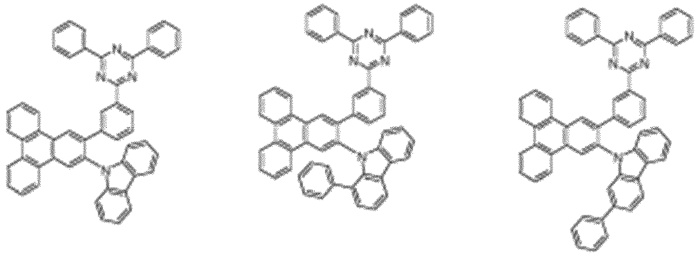

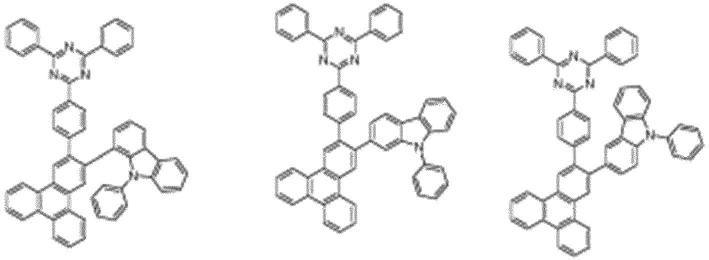

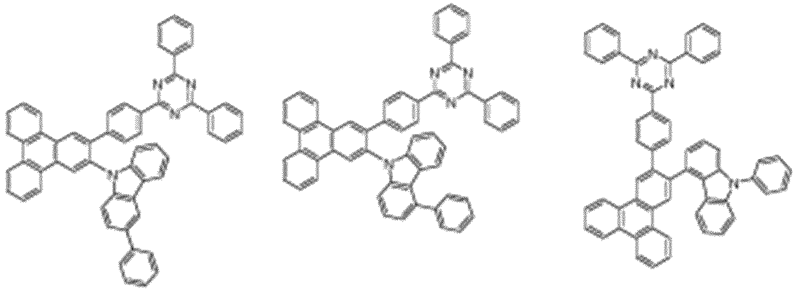

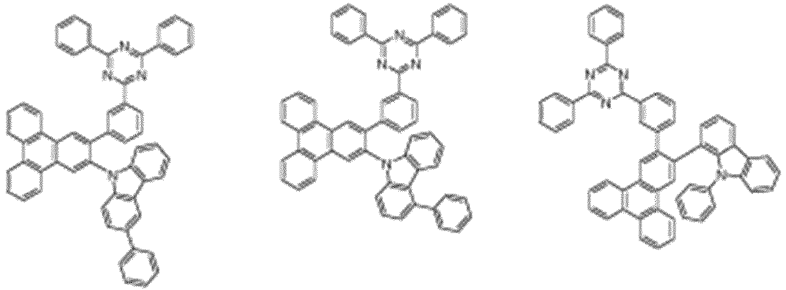

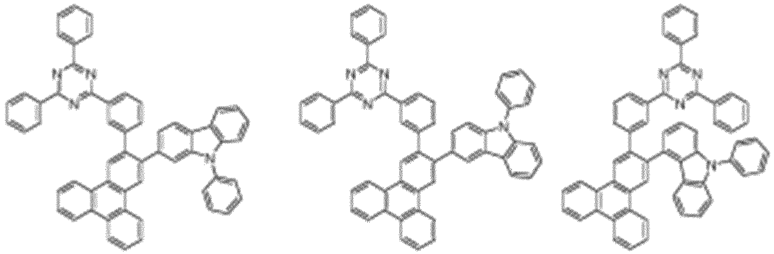

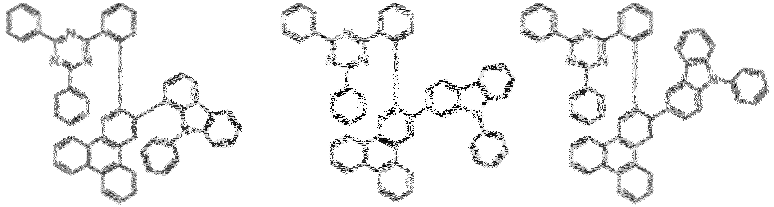

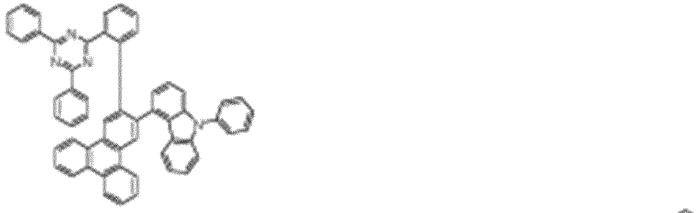

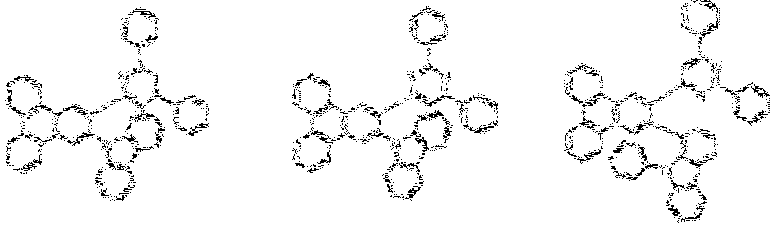

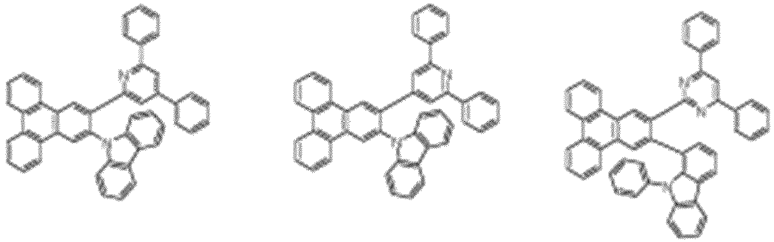

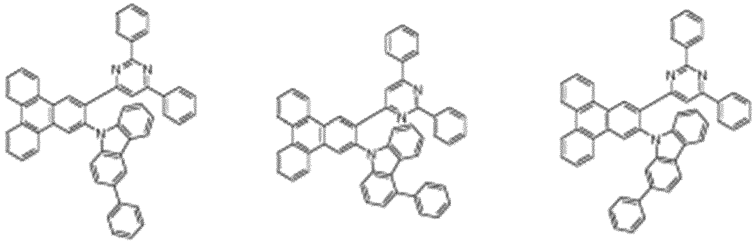

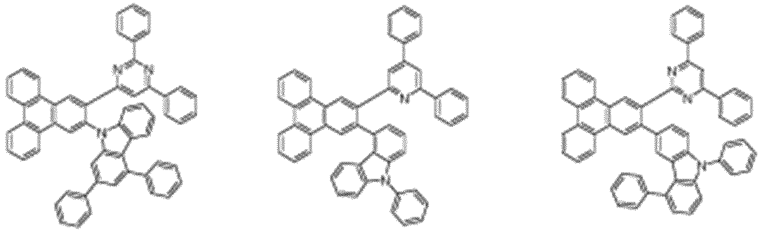

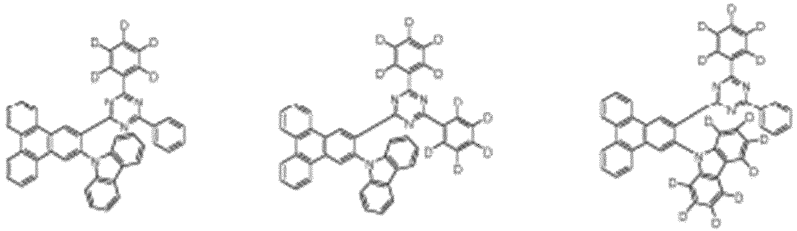

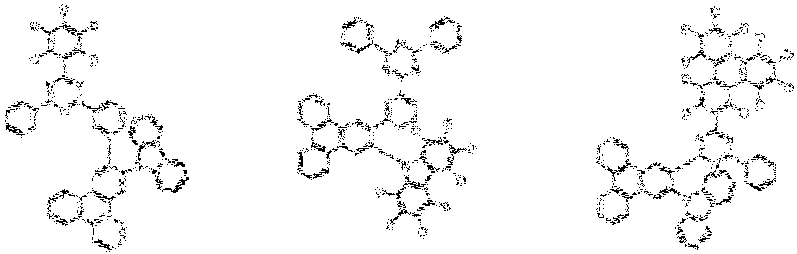

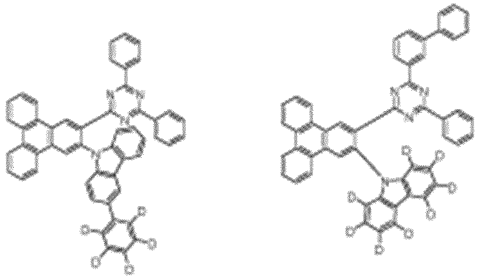

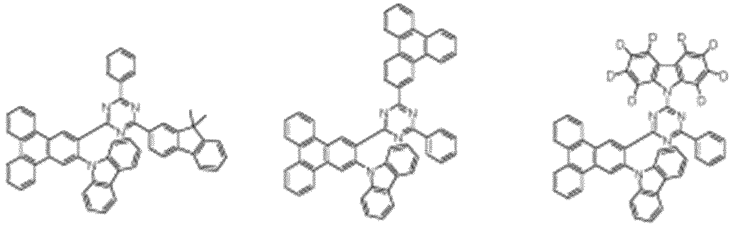

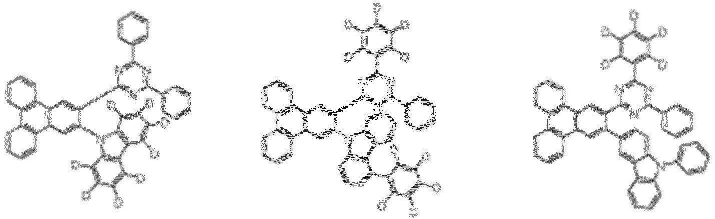

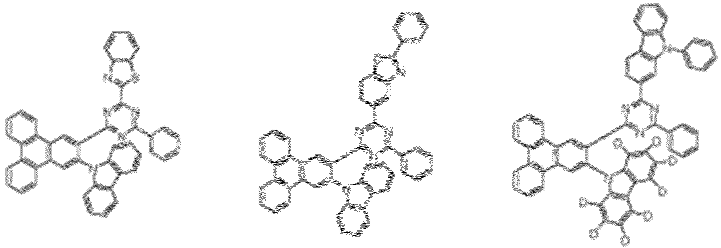

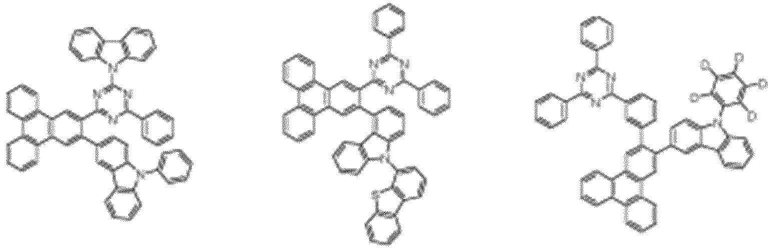

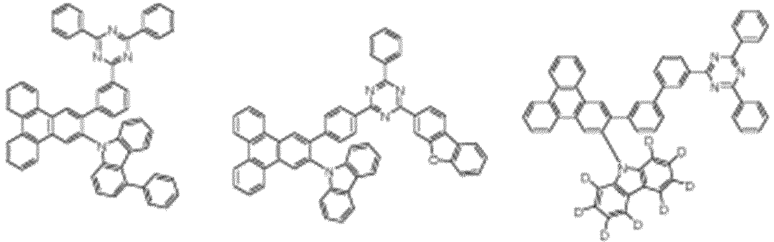

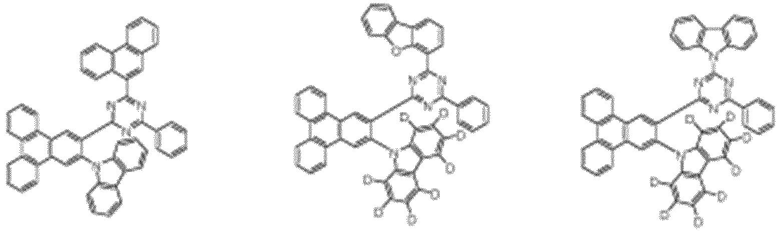

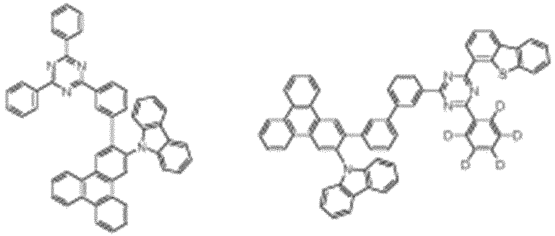

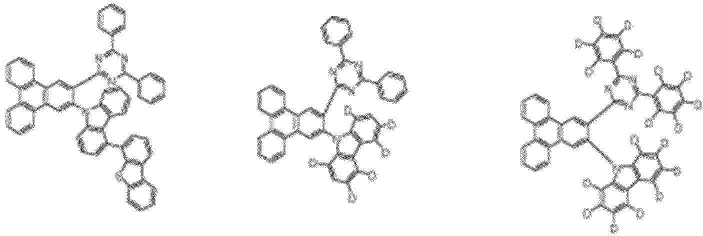

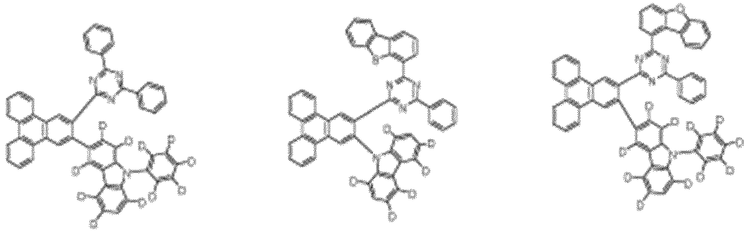

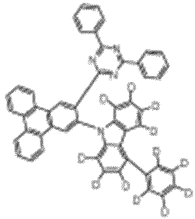

9. Organische lichtemittierende Vorrichtung nach Anspruch 1,
wobei die zweite Verbindung durch eine beliebige der folgenden chemischen Formeln 2-1 bis 2-5 dargestellt wird: 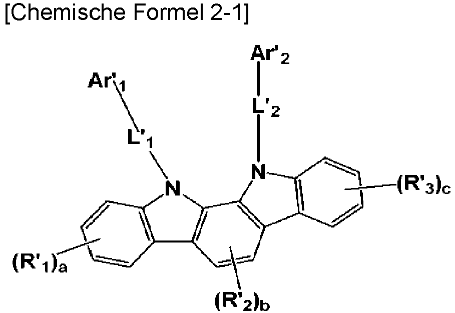 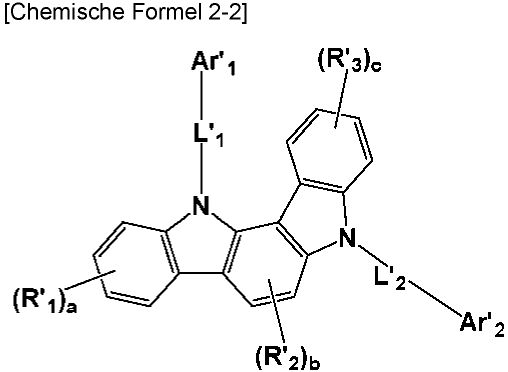 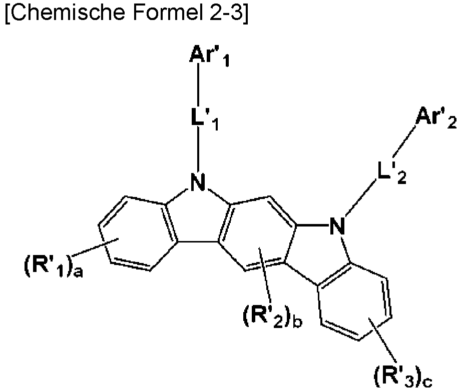 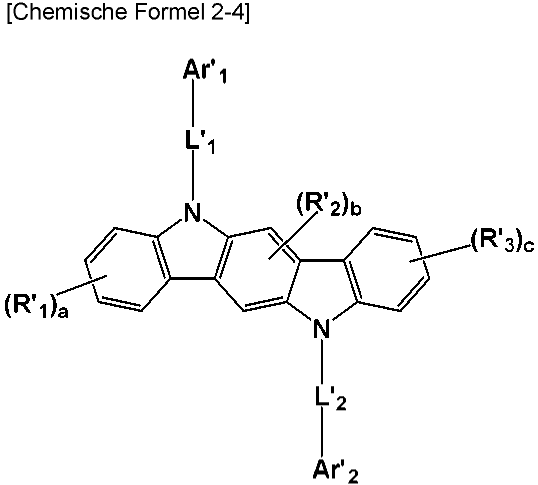 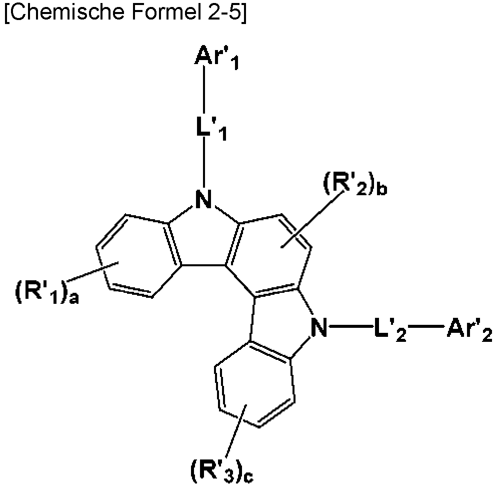 in den chemischen Formeln 2-1 bis 2-5,
sind L'₁, L'₂, Ar'₁, Ar'₂, R'₁, R'₂, R'₃, a, b und c wie in Anspruch 1 definiert.

10. Organische lichtemittierende Vorrichtung nach Anspruch 1,
wobei L'₁ und L'₂ jeweils unabhängig eine Einfachbindung, Phenylen oder Biphenylylen sind, und
das Phenylen und das Biphenylylen jeweils unabhängig unsubstituiert oder mit mindestens einem Deuterium substituiert sind.

11. Organische lichtemittierende Vorrichtung nach Anspruch 1,
wobei Ar'₁ und Ar'₂ jeweils unabhängig Phenyl, Biphenylyl, Terphenylyl, Naphthyl, Phenanthrenyl, Triphenylenyl, Fluorenyl, Dibenzofuranyl, Dibenzothiophenyl, Carbazol-9-yl oder 9-Phenyl-9H-carbazolyl sind, und
Ar'₁ und Ar'₂ jeweils unabhängig unsubstituiert oder mit mindestens einem Substituenten, ausgewählt aus der Gruppe bestehend aus Deuterium, C₁₋₁₀-Alkyl und C₆₋₂₀-Aryl, substituiert sind.

12. Organische lichtemittierende Vorrichtung nach Anspruch 1,
wobei R'₁, R'₂ und R'₃ jeweils unabhängig Wasserstoff, Deuterium, Phenyl, Dibenzofuranyl, Dibenzothiophenyl, Carbazol-9-yl oder 9-Phenyl-9H-carbazolyl sind, und
das Phenyl, Dibenzofuranyl, Dibenzothiophenyl, Carbazol-9-yl und 9-Phenyl-9H-carbazolyl jeweils unabhängig unsubstituiert oder mit mindestens einem Deuterium substituiert sind.

13. Organische lichtemittierende Vorrichtung nach Anspruch 1,
wobei die zweite Verbindung eine beliebige Verbindung ist, ausgewählt aus der Gruppe bestehend aus den folgenden Verbindungen:

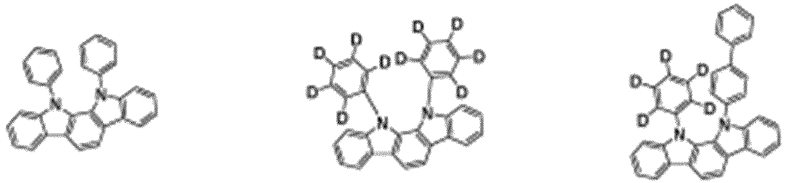

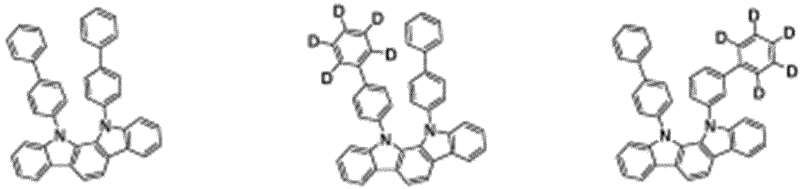

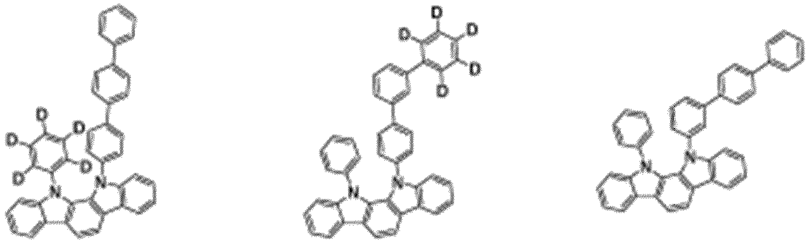

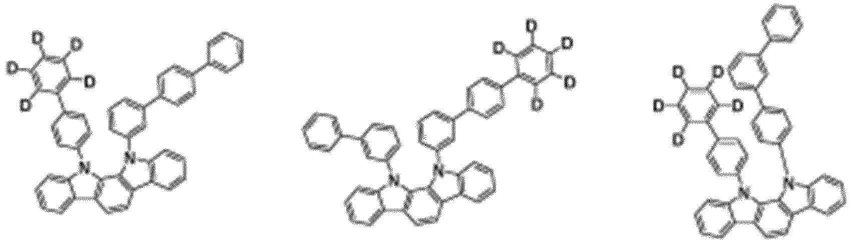

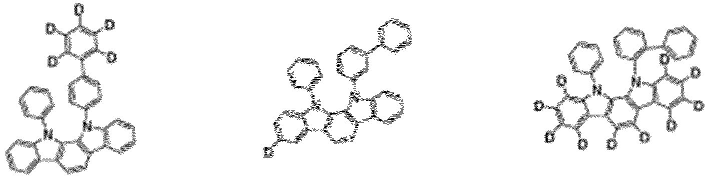

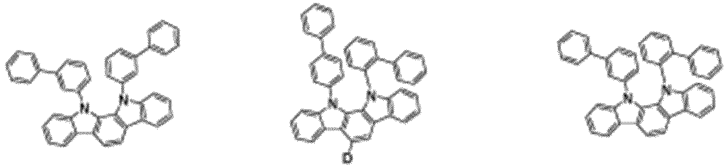

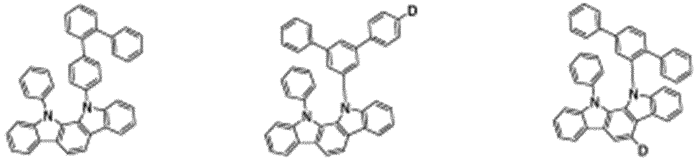

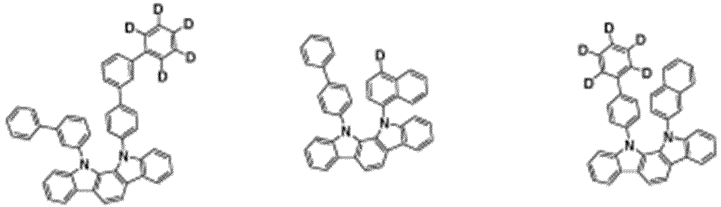

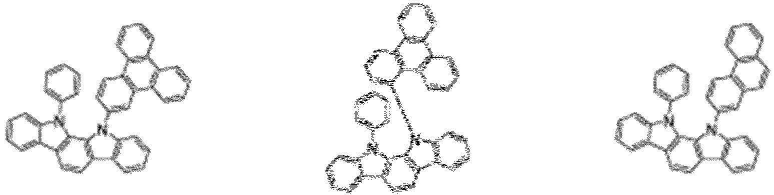

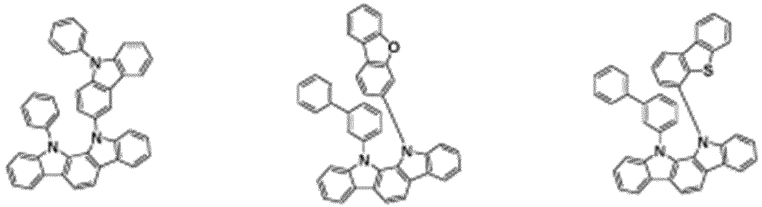

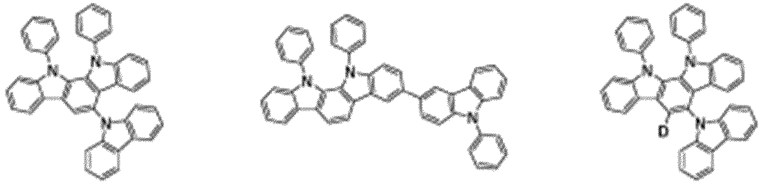

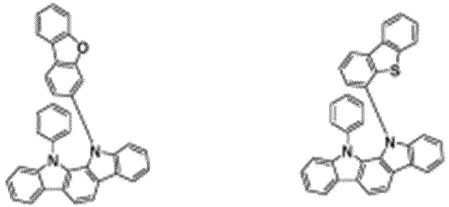

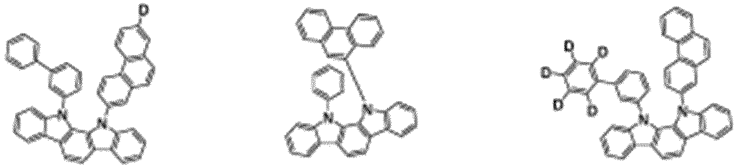

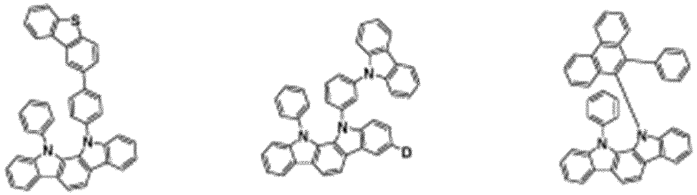

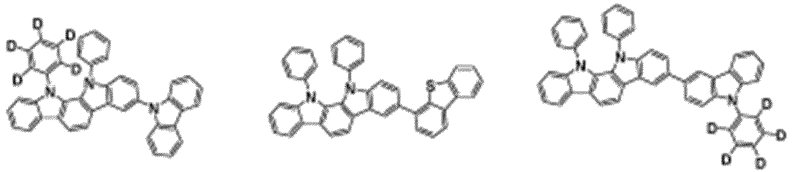

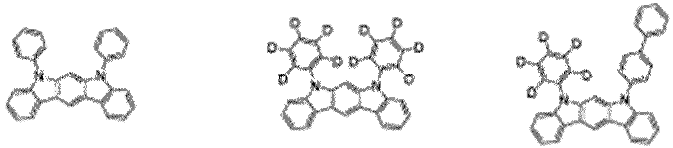

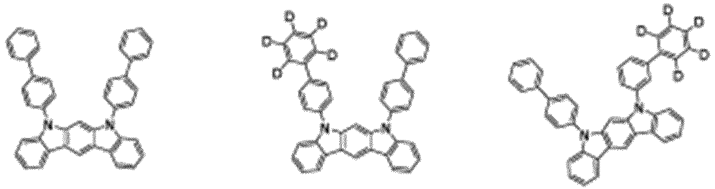

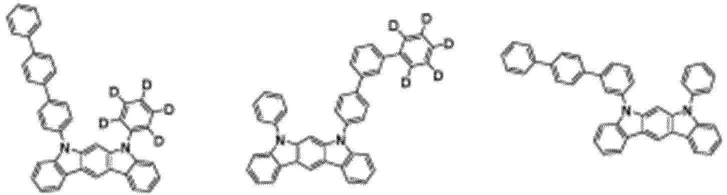

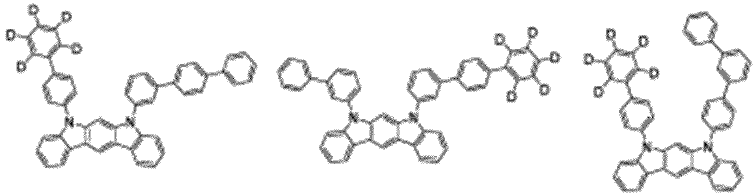

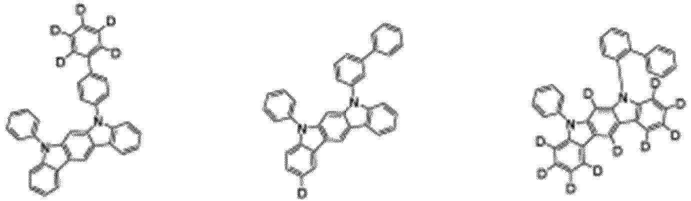

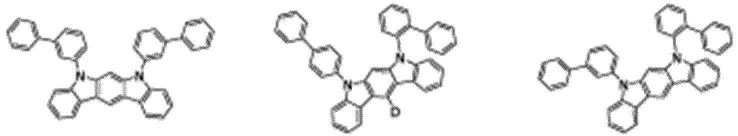

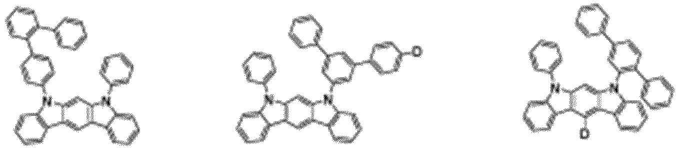

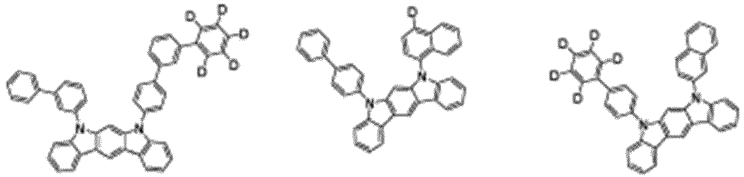

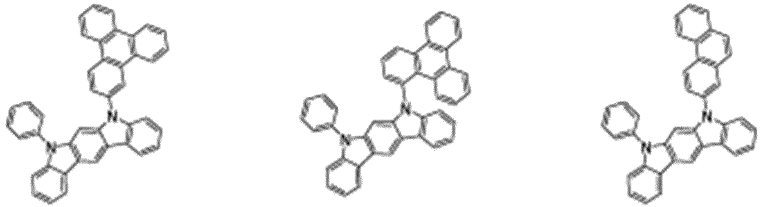

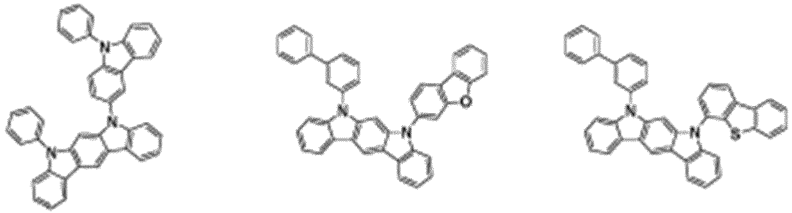

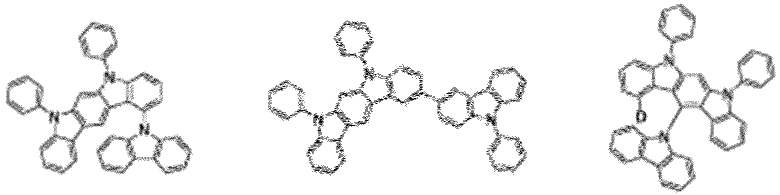

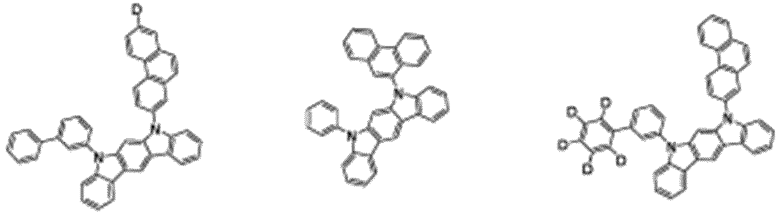

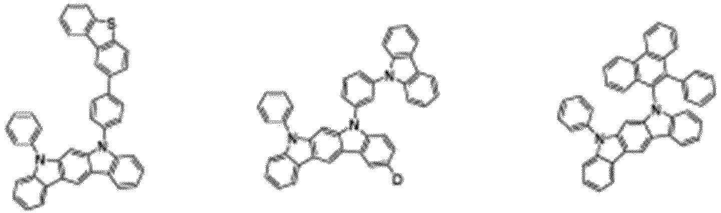

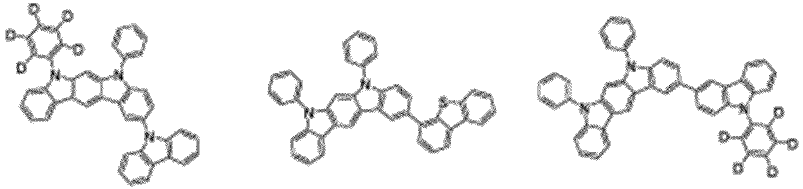

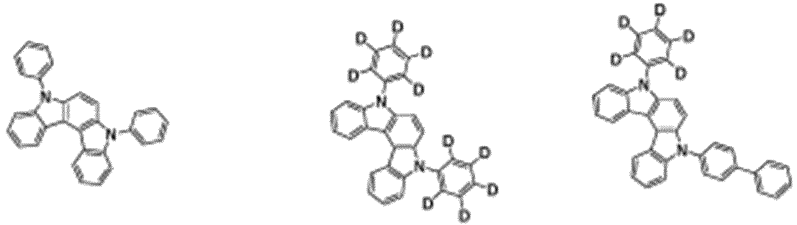

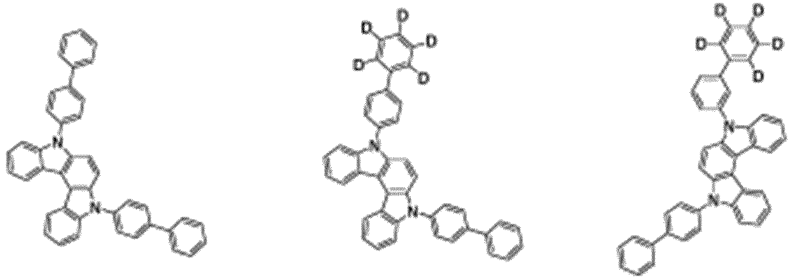

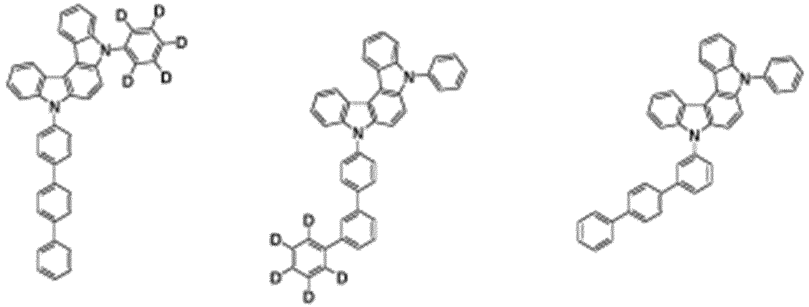

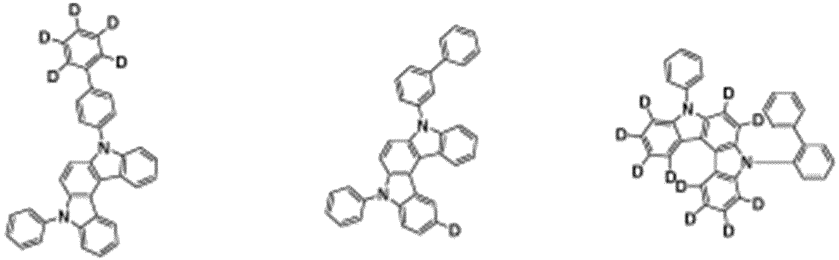

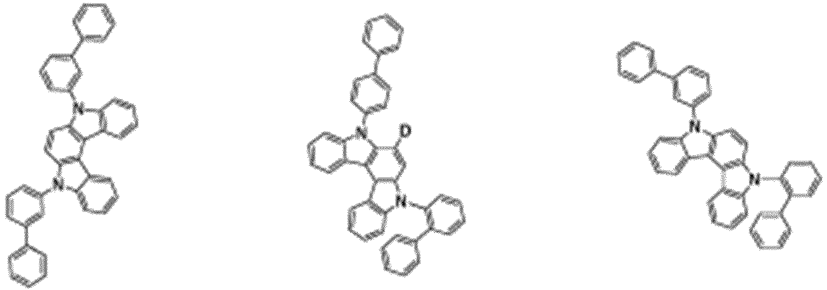

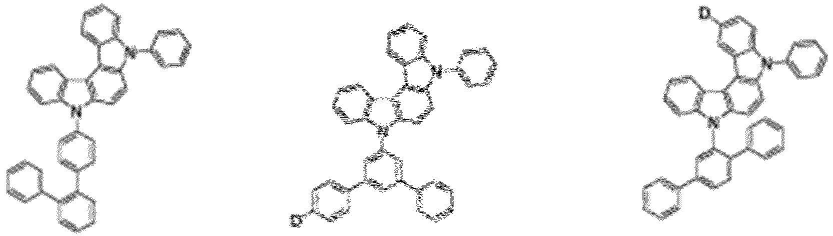

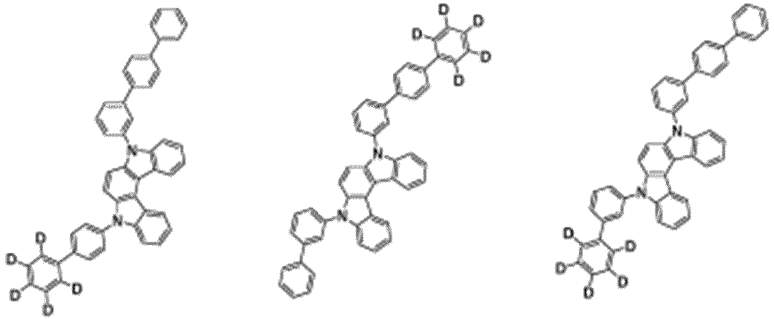

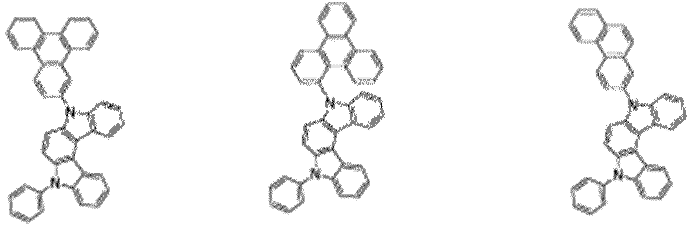

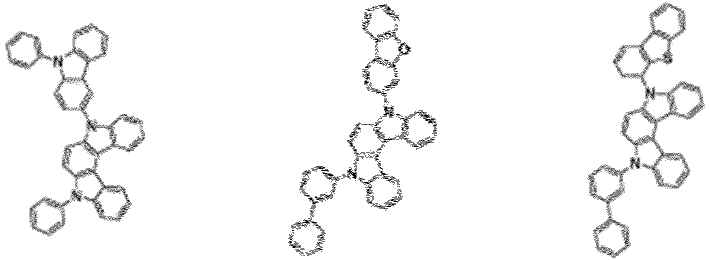

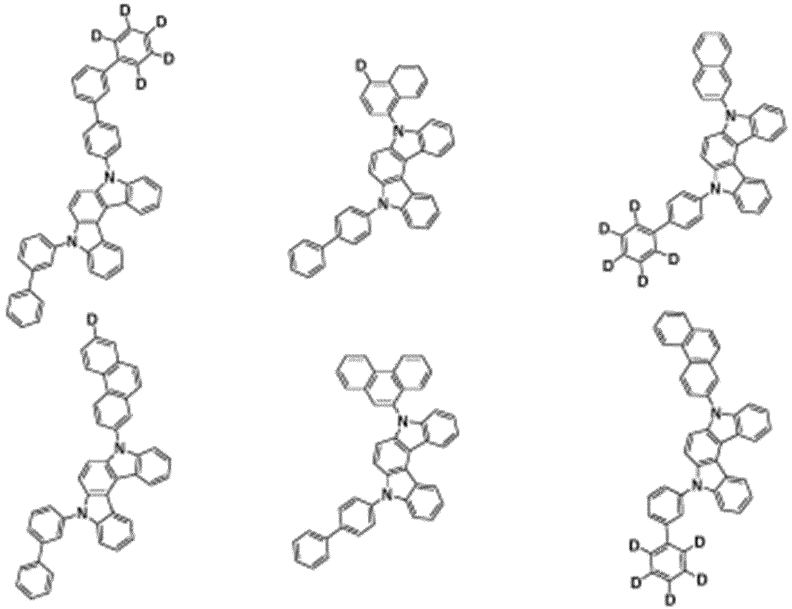

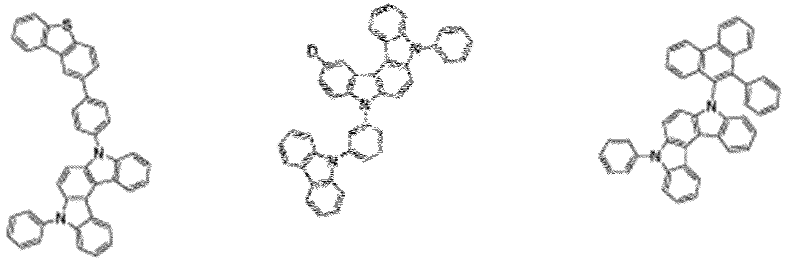

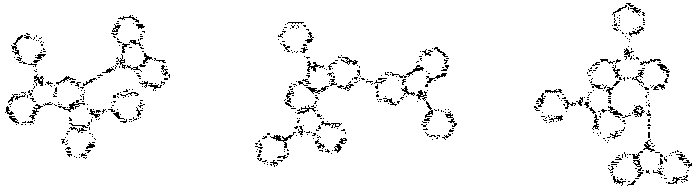

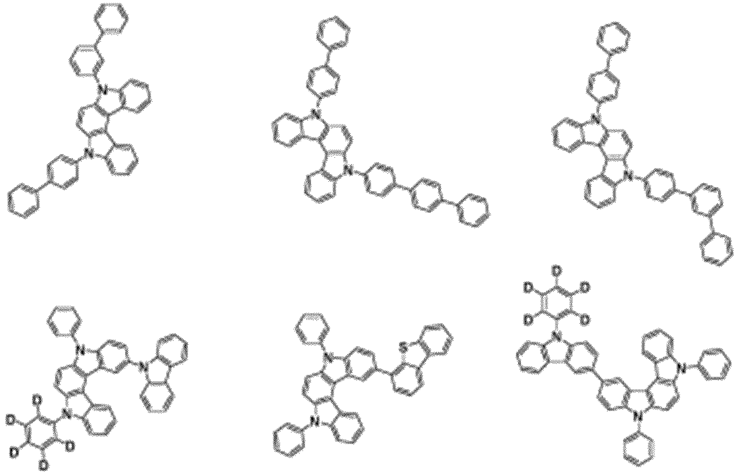

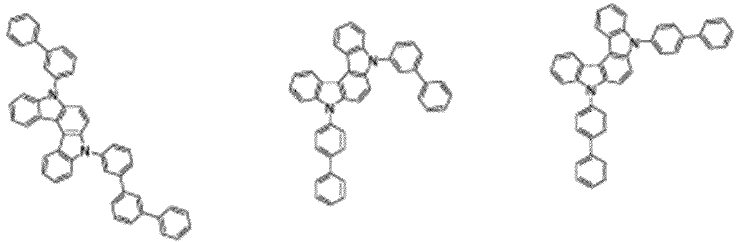

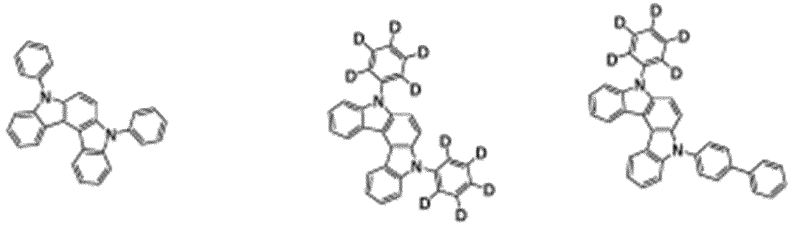

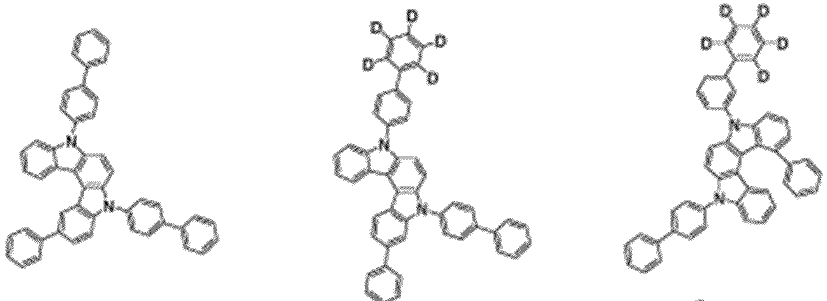

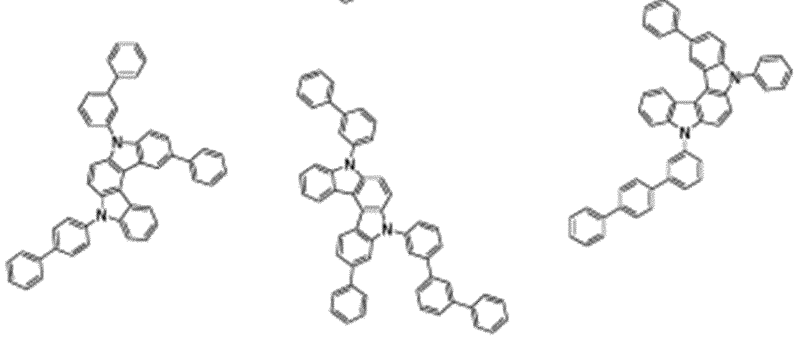

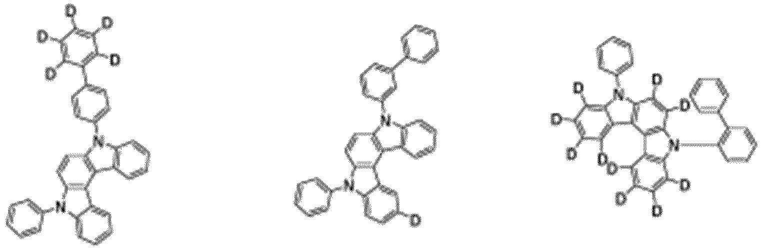

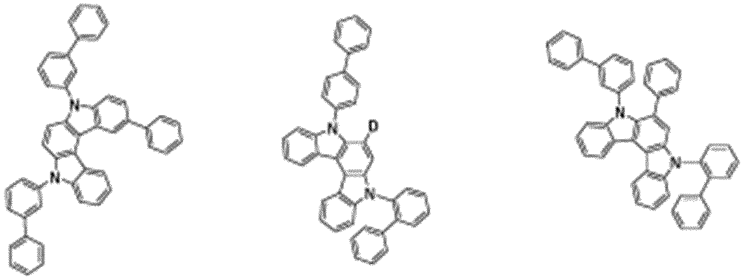

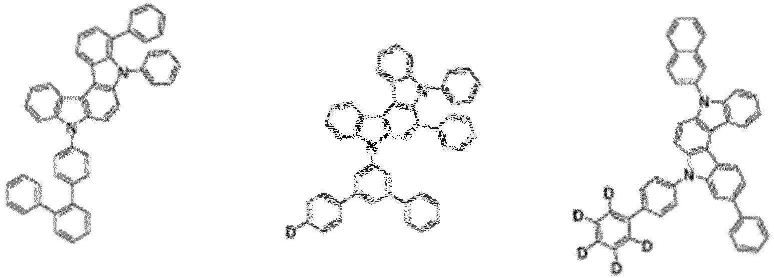

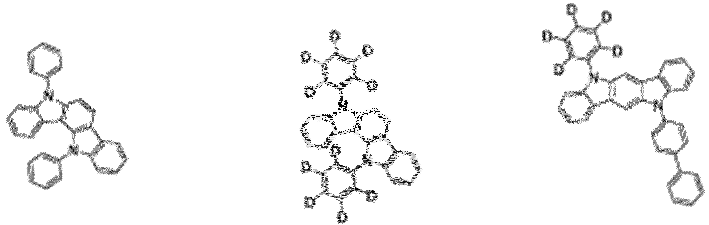

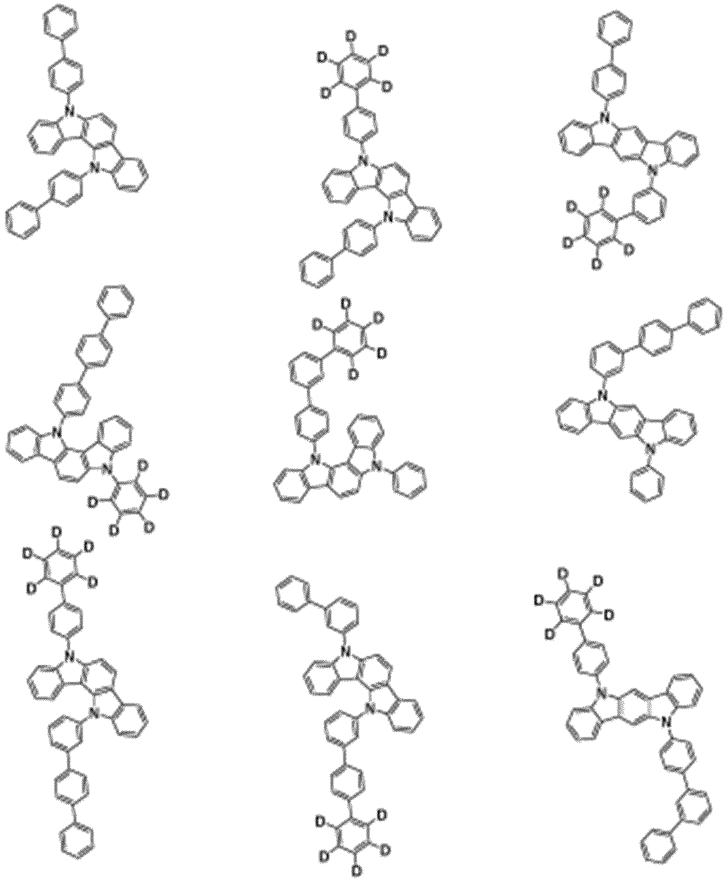

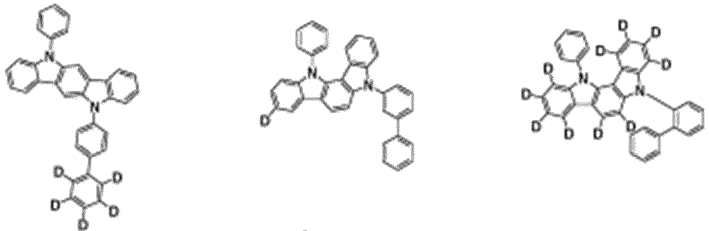

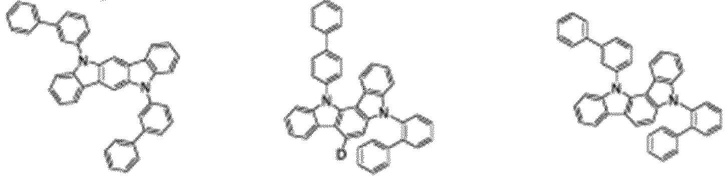

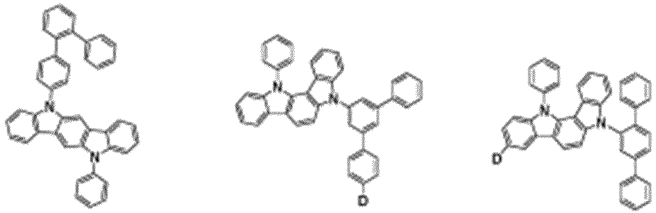

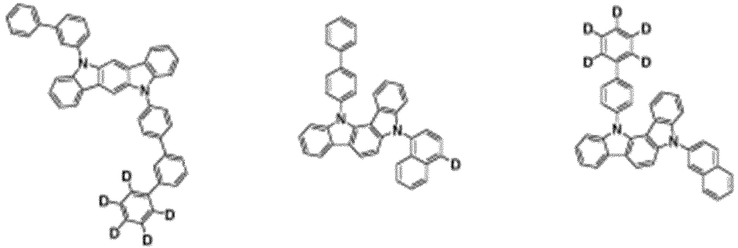

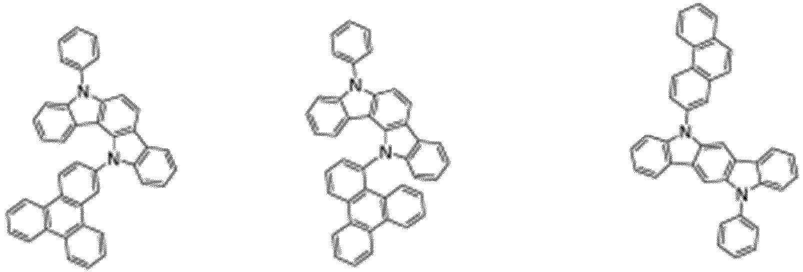

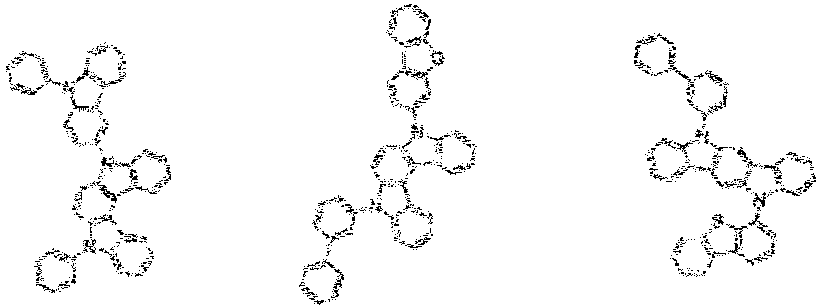

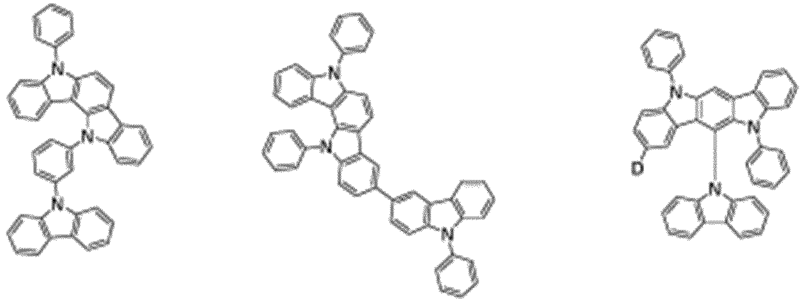

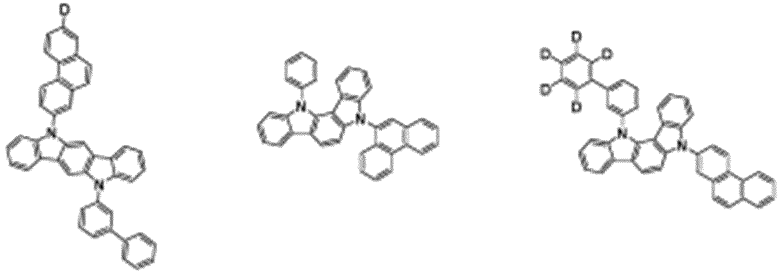

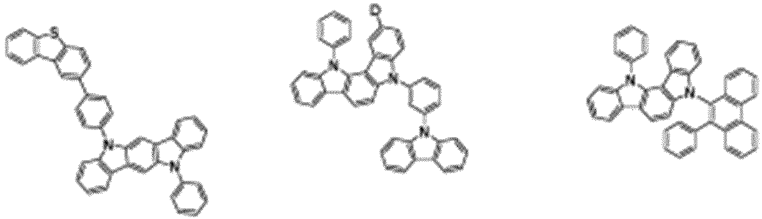

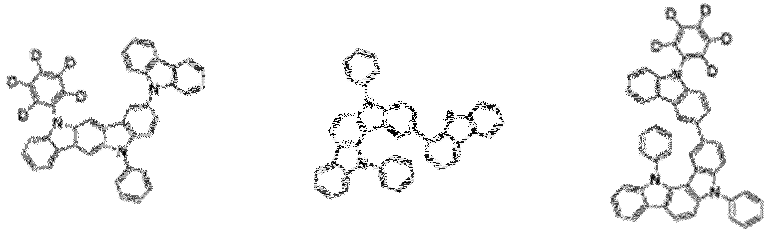

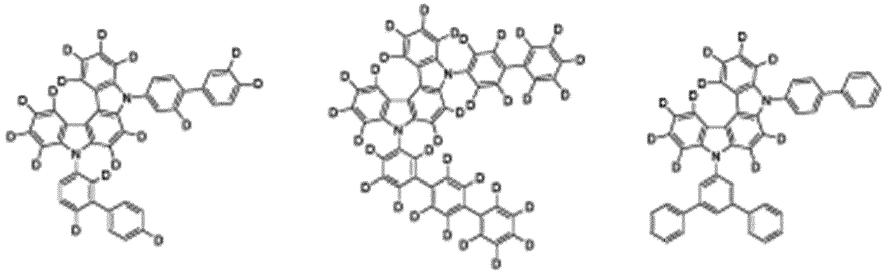

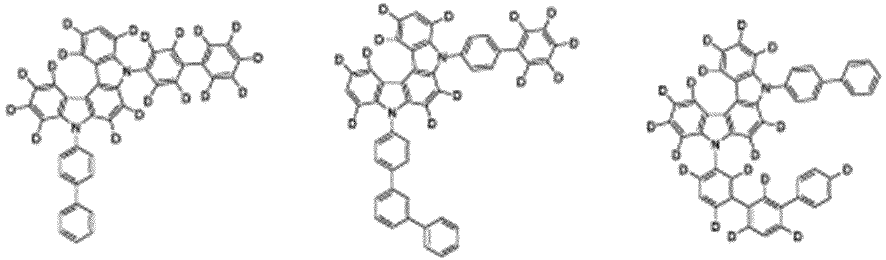

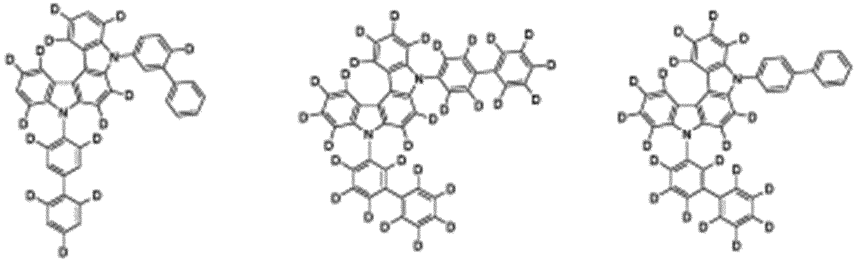

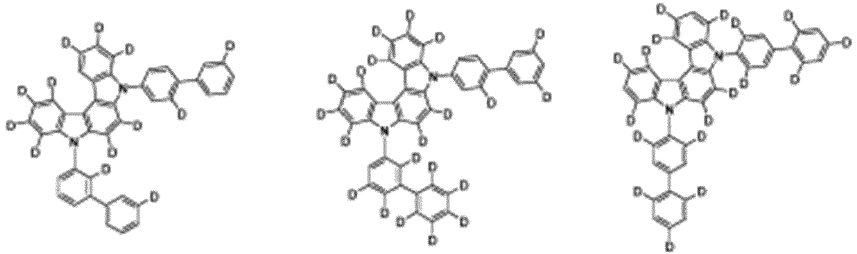

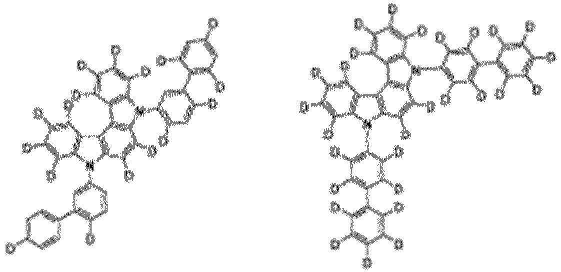

## Revendications

1. Dispositif électroluminescent organique comprenant :
une anode (2) ;
une cathode (4) qui est placée en regard de l'anode ; et
une couche électroluminescente (3) qui est placée entre l'anode et la cathode,
dans lequel la couche électroluminescente comprend un premier composé représenté par la formule chimique 1 suivante et un second composé représenté par la formule chimique 2 suivante : 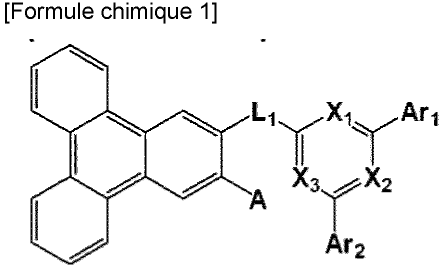 dans la formule chimique 1,
A est un carbazolyle substitué ou non substitué,
L₁ est une liaison simple, ou un arylène en C₆₋₆₀ substitué ou non substitué,
X₁, X₂ et X₃ sont chacun indépendamment N, ou CH, à condition qu'au moins un parmi X₁, X₂ et X₃ soit N, et
Ar₁ et Ar₂ sont chacun indépendamment un aryle en C₆₋₆₀ substitué ou non substitué, ou un hétéroaryle en C₂₋₆₀ substitué ou non substitué contenant au moins un hétéroatome sélectionné dans le groupe consistant en N, O et S, 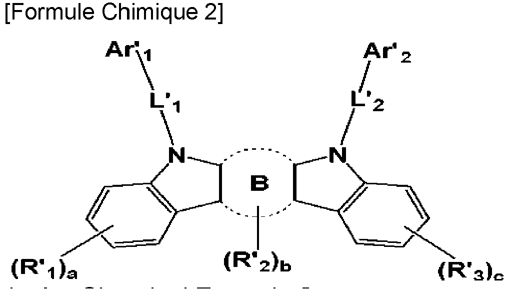 dans la formule chimique 2,
B est un cycle benzène condensé avec deux cycles pentagonaux adjacents,
L'₁ et L'₂ sont chacun indépendamment une liaison simple, ou un arylène en C₆₋₆₀ substitué ou non substitué,
Ar'₁ et Ar'₂ sont chacun indépendamment un aryle en C₆₋₆₀ substitué ou non substitué, ou un hétéroaryle en C₂₋₆₀ substitué ou non substitué contenant au moins un hétéroatome sélectionné dans le groupe consistant en N, O et S,
R'₁, R'₂ et R'₃ sont chacun indépendamment un hydrogène, un deutérium, un aryle en C₆₋₆₀ substitué ou non substitué, ou un hétéroaryle en C₂₋₆₀ substitué ou non substitué contenant au moins un hétéroatome sélectionné dans le groupe consistant en N, O et S,
a est un nombre entier de 0 à 4,
b est un nombre entier de 0 à 2, et
c est un nombre entier de 0 à 4.

2. Dispositif électroluminescent organique selon la revendication 1,
dans lequel le premier composé est représenté par la formule chimique 1-1 ou la formule chimique 1-2 suivante : 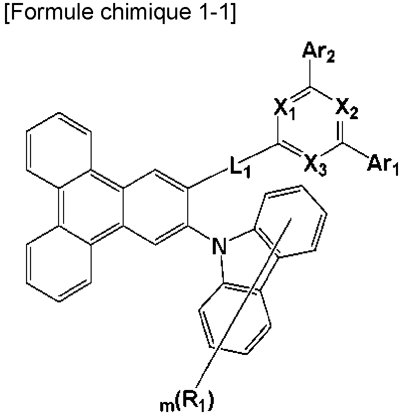 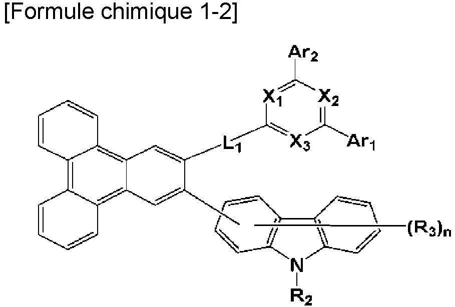 dans la formule chimique 1-1 ou la formule chimique 1-2,
L₁, X₁, X₂, X₃, Ar₁, et Ar₂ sont tels que définis dans la revendication 1,
les R₁ sont chacun indépendamment un hydrogène, un deutérium, un alkyle en C₁₋₆₀ substitué ou non substitué, un aryle en C₆₋₆₀ substitué ou non substitué, ou un hétéroaryle en C₂₋₆₀ substitué ou non substitué contenant au moins un hétéroatome sélectionné dans le groupe consistant en N, O et S,
R₂ est un alkyle en C₁₋₆₀ substitué ou non substitué, ou un aryle en C₆₋₆₀ substitué ou non substitué,
les R₃ sont chacun indépendamment un hydrogène, un deutérium, un alkyle en C₁₋₆₀ substitué ou non substitué, un aryle en C₆₋₆₀ substitué ou non substitué, ou un hétéroaryle en C₅₋₆₀ substitué ou non substitué contenant au moins un hétéroatome sélectionné dans le groupe consistant en N, O et S,
m est un nombre entier de 0 à 8, et
n est un nombre entier de 0 à 7.

3. Dispositif électroluminescent organique selon la revendication 1,
dans lequel L₁ est une liaison simple, un phénylène ou un biphénylylène, et
le phénylène et le biphénylylène sont chacun indépendamment non substitués ou substitués avec au moins un deutérium.

4. Dispositif électroluminescent organique selon la revendication 1,
dans lequel Ar₁ et Ar₂ sont chacun indépendamment un phényle, un biphénylyle, un terphénylyle, un diméthylfluorényle, un naphtyle, un phénanthrényle, un triphénylényle, un dibenzofuranyle, un dibenzothiophényle, un carbazol-9-yle, un 9-phényl-9H-carbazolyle, un benzoxazolyle, un benzothiazolyle, un 2-phénylbenzoxazolyle ou un 2-phénylbenzothiazolyle, et
ils sont chacun indépendamment non substitués ou substitués avec au moins un deutérium.

5. Dispositif électroluminescent organique selon la revendication 2,
dans lequel les R₁ sont chacun indépendamment un hydrogène, un deutérium, un phényle, un dibenzofuranyle, un dibenzothiophényle, ou un carbazolyle, et
le phényle, le dibenzofuranyle, le dibenzothiophényle, et le carbazolyle sont chacun indépendamment non substitués ou substitués avec au moins un deutérium.

6. Dispositif électroluminescent organique selon la revendication 2,
dans lequel les R₂ sont chacun indépendamment un phényle non substitué ou substitué avec au moins un deutérium.

7. Dispositif électroluminescent organique selon la revendication 2,
dans lequel les R₃ sont chacun indépendamment un hydrogène, un deutérium, un phényle, un dibenzofuranyle, un dibenzothiophényle, ou un carbazolyle, et
le phényle, le dibenzofuranyle, le dibenzothiophényle, et le carbazolyle sont chacun indépendamment non substitués ou substitués avec au moins un deutérium.

8. Dispositif électroluminescent organique selon la revendication 1,
dans lequel le premier composé est l'un quelconque sélectionné dans le groupe consistant en les composés suivants :

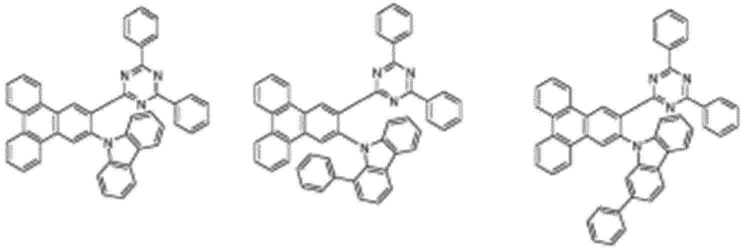

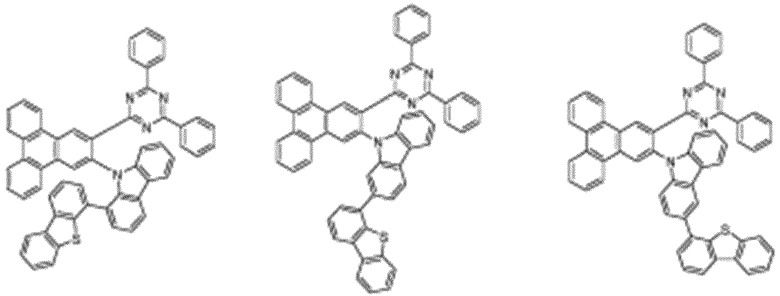

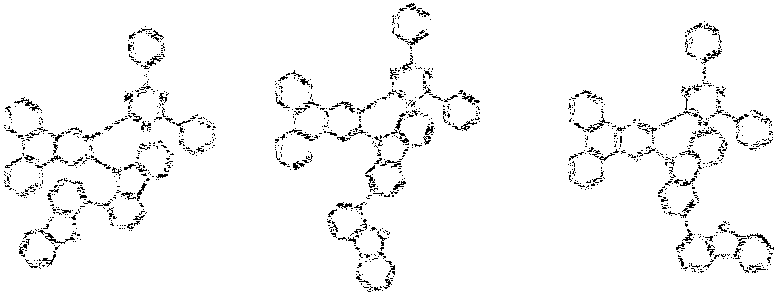

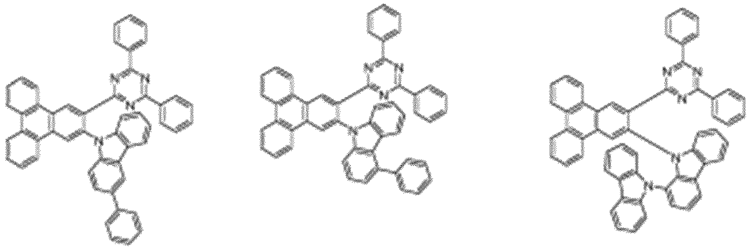

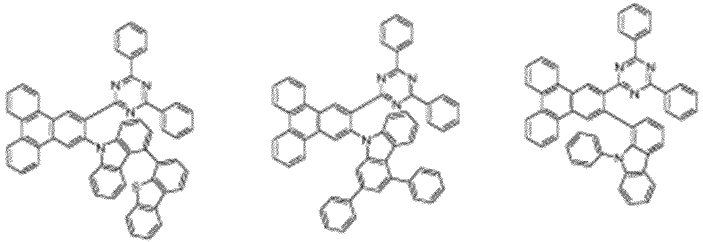

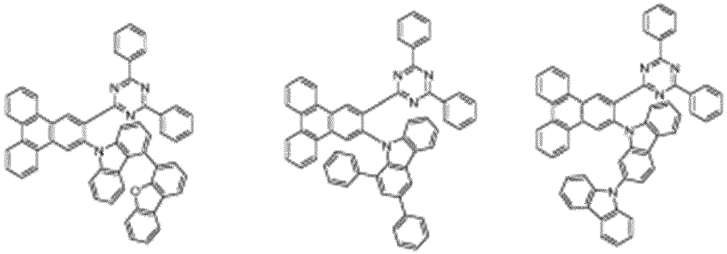

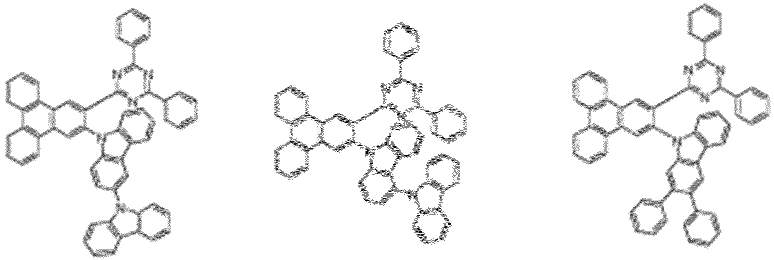

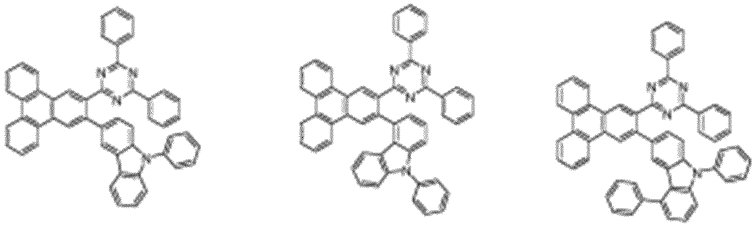

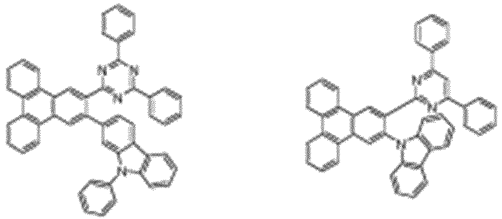

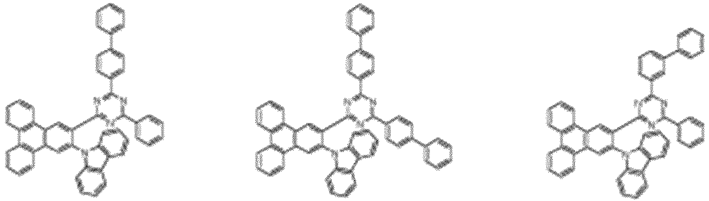

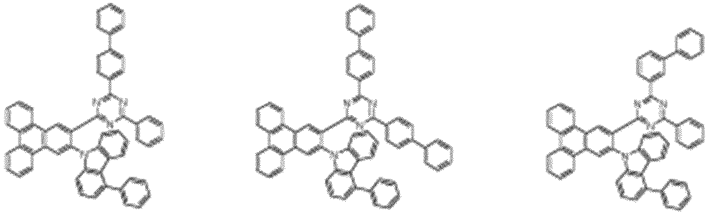

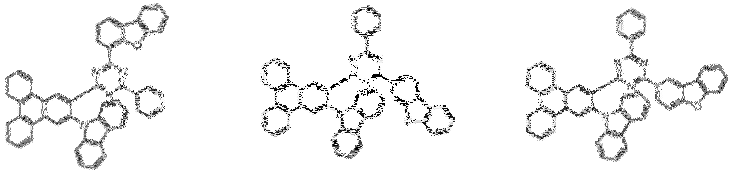

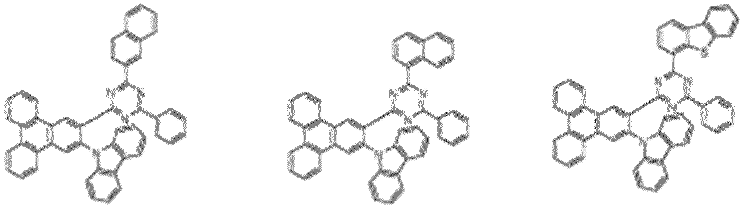

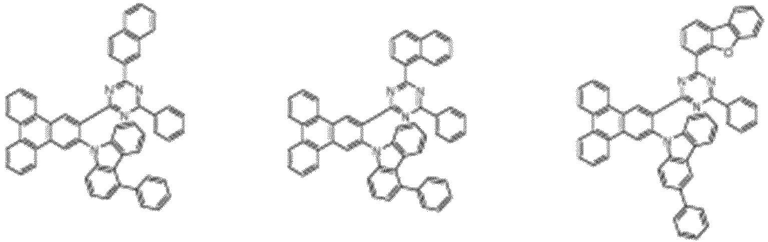

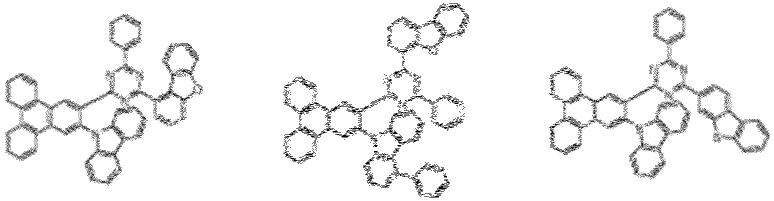

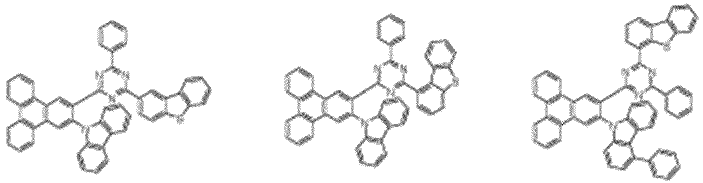

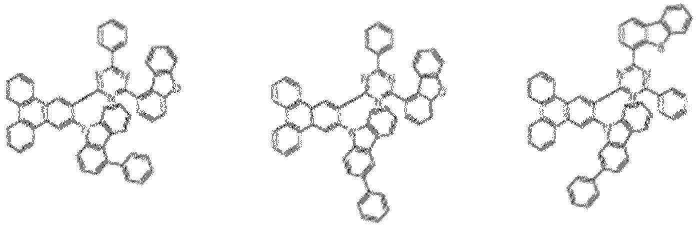

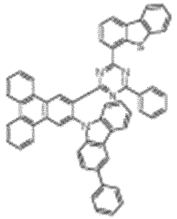

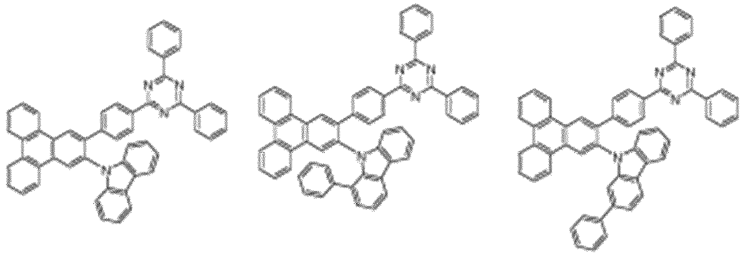

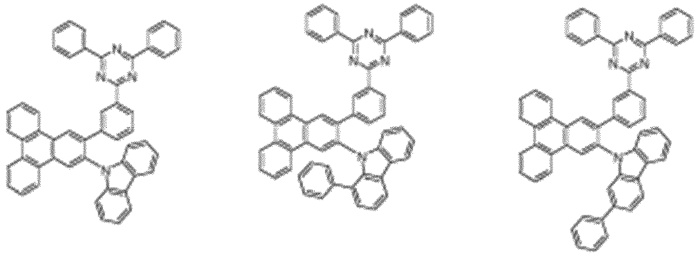

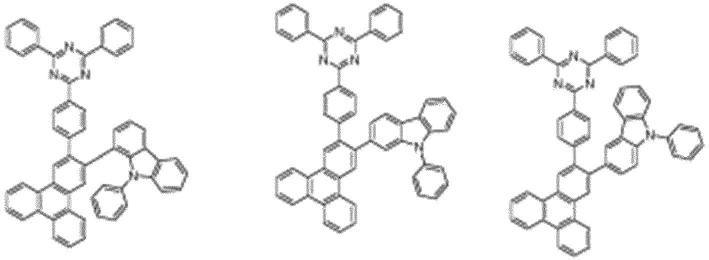

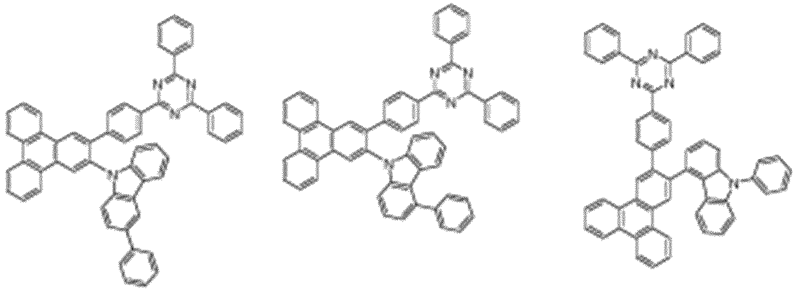

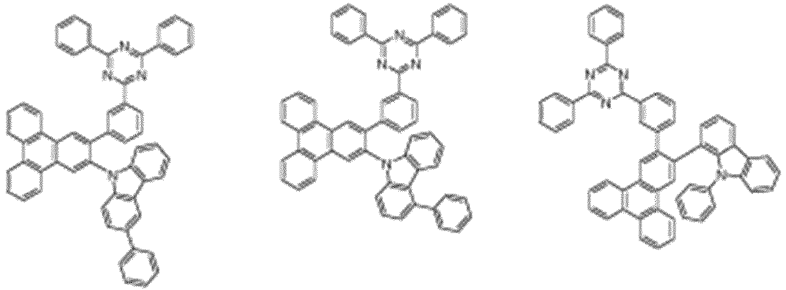

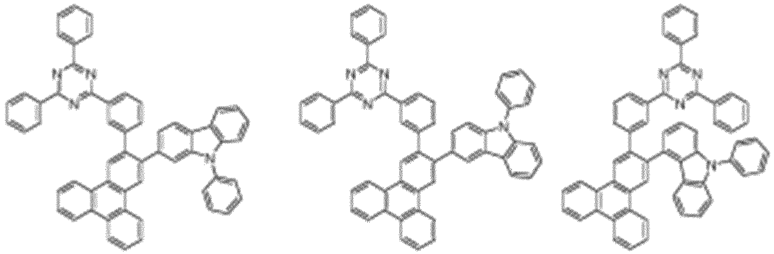

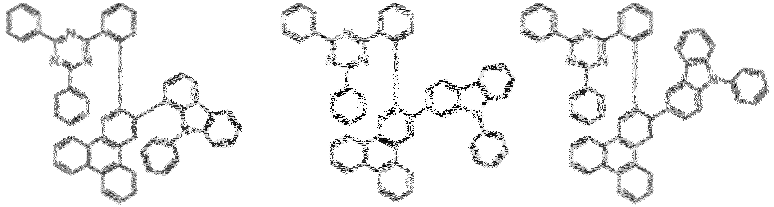

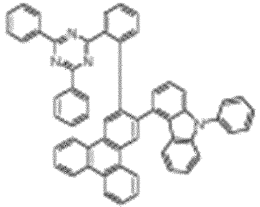

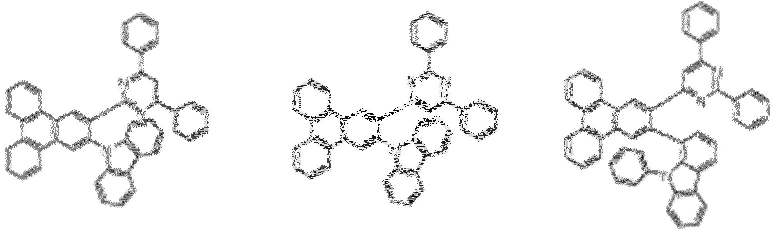

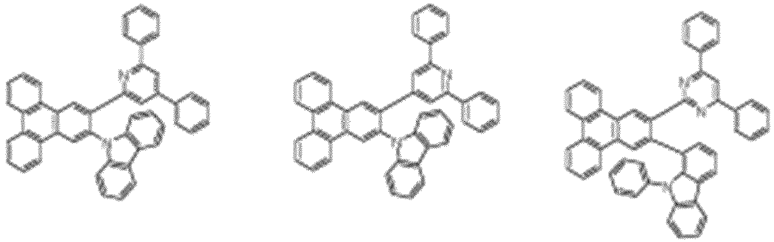

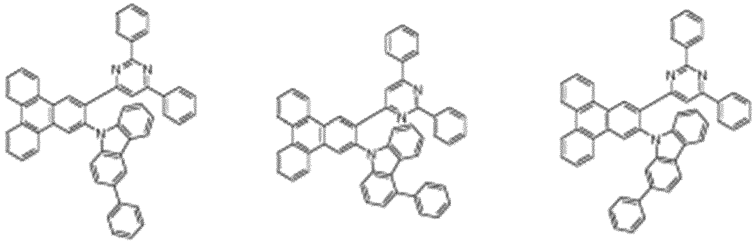

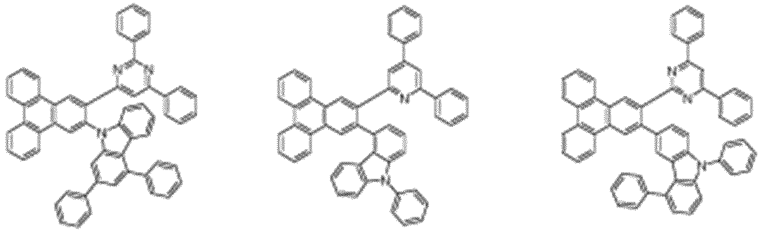

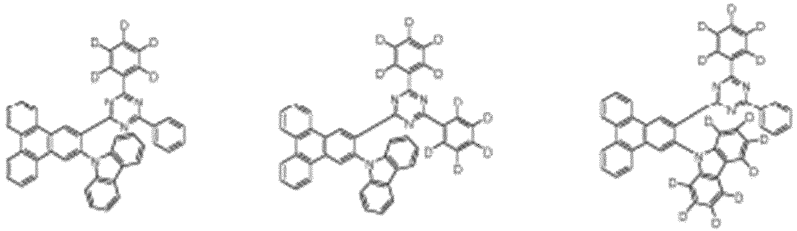

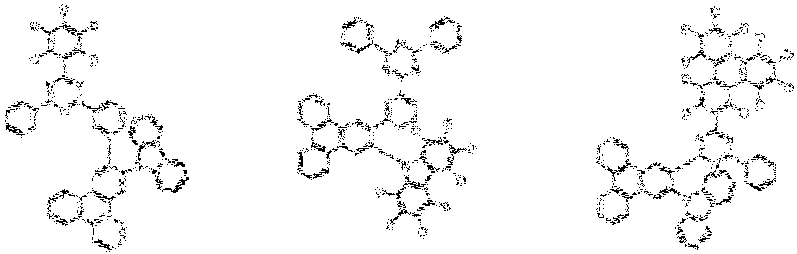

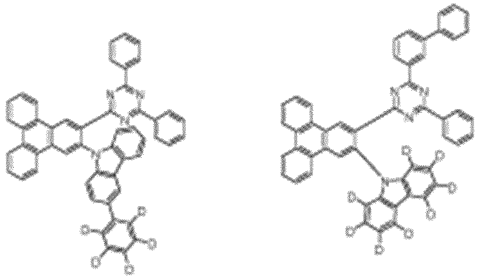

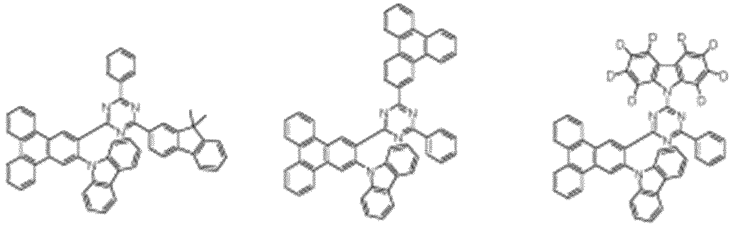

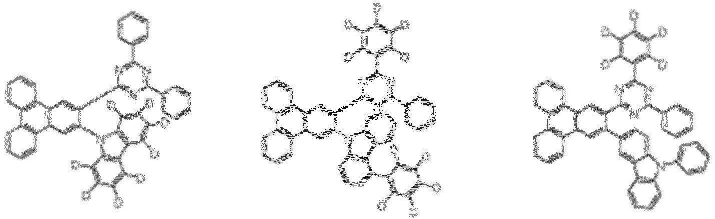

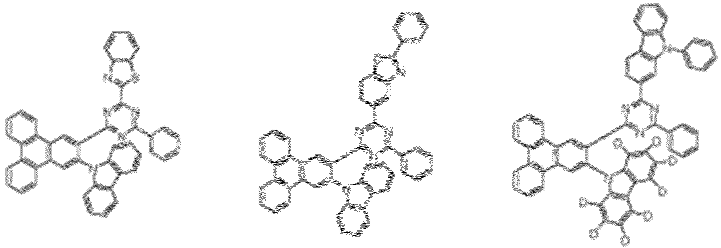

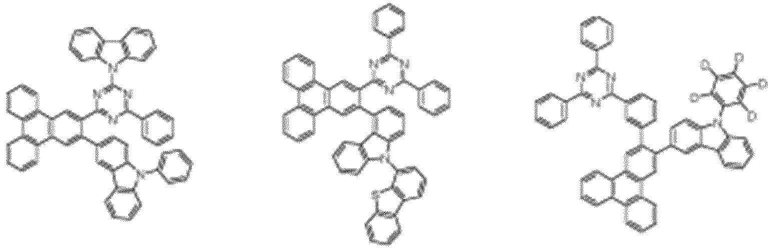

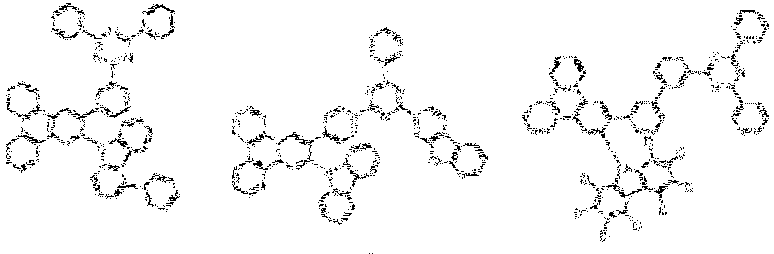

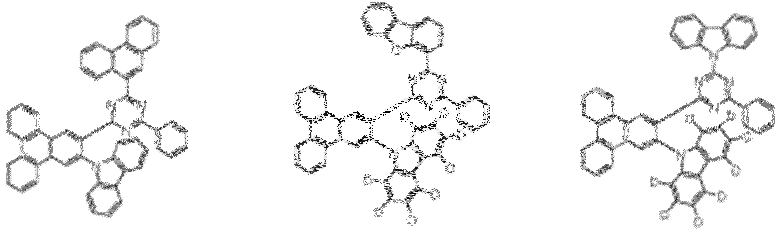

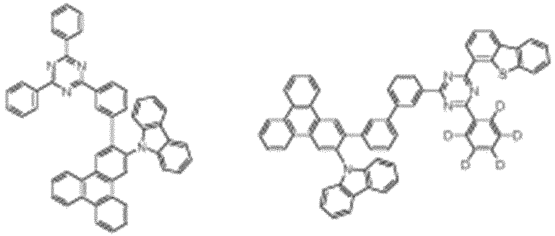

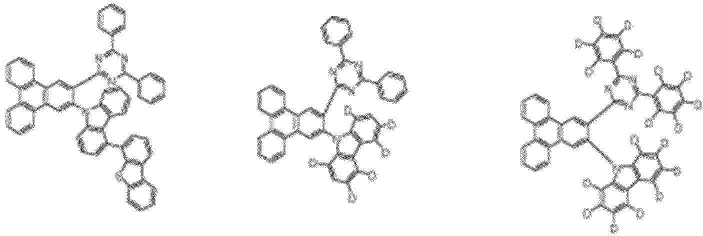

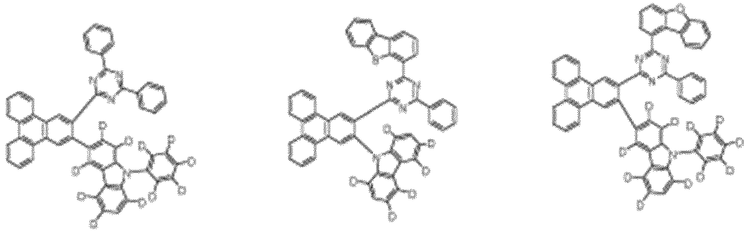

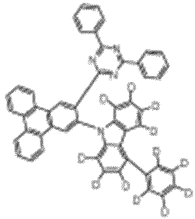

9. Dispositif électroluminescent organique selon la revendication 1,
dans lequel le second composé est représenté par l'une quelconque des formules chimiques 2-1 à 2-5 suivantes : 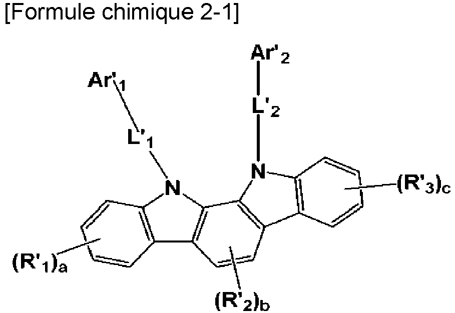 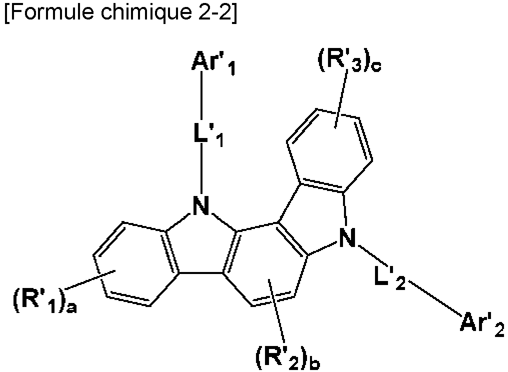 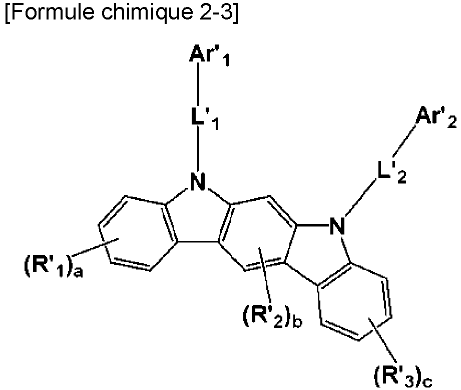 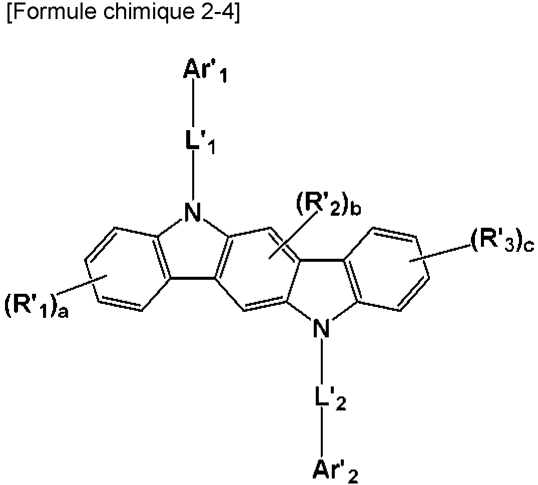 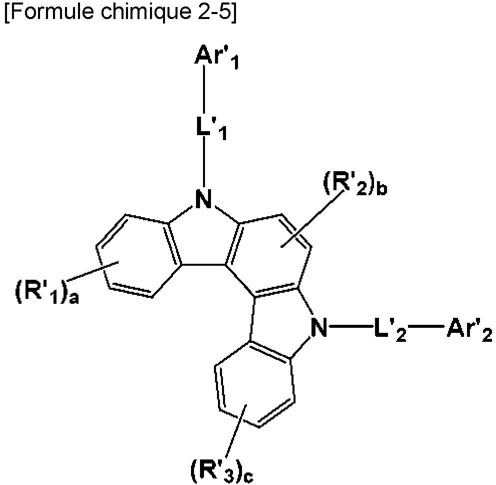 dans les formules chimiques 2-1 à 2-5,
L'₁, L'₂, Ar'₁, Ar'₂, R'₁, R'₂, R'₃, a, b et c sont tels que définis dans la revendication 1.

10. Dispositif électroluminescent organique selon la revendication 1,
dans lequel L'₁ et L'₂ sont chacun indépendamment une liaison simple, un phénylène ou un biphénylylène, et
le phénylène et le biphénylylène sont chacun indépendamment non substitués ou substitués avec au moins un deutérium.

11. Dispositif électroluminescent organique selon la revendication 1,
dans lequel Ar'₁ et Ar'₂ sont chacun indépendamment un phényle, un biphénylyle, un terphénylyle, un naphtyle, un phénanthrényle, un triphénylényle, un fluorényle, un dibenzofuranyle, un dibenzothiophényle, un carbazole-9-yle, ou un 9-phényl-9H-carbazolyle, et
Ar'₁ et Ar'₂ sont chacun indépendamment non substitués ou substitués avec au moins un substituant sélectionné dans le groupe consistant en un deutérium, un alkyle en C₁₋₁₀ et un aryle en C₆₋₂₀.

12. Dispositif électroluminescent organique selon la revendication 1,
dans lequel R'₁, R'₂ et R'₃ sont chacun indépendamment un hydrogène, un deutérium, un phényle, un dibenzofuranyle, un dibenzothiophényle, un carbazol-9-yle ou un 9-phényl-9H-carbazolyle, et
le phényle, le dibenzofuranyle, le dibenzothiophényle, le carbazol-9-yle et le 9-phényl- 9H-carbazolyle sont chacun indépendamment non substitués ou substitués avec au moins un deutérium.

13. Dispositif électroluminescent organique selon la revendication 1,
dans lequel le second composé est l'un quelconque sélectionné dans le groupe consistant en les composés suivants :
